# EUROPEAN PATENT SPECIFICATION

(11) **EP 1 482 935 B1**
(45) Date of publication and mention of the grant of the patent: **20.08.2008**
(21) Application number: 03702943.6
(22) Date of filing: 24.02.2003
(51) Int. Cl.: A61K 31/426, A61K 31/427, C07D 277/26, C07D 277/24, C07D 417/12

(54) **THIAZOLE AND OXAZOLE DERIVATIVES WHICH MODULATE PPAR ACTIVITY**
THIAZOL- UND OXAZOLDERIVATEN ALS PPAR MODULATOREN
DERIVES DE THIAZOLE ET D'OXAZOLE QUI MODULENT L'ACTIVITE DE PPAR

(30) Priority: 07.03.2002 US 362402 P
(43) Date of publication of application: 08.12.2004
(73) Proprietor: Warner-Lambert Company LLC, New York, NY 10017 (US)
(72) Inventor: CHENG, Xue-Min, Pfizer Global Res. and Develop., Ann Arbor, MI 48105 (US); ERASGA, Noe, Ouano, Pfizer Global Res. and Devolp., Ann Arbor, MI 48105 (US); FILZEN, Gary F., Pfizer Global Res. and Develop., Ann Arbor, MI 48105 (US); GEYER, Andrew G., Pfizer Global Res. and Develop., Ann Arbor, MI 48105 (US); LEE, Chitase, Pfizer Global Res. and Development, Ann Arbor, MI 48105 (US); TRIVEDI, Bharat K., Pfizer Global Res. and Devel., Ann Arbor, MI 48105 (US)
(74) Representative: Hayles, James Richard
(86) International application number: PCT/IB2003/000708
(87) International publication number: WO 2003/074050

(56) References cited:
- EP-A- 0 930 299
- WO-A-01/00603
- WO-A-02/50047
- WO-A-02/062774
- WO-A-02/092590
- WO-A-02/100403

## Description

### FIELD OF THE INVENTION

The present invention relates to compounds and pharmaceutical formulations that can be used to treat conditions mediated by nuclear hormone receptors, more specifically, to compounds and pharmaceutical formulations that modulate PPAR activity.

### BACKGROUND OF THE INVENTION

Hypercholesterolemia, hyperlipidemia, and diabetes are well recognized risk factors in the onset of atherosclerosis and coronary heart disease. Hypercholesterolemia and hyperlipidemia are characterized by excessively high levels of blood cholesterol and lipids. The blood cholesterol pool is generally dependent on dietary uptake of cholesterol from the intestine and biosynthesis of cholesterol throughout the body, especially the liver. The majority of cholesterol in plasma is carried on apolipoprotein B-containing lipoproteins, such as low-density lipoproteins (LDL) and very-low-density lipoproteins (VLDL). The risk of coronary artery disease in man increases when LDL and VLDL levels increase. Conversely, high levels of cholesterol carried in high-density lipoproteins (HDL) is protective against coronary artery disease (Am. J. Med., 1977;62:707-714).

The statins represent perhaps the most important class of lipid-lowering drugs. These compounds inhibit HMG-CoA reductase which is implicated in the rate-limiting step in cellular cholesterol biosynthesis. Representative statins include atorvastatin, lovastatin, pravastatin, and simvastatin. The effectiveness of these compounds depends on LDL receptor regulation. Other important antilipidemia drugs include fibrates such as gemfibril and clofibrate, bile acid sequestrants such as cholestyramine and colestipol, probucol, and nicotinic acid analogs.

To date, a number of oral antidiabetic agents have been developed. The most commonly used hypoglygemic drugs are the sulfonylureas. Sulfonylureas are generally used to stimulate insulin. The biguanide metformin is generally used to improve insulin sensitivity and to decrease hepatic glucose output. Acarbose is used to limit postprandial hyperglycemia. Thiazolidine 2,4 diones are used to enhance insulin action without increasing insulin secretion.

Peroxisome Proliferator Activation Receptors (PPAR) are implicated in a number of biological processes and disease states including hypercholesterolemia, hyperlipidemia, and diabetes. PPARs are members of the nuclear receptor superfamily of transcription factors that includes steroid, thyroid, and vitamin D receptors. They play a role in controlling expression of proteins that regulate lipid metabolism. Furthermore, the PPARs are activated by fatty acids and fatty acid metabolites. There are three PPAR subtypes PPAR α, PPAR β (also referred to as PPAR δ), and PPAR γ. Each receptor shows a different pattern of tissue expression, and differences in activation by structurally diverse compounds. PPAR γ, for instance, is expressed most abundantly in adipose tissue and at lower levels in skeletal muscle, heart, liver, intestine, kidney, vascular endothelial and smooth muscle cells as well as macrophages. PPAR receptors are associated with regulation of insulin sensitivity and blood glucose levels, macrophage differentiation, inflammatory response, and cell differentiation. Accordingly, PPARs have been associated with obesity, diabetes, carcinogenesis, hyperplasia, atherosclerosis, hyperlipidemia, and hypercholesterolemia.

In addition, PPARα agonists lower plasma triglycerides and LDL cholesterol and are therefore useful in treating hypertriglyceridemia, hyperlipidemia and obesity. PPAR γ is associated with the development of non-insulin-dependent diabetes mellitus (NIDDM), hypertension, coronary artery disease, hyperlipidemia and certain malignancies. Finally, activation of PPAR β has been demonstrated to increase HDL levels. (Leibowitz, WO97/28149, Aug. 1997.) More recently, a PPAR β selective agonist was reported to have shown a dose-related increase in serum HDL-C and decrease in LDL-C and VLDL-TG in insulin-resistant middle aged rhesus monkeys. (W. R. Oliver et al., PNAS, v. 98, pp. 5306-5311, 2001)

Antilipidemic and antidiabetic agents are still considered to have non-uniform effectiveness. The effectivieness of antidiabetic and antilipidemic therapies is limited, in part because of poor patient compliance due to unacceptable side effects. These side effects include diarrhea and gastrointestinal discomfort, and in the case of antidiabetics, edema, hypoglycemia and hepatoxicity. Furthermore, each type of drug does not work equally well in all patients.

For the reasons set forth above, there is a need for novel antilipidemic and antidiabetic agents that can be used alone or in combination. Furthermore, activation of PPARβ alone or in combination with the simultaneous activation of PPAR α and/or PPAR γ may be desirable in formulating a treatment for hyperlipidemia in which HDL is increased and LDL lowered.

WO 02 50047A Relates to substituted oxazoles and thiazoles as HPPAR alpha agonists. WO 02 092590A Relates to furan and thiophene derivatives that activate human peroxisome proliferator activated receptors. WO 02 062774A Relates to thiazole derivatives for treating PPAR related disorders. WO 01 00603A Relates to thiazole and oxazole derivatives that activate the delta subtype of the human peroxisome proliferator activated receptor. WO 02 100403A Relates to modulators of peroxisome proliferator activated receptors. EP-A-0 930 299 Relates to propionic acid derivatives which have hypoglycemic action.

### SUMMARY OF THE INVENTION

The present invention provides compounds capable of modulating PPAR activity. Compounds of the present invention are described by Formula I: 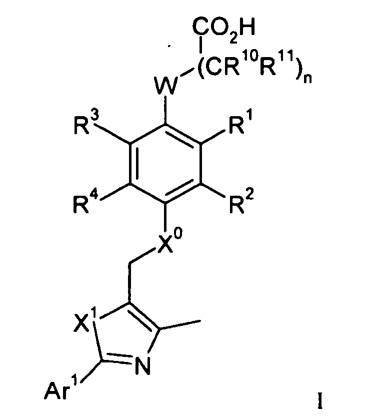 or a pharmaceutically acceptable salt thereof,
wherein:
W is CR⁵R⁶, -(CH₂)ₚ-(C₃-C₈cycloalkylene), or -(CH₂)ₚ-(C₃-C₈ heterocycloalkylene);
X⁰ and X¹ are independently O or S;
Ar¹ is aryl or heteroaryl;
R¹, R², R³, and R⁴ are hydrogen, C₁-C₆alkyl, C₁-C₆alkoxy, halo-C₁-C₁₁-alkyl, -O-(CH₂)ₚCF₃, halogen, nitro, cyano, -OH, -SH, -CF₃, -S(O)ₚC₁-C₁₁alkyl, S(O)ₚaryl, -(CH₂)ₘOR⁷, -(CH₂)ₘNR⁸R⁹, -COR⁷, -CO₂H, -CO₂R⁷, or -NR⁸R⁹;
R⁵ and R⁶ are joined together to form a 3 to 7 member cycloalkyl or cycloalkenyl;
R⁷ is independently hydrogen, C₁-C₁₁alkyl, C₂-C₁₂alkenyl, C₂-C₁₂alkynyl or aryl;
R⁸ and R⁹ are independently hydrogen, C₁-C₁₁alkyl, C₂-C₁₂alkenyl, C₂-C₁₂ alkynyl, -COC₁-C₁₁alkyl, -COaryl, C₃-C₈cycloalkyl, -CO₂C₁-C₁₁alkyl, -CO₂aryl, -SO₂ C₁-C₁₁alkyl, -SO₂aryl, or joined together to form a 4 to 7 member ring having 1 to 3 heteroatoms;
R¹⁰ and R¹¹ are independently hydrogen, halo, aryl or heteroaryl;
m is 0 to 5;
n is 0 to 5;
p is 0 to 2;
'aryl' means an aromatic ring which is unsubstituted or optionally substituted by 1 to 4 substituents selected from C₁-C₆alkyl, C₁-C₆alkoxy, C₁-C₆ thioalkoxy, halogen, nitro, cyano, -OH, -SH, -CF₃, -CO₂H, -CO₂C₁-C₆ alkyl, -(CH₂)₀₋₂CF₃, -NH₂, -NHC₁-C₆alkyl, -SO₂C₁-C₁₁alkyl, -SO₂NH₂, -CONR'R", or -N(C₁-C₆ alkyl)₂ where R' and R"are independently C₁-C₁₁alkyl, C₂-C₁₂ alkenyl, C₂-C₁₂alkynyl or joined together to form a 4 to 7 member ring; and 'heteroaryl' means an aromatic ring containing one or more heteroatoms which is unsubstituted or optionally substituted by 1 to 4 substituents selected from C₁-C₆alkyl, C₁-C₆alkoxy, C₁-C₆thioalkoxy, halogen, nitro, cyano, -OH, -SH, -CF₃, -CO₂H, -CO₂C₁-C₆alkyl, -(CH₂)₀₋₂CF₃, -NH₂, -NHC₁-C₆alkyl, -SO₂C₁-C₁₁alkyl, -SO₂NH₂, -CONR'R", or -N(C₁-C₆ alkyl)₂ where R' and R"are independently C₁-C₁₁alkyl, C₂-C₁₂alkenyl, C₂-C₁₂alkynyl or joined together to form a 4 to 7 member ring.

In one embodiment of the present invention a pharmaceutical composition comprising a compound of Formula I and one or more pharmaceutically acceptable carriers, diluents, or excipients is provided.

In one embodiment of the present invention of the use of a compound of Formula I for treating, preventing or controlling hypercholesteremia and hyperlipidemia in a mammal is provided. The use comprises administering to the mammal in need thereof a therapeutically effective amount of the compounds of the present invention.

In another embodiment of the present invention the use of a compound of Formula I for treating, preventing, or controlling atherosclerosis is provided.

For each disease state treatable, preventable, or controllable by the use of the present invention, a therapeutically effective amount of the compounds of the present invention are administered to the mammal in need thereof.

In yet another embodiment of the present invention, a method for preparing compounds of Formula I is provided.

### DETAILED DESCRIPTION OF THE INVENTION

The following definitions are used, unless otherwise described: alkyl, alkoxy, alkenyl, alkynyl, etc. denote both straight and branched groups; but reference to an individual radical such as "propyl" embraces only the straight chain radical, a branched chain isomer such as "isopropyl" being specifically referred to.

The term "alkyl" as used herein refers to a straight or branched hydrocarbon of from 1 to 11 carbon atoms and includes, for example, methyl, ethyl, n-propyl, isopropyl, n-butyl, sec-butyl, isobutyl, *tert*-butyl, n-pentyl, n-hexyl, and the like. Useful alkyl groups have from 1 to 6 carbon atoms (C₁-C₆ alkyl).

The term "lower alkyl" as used herein refers to a subset of alkyl which means a straight or branched hydrocarbon radical having from 1 to 6 carbon atoms and includes, for example, methyl, ethyl, n-propyl, isopropyl, n-butyl, sec-butyl, isobutyl, *tert*-butyl, n-pentyl, n-hexyl, and the like. Optionally, herein lower alkyl is referred to as "C₁-C₆alkyl."

The term "haloalkyl" as used herein refers to a lower alkyl radical, as defined above, bearing at least one halogen substituent, for example, chloromethyl, fluoroethyl, trifluoromethyl, or 1,1,1-trifluoroethyl and the like. Haloalkyl can also include perfluoroalkyl wherein all hydrogens of a loweralkyl group are replaced with fluorine atoms.

The term "alkenyl" means a straight or branched unsaturated hydrocarbon radical having from 2 to 12 carbon atoms and includes, for example, ethenyl, 1-propenyl, 2-propenyl, 1-butenyl, 2-butenyl, 1-pentenyl, 2-pentenyl, 3-methyl-3-butenyl, 1-hexenyl, 2-hexenyl, 3-hexenyl, 3-heptenyl, 1-octenyl, 1-nonenyl, 1-decenyl, 1-undecenyl, 1-dodecenyl, and the like.

The term "alkynyl" means a straight or branched hydrocarbon radical having from 2 to 12 carbon atoms having at least one triple bond and includes, for example, 1-propynyl, 1-butynyl, 3-butynyl, 1-pentynyl, 3-pentynyl, 3-methyl-3-butynyl, 1-hexynyl, 3-hexynyl, 3-heptynyl, 1-octynyl, 1-nonynyl, 1-decynyl, 1-undecynyl, 1-dodecynyl, and the like.

The term "alkylene" as used herein refers to a divalent group derived from a straight or branched chain saturated hydrocarbon having from 1 to 10 carbon atoms by the removal of two hydrogen atoms, for example methylene, 1,2-ethylene, 1,1-ethylene, 1,3-propylene, 2,2- dimethylpropylene, and the like. The alkylene groups of this invention can be optionally substituted. The alkylene group can also be substituted with one or more of the substituents selected from lower alkyl, lower alkoxy, lower thioalkoxy, -O(CH₂)₀₋₂CF₃, halogen, nitro, cyano, =O, =S, -OH, -SH, -CF₃, -CO₂H, -CO₂C₁-C₆ alkyl, -NH₂, -NHC₁-C₆ alkyl, -CONR'R", or -N(C₁-C₆alkyl)₂ where R' and R" are independently alkyl, akenyl, alkynyl, aryl, or joined together to form a 4 to 7 member ring. Useful alkylene groups have from 1 to 6 carbon atoms (C₁-C₆ alkylene).

The term "cycloalkylene" as used herein refers to a divalent group derived from a cyclic saturated hydrocarbon having from 3 to 8 carbon atoms by the removal of two hydrogen atoms. The cycloalkylene groups of this invention can be optionally substituted. The cycloalkylene group can also be substituted with one or more of the substituents selected from lower alkyl, lower alkoxy, lower thioalkoxy, -O(CH₂)₀₋₂CF₃, halogen, nitro, cyano, =O, =S, -OH, -SH, -OCF₃, -CO₂H, -CO₂C₁-C₆ alkyl, -NH₂, -NHC₁-C₆ alkyl, -CONR'R", or -N(C₁-C₆alkyl)₂ where R' and R" are independently alkyl, akenyl, alkynyl, aryl, or joined together to form a 4 to 7 member ring. Useful cycloalkylene groups have from 3 to 6 carbon atoms (C₃-C₆ alkyl).

The term "halogen" includes chlorine, fluorine, bromine, and iodine.

The term "heteroatom" as used herein represents oxygen, nitrogen, or sulfur (O, N, or S) as well as sulfoxyl or sulfonyl (SO or SO₂) unless otherwise indicated.

The term "heterocycloalkylene" as used herein, refers to a cycloalkylene group that includes one or more heteroatoms such as oxygen, sulfur, or nitrogen.

The term "hydrocarbon chain" as used herein refers to a straight hydrocarbon of from 2 to 6 carbon atoms. The hydrocarbon chain is optionally substituted with one or more substituents selected from lower alkyl, lower alkoxy, lower thioalkoxy, -O(CH₂)₀₋₂CF₃, halogen, nitro, cyano, =O, =S, -OH, -SH, -CF₃, -CO₂H, -CO₂(C₁-C₆ alkyl), -NH₂, -NHC₁-C₆ alkyl, -CONR'R", or -N(C₁-C₆alkyl)₂ where R' and R" are independently alkyl, akenyl, alkynyl, aryl, or joined together to form a 4 to 7 member ring.

The term "heterocycle" means a saturated or unsaturated mono- or polycyclic (i.e. bicyclic) ring incorporating one or more (i.e. 1-4) heteroatoms selected from N, O, and S. It is understood that a heterocycle is optionally substituted with -OH, -O(alkyl), SH, S(alkyl), amine, halogen, acid, ester, amide, amidine, alkyl ketone, aldehyde, nitrile, fluoroalkyl, nitro, sulphone, sulfoxide or C₁₋₆ alkyl. Examples of suitable monocyclic heterocycles include, but are not limited to substituted or unsubstituted thienyl, furanyl, pyrrolyl, imidazolyl, pyrazolyl, thiazolyl, isothiazolyl, oxazolyl, isoxazolyl, triazoiyl, tetrazolyl, pyridinyl, pyrazinyl, pyrimidinyl, piperidinyl, pyrrolidinyl, piperazinyl, azetidinyl, aziridinyl, morpholinyl, thietanyl, oxetaryl. Examples of monocyclic diheterocycles include, but are not limited to, 1-, 2-, 4-, or 5-imidazolyl, 1-, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 1, 3-, or 5-triazolyl, 1-, 2-, or 3-tetrazolyl, 2-pyrazinyl, 2-, 4-, or 5-pyrimidinyl, 1- or 2-piperazinyl, 2-, 3-, or 4-morpholinyl. Examples of suitable bicyclic heterocycles include, but are not limited to indolizinyl, isoindolyl, benzofuranyl, benzothienyl, benzoxazolyl, benzimidazolyl, quinolinyl, isoquinolinyl, quinazolinyl, 1-, 2-, 3-, 4-, 5-, 6-, or 7-indolyl, 1-, 2-, 3-, 5-, 6-, 7-, or 8-indolizinyl, 1-, 2-, 3-, 4-, 5-, 6-, or 7-isoindolyl, 2-, 3-, 4-, 5-, 6-, or 7-benzothienyl, 2-, 4-, 5-, 6-, or 7-benzoxazolyl, 1-, 2-, 4-, 5-, 6-, or 7-benzimidazolyl, 2-, 3-, 4-, 5-, 6-, 7-, or 8-quinolinyl, and 1-, 3-, 4-, 5-, 6-, 7-, or 8-isoquinolinyl.

The term "hydrocarbon-heteroatom chain" as used herein refers to a hydrocarbon chain wherein one or more carbon atoms are replaced with a heteroatom. The hydrocarbon-heteroatom chain is optionally substituted with one or more substituents selected from lower alkyl, lower alkoxy, lower thioalkoxy, -O(CH₂)₀₋₂CF₃, halogen, nitro, cyano, =O, =S, -OH, -SH, -CF₃, -CO₂H, -CO₂C₁-C₆ alkyl, -NH₂, -NHC₁-C₆ alkyl, -CONR'R", or -N(C₁-C₆alkyl)₂ where R' and R" are independently alkyl, akenyl, alkynyl, aryl, or joined together to form a 4 to 7 member ring.

The term "heteroalkylene" as used herein, refers to an alkylene radical as defined above that includes one or more heteroatoms such as oxygen, sulfur, or nitrogen (with valence completed by hydrogen or oxygen) in the carbon chain or terminating the carbon chain.

The terms "lower alkoxy" and "lower thioalkoxy" as used herein refers to O-alkyl or S-alkyl of from 1 to 6 carbon atoms as defined above for "lower alkyl."

The term "aryl" as used herein refers to an aromatic ring which is unsubstituted or optionally substituted by 1 to 4 substituents selected from lower alkyl, lower alkoxy, lower thioalkoxy, halogen, nitro, cyano -OH, -SH, -CF₃, -CO₂H, -CO₂C₁-C₆ alkyl, -(CH₂)₀₋₂CF₃, -NH₂, -NHC₁-C₆ alkyl, -SO₂alkyl, -SO₂NH₂, -CONR'R", or -N(C₁-C₆alkyl)₂ where R' and R" are independently alkyl, akenyl, alkynyl, aryl, or joined together to form a 4 to 7 member ring. Examples include, but are not limited to, phenyl, 2-chlorophenyl, 3-chlorophenyl, 4-chlorophenyl, 2-methylphenyl, 3-methylphenyl, 4-methylphenyl, 2-methoxyphenyl, 3-methoxyphenyl, 4-methoxyphenyl, 2-chloro-3-methylphenyl, 2-chloro-4-methylphenyl, 2-chloro-5-methylphenyl, 3-chloro-2-methylphenyl, 3-chloro-4-methylphenyl, 4-chloro-2-methylphenyl, 4-chloro-3-methylphenyl, 5-chloro-2-methylphenyl, 2,3-dichlorophenyl, 2,5-dichlorophenyl, 3,4-dichlorophenyl, 2,3-dimethylphenyl, 3,4-dimethylphenyl, and the like.

The term "heteroaryl" means an aromatic ring containing one or more heteroatoms. The heteroaryl is optionally substituted with one or more groups enumerated for aryl. Examples of heteroaryl include, but are not limited to, thienyl, furanyl, pyrrolyl, pyridyl, pyrimidyl, imidazoyl, pyrazinyl, oxazolyl, thiazolyl, benzothienyl, benzofuranyl, indolyl, quinolinyl, isoquinolinyl, and quinazolinyl, and the like.

The term "cycloalkenyl" means a cycloalkyl group having one or more carbon-carbon double. Example includes cyclobutene, cyclopentene, cyclohexene, cycloheptene, cyclobutadiene, cyclopentadiene, and the like.

The term "patient" means all mammals including humans. Examples of patients include humans, cows, dogs, cats, goats, sheep, pigs, and rabbits.

A "therapeutically effective amount" is an amount of a compound of the present invention that when administered to a patient ameliorates a symptom of dyslipidemia, non-insulin dependent diabetes mellitus, obesity, hyperglycemia, hypercholesteremia, hyperlipidemia, atherosclerosis, hypertriglyceridemia, or hyperinsulinemia.

The term "a pharmaceutically acceptable salt" refers to the relatively non-toxic, inorganic and organic base or acid addition salts of compounds of the present invention. These salts can be prepared in situ during the final isolation and purification of the compounds or by separately reacting the purified compound in its free form with a suitable organic or inorganic base or acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobionate, and laurylsulphonate salts, and the like. These also include cations based on the alkali and alkaline earth metals, such as sodium, lithium, potassium, calcium, magnesium, and the like, as well as non-toxic ammonium, quaternary ammonium, and amine cations including, but not limited to ammonium, tetramethylammonium, tetraethylammonium, methylamine, dimethylamine, trimethylamine, triethylamine, ethylamine, and the like. (See, for example, Berge S.M., et al., "Pharmaceutical Salts," J Pharm. Sci., 1977;66:1-19, which is incorporated herein by reference.) The free base form may be regenerated by contacting the salt form with a base. While the free base may differ from the salt form in terms of physical properties, such as solubility, the salts are equivalent to their respective free bases for the purposes of the present invention.

The symbol " 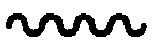 " indicates a point of attachment.

The present invention provides compounds capable of modulating PPAR activity. Compounds of the present invention are described by Formula I: 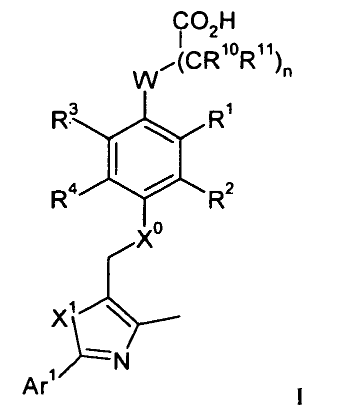 or a pharmaceutically acceptable salt thereof,
wherein:
W is CR⁵R⁶, -(CH₂)ₚ-(C₃-C₈cycloalkylene), or -(CH₂)ₚ-(C₃-C₈ heterocycloalkylene);
X⁰ and X¹ are independently O or S;
Ar¹ is aryl or heteroaryl;
R¹, R², R³, and R⁴ are hydrogen, C₁-C₆alkyl, C₁-C₆alkoxy, halo-C₁-C₁₁-alkyl, -O-(CH₂)ₚCF₃, halogen, nitro, cyano, -OH, -SH, -CF₃, -S(O)ₚC₁-C₁₁alkyl, S(O)ₚaryl, -(CH₂)ₘOR⁷, -(CH₂)ₘNR⁸R⁹, -COR⁷, -CO₂H, -CO₂R⁷, or -NR⁸R⁹;
R⁵ and R⁶ are joined together to form a 3 to 7 member cycloalkyl or cycloalkenyl;
R⁷ is independently hydrogen, C₁-C₁₁alkyl, C₂-C₁₂alkenyl, C₂-C₁₂alkynyl or aryl;
R⁸ and R⁹ are independently hydrogen, C₁-C₁₁alkyl, C₂-C₁₂alkenyl, C₂-C₁₂ alkynyl, -COC₁-C₁₁alkyl, -COaryl, C₃-C₈cycloalkyl, -CO₂C₁-C₁₁alkyl, -CO₂aryl, -SO₂ C₁-C₁₁alkyl, -SO₂aryl, or joined together to form a 4 to 7 member ring having 1 to 3 heteroatoms;
R¹⁰ and R¹¹ are independently hydrogen, halo, aryl or heteroaryl;
m is 0 to 5;
n is 0 to 5;
p is 0 to 2;
'aryl' means an aromatic ring which is unsubstituted or optionally substituted by 1 to 4 substituents selected from C₁-C₆alkyl, C₁-C₆alkoxy, C₁-C₆ thioalkoxy, halogen, nitro, cyano, -OH, -SH, -CF₃, -CO₂H, -CO₂C₁-C₆ alkyl, -(CH₂)₀-₂CF₃, -NH₂, -NHC₁-C₆alkyl, -SO₂C₁-C₁₁alkyl, -SO₂NH₂, -CONR'R", or -N(C₁-C₆ alkyl)₂ where R' and R"are independently C₁-C₁₁alkyl, C₂-C₁₂ alkenyl, C₂-C₁₂alkynyl or joined together to form a 4 to 7 member ring; and 'heteroaryl' means an aromatic ring containing one or more heteroatoms which is unsubstituted or optionally substituted by 1 to 4 substituents selected from C₁-C₆alkyl, C₁-C₆alkoxy, C₁-C₆thioalkoxy, halogen, nitro, cyano, -OH, -SH, -CF₃, -CO₂H, -CO₂C₁-C₆alkyl, -(CH₂)₀₋₂CF₃, -NH₂, -NHC₁-C₆alkyl, -SO₂C₁-C₁₁alkyl, -SO₂NH₂, -CONR'R", or -N(C₁-C₆ alkyl)₂ where R' and R"are independently C₁-C₁₁alkyl, C₂-C₁₂alkenyl, C₂-C₁₂alkynyl or joined together to form a 4 to 7 member ring.

Examples of compounds of Formula I include those where R¹, R², R³, and R⁴ are independently hydrogen, C₁-C₆alkyl, C₁-C₆alkoxy, -(CH₂)ₘNR⁸R⁹, or -(CH₂)ₘOR⁷; where R⁷ is C₁-C₁₁alkyl, R⁸ and R⁹ are independently hydrogen, C₁-C₁₁alkyl, or -COC₁-C₁₁alkyl. For example such compounds include those compounds where R¹, R², R³, and R⁴ are the following combinations shown in Table 1 below.

**Table 1**

| R¹ | R² | R³ | R⁴ |
|---|---|---|---|
| methyl | hydrogen | hydrogen | methyl |
| methyl | hydrogen | hydrogen | ethyl |
| methyl | hydrogen | hydrogen | iso-propyl |
| methyl | hydrogen | methyl | hydrogen |
| methyl | hydrogen | hydrogen | methoxy |
| hydrogen | methyl | methoxy | hydrogen |
| hydrogen | methyl | hydrogen | methyl |
| hydrogen | methoxy | hydrogen | methyl |
| hydrogen | methyl | iso-propyl | hydrogen |
| iso-propyl | hydrogen | iso-propyl | hydrogen |
| hydrogen | hydrogen | hydrogen | hydrogen |
| hydrogen | hydrogen | hydrogen | methoxy |
| methoxy | methoxy | hydrogen | hydrogen |
| hydrogen | methoxy | methoxy | hydrogen |
| methyl | hydrogen | hydrogen | -NH-CO-CH₃ |
| methyl | hydrogen | hydrogen | fluoro |
| methyl | hydrogen | hydrogen | chloro |
| methyl | hydrogen | hydrogen | -CH₂-O-CH₃ |

Other examples of compounds of Formula I also include those where R² is hydrogen; R¹, R³, and R⁴ are independently C₁-C₆alkyl, C₁-C₆alkoxy, haloC₁-C₁₁alkyl,-O-(CH₂)ₚCF₃, halogen, nitro, cyano, -OH, -SH, -CF₃, -S(O)ₚ C₁-C₁₁alkyl, S(O)ₚaryl, -(CH₂)ₘOR⁷, -(CH₂)ₘNR⁸R⁹, -COR⁷, -CO₂H, -CO₂R⁷, or-NR⁸R⁹; R⁷ is hydrogen, C₁-C₁₁alkyl, C₂-C₁₂alkenyl, C₂-C₁₂alkynyl, or aryl; and R⁸ and R⁹ are independently hydrogen, C₁-C₁₁alkyl, C₂-C₁₂alkenyl, C₂-C₁₂alkynyl, -COC₁-C₁₁alkyl, -COaryl, C₃-C₈cycloalkyl, -CO₂ C₁-C₁₁alkyl, -CO₂aryl, -SO₂alkyl, -SO₂aryl, or joined together to form a 4 to 7 member ring having 1 to 3 heteroatoms.

Additional examples of compounds of Formula I also include those where R² and R³ are both hydrogen. For example compounds of Formula I where R² and R³ are hydrogen include those where R² and R³ are hydrogen, and R¹ and R⁴ are C₁-C₆alkyl, such as methyl, ethyl, isopropyl, n-propyl, t-butyl, n-butyl, and isobutyl, or C₁-C₆ alkoxy, such as methoxy, ethoxy, isopropoxy, n-propoxy, t-butoxy, n-butoxy, and isobutoxy. More specific examples of compounds of Formula I where R² and R³ are hydrogen include those where R² and R³ are hydrogen, R¹ is C₁-C₆alkyl, and R⁴ is C₁-C₆alkoxy.

Other examples of compounds of Formula I include those where R¹⁰ and R¹¹ are independently fluorine, phenyl or pyrrolyl;

Additional examples of compounds of Formula I include those where W is CR⁵R⁶, -(CH₂)ₚ-(C₃-C₈cycloalkylene), or -(CH₂)ₚ-(C₃-C₈heterocycloalkylene), R⁵ and R⁶ are joined together to form a 3 to 7 member cycloalkyl or cycloalkenyl ring, and p is 0 to 2. For example, compounds of Formula I where W is CR⁵R⁶, -(CH₂)ₚ-( C₃-C₈cycloalkylene), or -(CH₂)ₚ-(C₃-C₈heterocycloalkylene) include those where W is

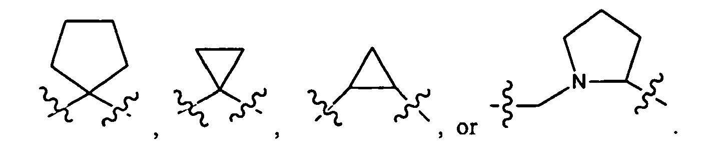

Other examples, compounds of Formula I where W is CR⁵R⁶, -(CH₂)ₚ-(C₃-C₈cycloalkylene), or -(CH₂)ₚ-(C₃-C₈heterocycloalkylene) include those where X⁰ and X¹ are S.

Additional examples, compounds of Formula I where W is CR⁵R⁶, -(CH₂)ₚ-(C₃-C₈cycloalkylene), or -(CH₂)ₚ-(C₃-C₈heterocycloalkylene) include those where Ar¹ is 4-trifluoromethylphenyl.

Further examples, compounds of Formula I where W is CR⁵R⁶, -(CH₂)ₚ- C₃-C₈ (cycloalkylene), or -(CH₂)ₚ-(C₃-C₈heterocycloalkylene) include those where R¹, R², R³, and R⁴ are independently hydrogen, C₁-C₆alkyl, C₁-C₆alkoxy, halogen, -CF₃, -(CH₂)ₘOR⁷, or -(CH₂)ₘNR⁸R⁹; R⁷ is hydrogen, or C₁-C₁₁alkyl; and R⁸ and R⁹ are independently hydrogen, C₁-C₁₁alkyl, or-CO C₁-C₁₁alkyl. Such compounds include those where R¹, R², R³, and R⁴ are independently hydrogen, methyl, ethyl, isopropyl, n-propyl, t-butyl, n-butyl, or isobutyl, methoxy, ethoxy, isopropoxy, n-propoxy, t-butoxy, n-butoxy, isobutoxy, -(CH₂)-OCH₃, or -NH-COCH₃.

Examples of compounds of Formula I include
2- {4-[4-Methyl-2-(4-trifluoromethyl-phenyl)-thiazol-5-ylmethylsulfanyl]-phenyl}-cyclopropanecarboxylic acid;
1-{4-[4-Methyl-2-(4-trifluoromethyl-phenyl)-thiazol-5-ylmethylsulfanyl]-phenyl}-cyclopropanecarboxylic acid;
1-{4-[4-Methyl-2-(4-trifluoromethyl -phenyl)-thiazol-5-ylmethylsulfanyl]-phenyl}-cyclopentanecarboxylic acid;
1-[ {3-Methoxy-4-[4-methyl-2(4-trifluoromethylphenyl)thiazol-5-ylmethylsulfanyl]benzyl}pyrrolidine-2-carboxylic acid;
(4- {4-[4-Methyl-2-(4-trifluoromethyl-phenyl)-thiazol-5-ylmethylsulfanyl]-phenyl}cyclohexyl)-acetic acid; and
pharmaceutically acceptable salts thereof.

Certain of the compounds of the present invention possess one or more chiral centers and each center may exist in the R or S configuration. The present invention includes all diastereomeric, enantiomeric, and epimeric forms as well as the appropriate mixtures thereof. Stereoisomers may be obtained, if desired, by methods known in the art as, for example, the separation of stereoisomers by chiral chromatographic columns. Additionally, the compounds of the present invention may exist as geometric isomers. The present invention includes all cis, trans, syn, anti, entgegen (E), and zusammen (Z) isomers as well as the appropriate mixtures thereof.

In some situations, compounds may exist as tautomers. All tautomers are included within Formula I and are provided by this invention.

In addition, the compounds of the present invention can exist in unsolvated as well as solvated forms with pharmaceutically acceptable solvents such as water, ethanol, and the like. In general, the solvated forms are considered equivalent to the unsolvated forms for the purposes of the present invention.

The present invention includes all pharmaceutically acceptable, non-toxic esters of the compounds of Formula I. Such esters include C₁-C₆ alkyl esters wherein the alkyl group is a straight or branched chain. Acceptable esters also include C₅-C₇ cycloalkyl esters as well as arylalkyl esters such as, but not limited to benzyl. C₁-C₄ alkyl esters are preferred. Esters of the compounds of the present invention may be prepared according to conventional methods.

The compounds of the present invention are suitable to be administered to a patient for the treatment, control, or prevention of non-insulin dependent diabetes mellitus, hypercholesteremia, hyperlipidemia, obesity, hyperglycemia, hyperlipidemia, atherosclerosis, hypertriglyceridemia, and hyperinsulinemia. Accordingly, the compounds may be administered to a patient alone or as part of a composition that contains other components such as excipients, diluents, and carriers, all of which are well-known in the art. The compositions can be administered to humans and/or animals either orally, rectally, parenterally (intravenously, intramuscularly, or subcutaneously), intracisternally, intravaginally, intraperitoneally, intravesically, locally (powders, ointments, or drops), or as a buccal or nasal spray.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil), and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid; (b) binders, as for example, carboxymethylcellulose, alignates, gelatin, polyvinylpyrrolidone, sucrose, and acacia; (c) humectants, as for example, glycerol; (d) disintegrating agents, as for example, agar, calcium carbonate, potato or tapioca starch, alginic acid, certain complex silicates, and sodium carbonate; (e) solution retarders, as for example paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft and hard-filled gelatin capsules using such excipients as lactose or milk sugar, as well as high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound or compounds in a certain part of the intestinal tract in a delayed manner. Examples of embedding compositions which can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are preferably suppositories which can be prepared by mixing the compounds of the present invention with suitable nonirritating excipients or carriers such as cocoa butter, polyethyleneglycol, or a suppository wax, which are solid at ordinary temperatures but liquid at body temperature and therefore, melt in the rectum or vaginal cavity and release the active component.

Dosage forms for topical administration of a compound of this invention include ointments, powders, sprays, and inhalants. The active component is admixed under sterile conditions with a physiologically acceptable carrier and any preservatives, buffers, or propellants as may be required. Ophthalmic formulations, eye ointments, powders, and solutions are also contemplated as being within the scope of this invention.

The compounds of the present invention can be administered to a patient at dosage levels in the range of about 0.1 to about 2,000 mg per day. For a normal human adult having a body weight of about 70 kilograms, a dosage in the range of about 0.01 to about 10 mg per kilogram of body weight per day is preferable. However, the specific dosage used can vary. For example, the dosage can depend on a numbers of factors including the requirements of the patient, the severity of the condition being treated, and the pharmacological activity of the compound being used. The determination of optimum dosages for a particular patient is well-known to those skilled in the art.

### PREPARATION OF COMPOUNDS OF THE INVENTION

The present invention contains compounds that can be synthesized in a number of ways familiar to one skilled in organic synthesis. The compounds disclosed herein can be synthesized according to the methods described below and in the examples, along with methods typically utilized by a synthetic chemist, and combinations or variations of those methods, which are generally known to one skilled in the art of synthetic chemistry. The synthetic route of compounds in the present invention is not limited to the methods outlined below. It is assumed one skilled in the art will be able to use the schemes outlined below to synthesize compounds claimed in this invention. Individual compounds may require manipulation of the conditions in order to accommodate various functional groups. A variety of protecting groups generally known to one skilled in the art may be required. Purification, if necessary, can be accomplished on a silica gel column eluted with the appropriate organic solvent system. Also, reverse phase HPLC or recrystallization may be employed.

The compounds or Formula I can be prepared by reacting: 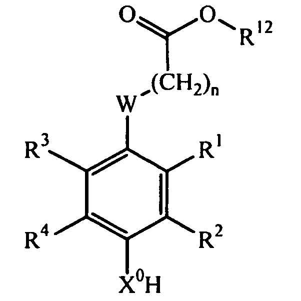 in a solvent in the presence of a base such as cesium carbonate, with the aryl halide: 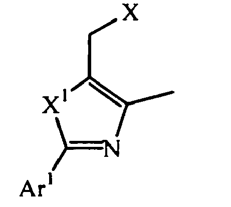 wherein:
W, Y, n, R¹, R², R³, R⁴, X⁰, X¹ and Ar¹ are the same as defined above for Formula I;
R¹² is a C₁-C₆alkyl; and
X is a halogen.

The resulting ester is then converted to the compounds of Formula I by various methods known in the art for the conversion of esters to acids, such as via hydrolysis for example. Useful aryl halides include, for example, 5-chloromethyl-4-methyl-2-(4-trifluoromethyl-phenyl)-thiazole.

Reference compounds of Formula I can be prepared using the synthetic route outlined in Scheme 1 when W is O or S and X⁰ is S. With reference to Scheme 1, compounds of the general formula A are thiocyanated with a mixture of bromine and sodium thiocyanate to give compounds of the general formula **B**. Compounds of the general formula **B** are then alkylated with the haloester **C** to give compounds of the general formula **D.** A useful haloester **C** is the corresponding bromoester. Compounds of the general formula **E** are then prepared by reduction of **D** with dithiothreitol in methanol. Compounds of the general formula **E** are then alkylated with the halide compound **1B** to form compound **F.** Suitable halide compounds **1B** include, for example, 5-chloromethyl-4-methyl-2-(4-trifluoromethyl-phenyl)-thiazole. Compounds of the general formula **F** are then saponified with LiOH in THF to give the final compound **G.** W, Y, R¹, R², R³, and R⁴ are the same as defined above for Formula I above; X is a halogen. Compound **G** corresponds to compounds described by Formula I above when X⁰ is S.

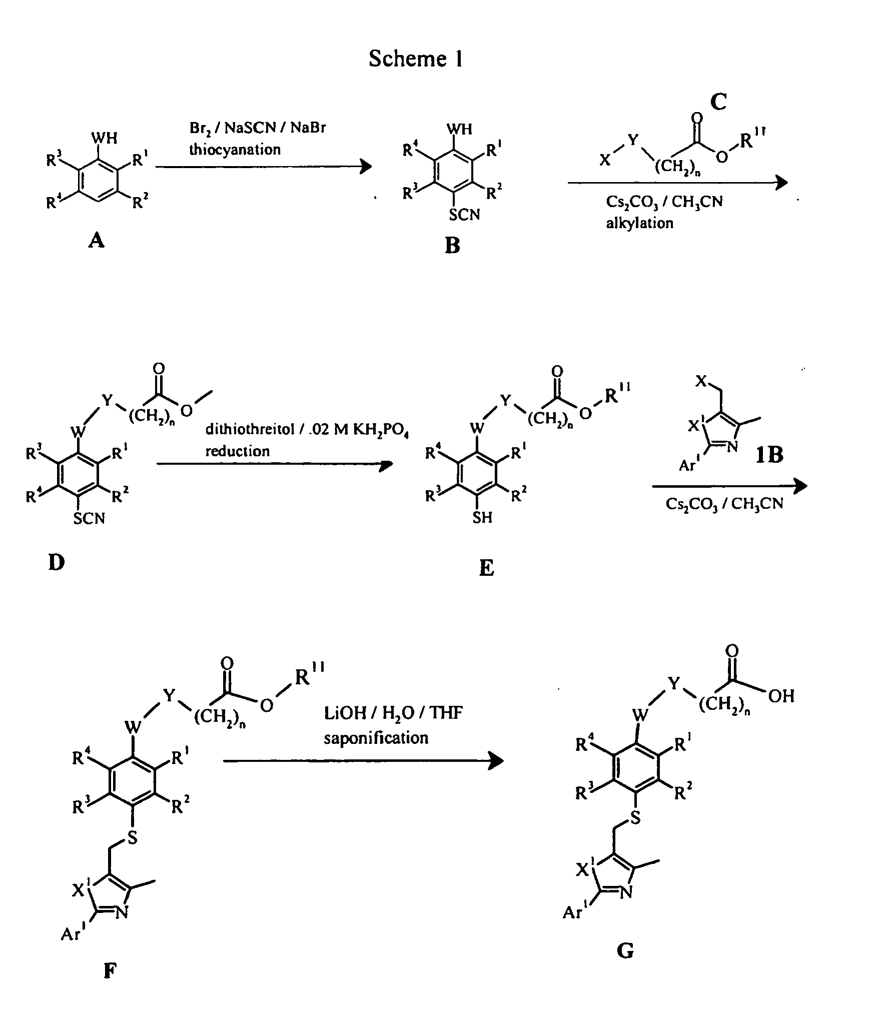

Alternatively, compounds of Formula I can be prepared using the synthetic route outlined in Scheme 2 when X⁰ is S. With reference to Scheme 2, compounds of the general formula **H** are debenzylated and then reacted with 2-chloro-N,N-dimethyl-thioacetamide to form compound **J.** A useful method for step 1 is reaction with hydrogen gas in the presence of carbon activated palladium. Compound **J** is then heated followed by saponification to form compound **E.** Compounds of the general formula **E** are then alkylated with the halide compound **1B** to form compound **F.** Suitable halide compounds **1B** include, for example, 5-chloromethyl-4-methyl-2-(4-trifluoromethyl-phenyl)-thiazole. Compounds of the general formula F are then saponified with LiOH in the THF to give the final compound **G.** W, Y, R¹, R², R³, and R⁴ are the same as defined above for Formula I above; X is a halogen. Compound **G** corresponds to compounds described by Formula I above when X⁰ is S.

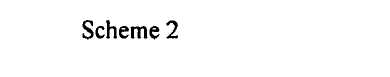

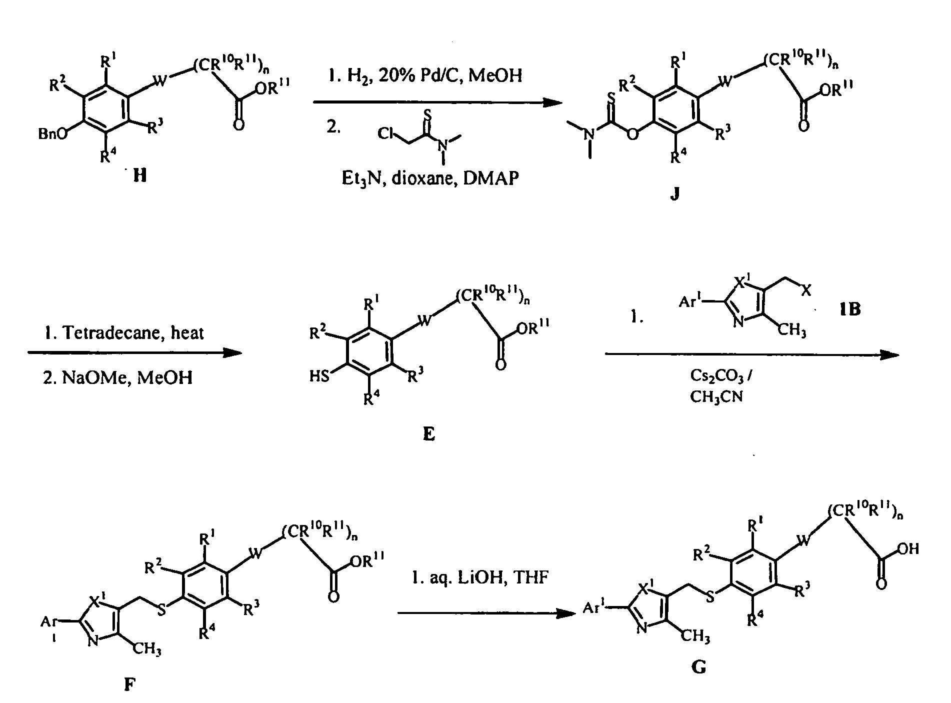

Finally, compounds of Formula I can be prepared using the synthetic route outlined in Scheme 3. With reference to Scheme 3, compounds of the general formula **K** are converted to the ester **L.** A useful method includes reacting compound **K** with an alcohol in the presence of an acid such as hydrochloric acid, although any compatible method for esterification may be used. Ester **L** is then reacted with cholorsulfonic acid to form compound **M.** Compound **M** is then reduced to form compound **E.** Compounds of the general formula **E** are then alkylated with the halide compound **1B** to form compound **F.** Suitable halide compounds **1B** include, for example, 5-chloromethyl-4-methyl-2-(4-trifluoromethyl-phenyl)-thiazole. Compounds of the general formula **F** are then saponified with LiOH in the THF to give the final compound **G.** W, Y, R¹, R², R³, and R⁴ are the same as defined above for Formula I above; X is a halogen. Compound **G** corresponds to compounds described by Formula I above when X⁰ is S.

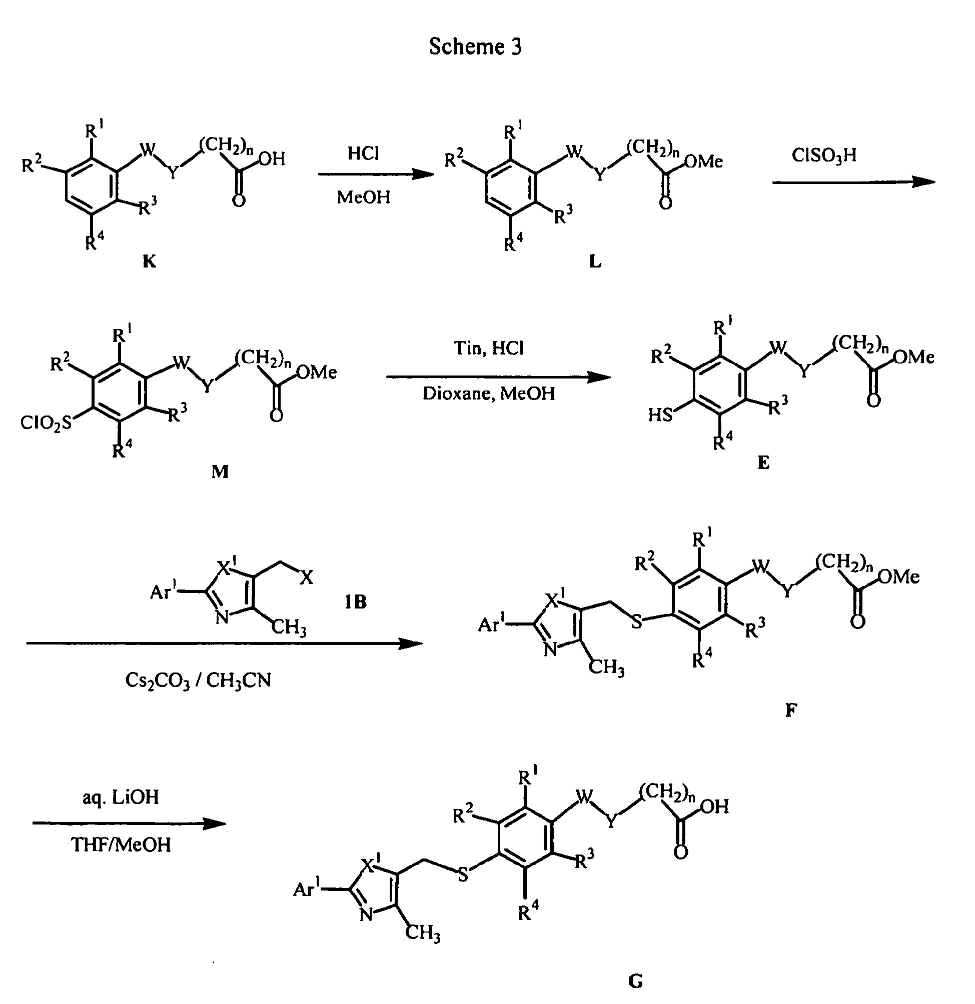

The following descriptions also demonstrate methods for the synthesis of compounds of Formula I.

### Reference Example 1

### Synthesis of {2,5-Dimethyl-4-[4-methyl-2-(4-trifluoromethyl-phenyl)-thiazol-5-ylmethylsulfanyl]-phenoxy}-acetic acid (Reference Compound 1)

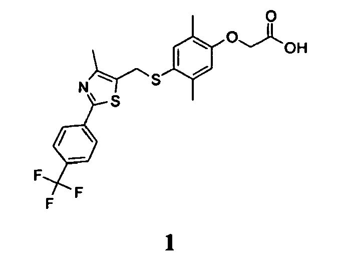

### Preparation of [4-Methyl-2-(4-trifluoromethyl-phenyl)-thiazol-5-yl]-methanol (compound 1A)

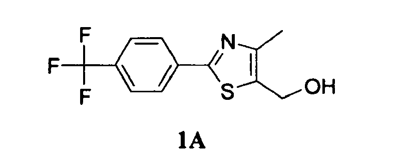

A solution of 4-methyl-2-(4-trifluoromethyl-phenyl)-thiazole-5-carboxylic acid ethyl ester (3.0 g, 9.5 mmol) in 25 mL THF at 0 °C was treated portionwise with lithium aluminum hydride (0.4 g, 10.5 mmol). After 4 hours, the reaction mixture was carefully quenched with water, followed by the addition of 10 mL 6N NaOH. The reaction mixture was then extracted with EtOAc, dried, and concentrated in vacuo. Recrystalisation from CHCl₃/hexanes gave the title compound (2.2 g, 85%). 400 MHz ¹H NMR (DMSO-*d*₆) δ 8.03 (d, 2H, *J=* 8.3 Hz), 7.78 (d, 2H, *J=* 8.3 Hz), 5.66 (s(br), 1H), 4.62 (s, 2H), 2.31 (s, 3H).

### Preparation of 5-Chloromethyl-4-methyl-2-(4-trifluoromethyl-phenyl)-thiazole (compound 1B)

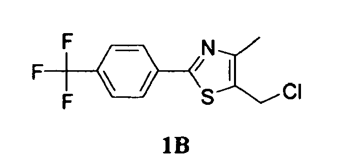

Methanesulfonyl chloride (1.0 mL, 12.9 mmol) was added to a stirred solution of [4-methyl-2-(4-trifluoromethyl-phenyl)-thiazol-5-yl]-methanol (2.2 g, 8.1 mmol) and triethylamine (2.2 mL, 16.1 mmol) in 25 mL THF at 0 °C. After 3 hours, the reaction mixture was diluted with dichloromethane, washed with 1 x sat. NaHCO₃, 1 x brine, dried (Na₂SO₄) and the solvent removed in vacuo to give 2.0 g (84%) of the title compound pure enough for subsequent use. 400 MHz ¹H NMR (DMSO-*d*₆) δ 8.06 (d, 2H, *J=* 8.3 Hz), 7.80 (d, 2H, *J*= 8.3 Hz), 5.08 (s, 2H), 2.40 (s, 3H).

### Preparation of 2,5-Dimethyl-4-thiocyanato-phenol (compound 1C)

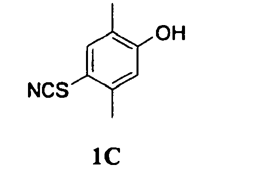

The title compound was prepared in a manner analogous to compound **3A**. 400 MHz ¹H NMR (DMSO-d₆) δ 10.0 (s, 1H), 7.35 (s, 1H), 6.73 (s, 1H), 2.3 (s, 3H), 2.04 (s, 3H); MS *m*/*z* 180 (m+1).

### Preparation of (2,5-Dimethyl-4-thiocyanato-phenoxy)-acetic acid methyl ester (compound 1D)

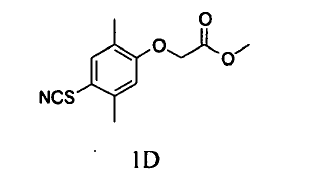

The title compound was prepared from compound 1C in a manner analogous to compound **3B.** 400 MHz ¹H NMR (DMSO-*d*₆) δ 7.07 (s, 1H), 6.50 (s, 1H), 4.56 (s, 2H), 3.76 (s, 3H), (s, 1H), 2.26 (s, 3H), 2.17 (s, 3H); MS *m*/*z* 252 (m+1).

### Preparation of (4-Mercapto-2,5-dimethyl-phenoxy)-acetic acid methyl ester (compound 1E)

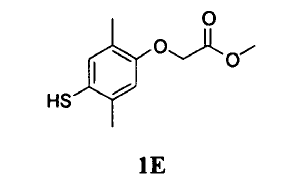

The title compound was prepared from compound 1D in a manner analogous to compound **3C**. 400 MHz ¹H NMR (DMSO-*d*₆) δ 7.07 (s, 1H), 6.50 (s, 1H), 4.56 (s, 2H), 3.76 (s, 3H), 3.07 (s, 1H), 2.26 (s, 3H), 2.17 (s, 3H); MS *m*/*z* 227 (M+1).

### Preparation of {2,5-Dimethyl-4-[4-methyl-2-(4-trifluoromethyl-phenyl)-thiazol-5-ylmethylsulfanyl]-phenoxy}-acetic acid methyl ester (compound 1F)

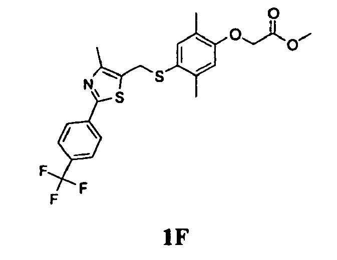

Compound **1E** (207 mg, 0.92 mmol) was dissolved in 5 ml anhydrous acetonitrile. Compound **1B** (294 mg, 1 mmol) was added followed by cesium carbonate (600 mg, 1.84 mmol). The reaction was stirred at ambient temperature for 2 hours, filtered and concentrated. Purification by flash column chromatography afforded the title compound. 400 MHz ¹H NMR (DMSO-*d*₆) δ 8.00 (d, 2H, J = 8 Hz), 7.76 (d, 2H, *J=* 8.4 Hz), 7.15 (s, 1H), 6.72 (s, 1H), 4.76 (s, 2H), 4.22 (s, 2H), 2.17 (s, 3H), 3.63 (s, 3H), 2.17 (s, 3H), 2.09 (s, 3H), 2.05 (s, 3H). MS *mlz* 482 (M+1).

### Preparation of {2,5-Dimethyl-4-[4-methyl-2-(4-trifluoromethyl-phenyl)-thiazol-5-ylmethylsulfanyl]-phenoxy}-acetic acid (Reference Compound 1)

Compound **1F** (360 mg, 0.75 mmol), dissolved in 5 ml THF and 1 ml water, was treated with lithium hydroxide monohydrate (95 mg, 2.25 mmol); stirring at room temperature for 1 hour. The reaction mixture was then acidified to about pH 3 with 2 N HCl. The reaction was then extracted into ethyl acetate (2x20 ml). The organic extracts were washed with brine, dried over anhydrous sodium sulfate, decanted, and concentrated. The title compound was then recrystalized from chloroform/ hexanes. Mp 173-176°C; IR (thin film) cm⁻¹:2928, 1732, 1328, 1113; 400 MHz ¹H NMR (DMSO-*d*₆) δ 12.97 (s(br), 1H), 8.00 (d, 2H, J = 8 Hz), 7.76 (d, 2H, *J=* 8.4 Hz), 7.15 (s, 1H), 6.69 (s, I H), 4.63 (s, 2H), 4.22 (s, 2H), 2.17 (s, 3H), 2.09 (s, 3H), 6.00 (s, 2H), 2.04 (s, 3H); MS *m*/*z* 468 (M+1). Anal. Calc'd for C₂₂H₂₀F₃NO₃S₂ C, 56.52; H, 4.31; N, 3.00; found: C, 56.55; H, 4.31; N, 2.94.

### Reference Example 2

### Synthesis of {5-Ethyl-2-methyl-4-[4-methyl-2-(4-trifluoromethyl-phenyl)-thiazol-5-ylmethylsulfanyl]-phenoxy}-acetic acid (Reference Compound 2)

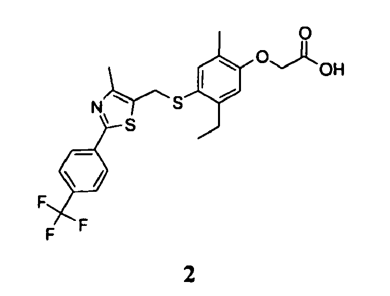

### Preparation of 5-Ethyl-2-methyl-phenol (compound 2A)

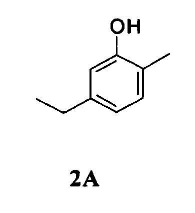

A 3:1 mixture of 1-(5-ethyl-2-methyl-phenyl)-ethanone and 1-(2-ethyl-5-methylphenyl)-ethanone (J. Chem. Soc., 152, 1123) (3.6 g, 22 mmol), m-chloroperbenzoic acid (8.4 g, 29 mmol), and p-toluenesulfonic acid (0.5 g, 2.6 mmol) in 100 mL dichloromethane was heated at reflux for 20 hours. The reaction was then cooled and washed with KI(aq.) (2 x 75 mL), NaHSO₃ (2 x 75mL), dried (Na₂SO₄) and concentrated in vacuo. The resulting residue was taken up in Et₂O and washed with sat. NaHCO₃(1 x 75 mL), dried (Na₂SO₄) and concentrated in vacuo to give 3.2 g of the crude product pure enough for subsequent use. 400 MHz ¹H NMR (DMSO-*d*₆) δ 7.11 (d, 2H, *J* = 7.8 Hz), 6.96 (d, 2 H, *J* = 7.8 Hz), 6.83 (s, 1H), 6.80 (s, 1H), 2.51 (q, 2H, *J*= 7.6 Hz), 2.37 (q, 2H, *J*= 7.6 Hz), 2.23 (s, 6H), 2.01 (s, 6H), 1.10 (t, 3H, *J*= 7.6 Hz), 1.08 (t, 3H, *J*= 7.6 Hz). The crude product was then dissolved in MeOH (75 mL) and K₂CO₃ (2.5 g, 18 mmol) was added followed by stirring for 15 minutes. The reaction mixture was then filtered, the filtrate collected and concentrated in vacuo. The residue was taken up in Et₂O and washed with 2M HCL (1 x 50 mL), brine (1 x 50 mL), dried (Na₂SO₄) and the solvent removed in vacuo to give the title compound and its regioisomer as a 3:1 mixture pure enough for subsequent use. 400 MHz ¹H NMR (DMSO-*d*₆) δ 400 MHz ¹H NMR (DMSO-*d*₆) δ 6.87 (d, 1H, *J*= 7.3 Hz *(title compound*))*,* 6.85 (d, 1H, *J=* 7.3 Hz), 6.54 (s, 1H(*title compound*))*,* 6.46 (d, 2H, *J* = 7.3 Hz), 2.44 (m, 4H), 2.37, 2.00 (s, 6H), 1.06 (m, 6H).

### Preparation of 5-Ethyl-2-methyl-4-thiocyanato-phenol (compound 2B)

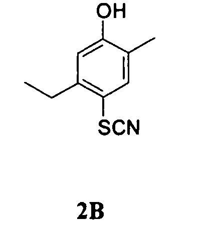

**2B** was prepared from **2A** in a similar manner as described for compound **3A** to give, after purification by flash column chromatography (gradient elution: 100% hexanes to 20% EtOAc/hexane) and then recrytalisation from CHCl₃/hexanes, 0.8 g (29%) of the title compound as a single regioisomer. 400 MHz ¹H NMR (DMSO-*d*₆) δ 7.36 (s, 1H), 6.77 (s, 1H), 2.67 (q, 2H, *J=* 7.4 Hz), 2.05 (s, 3H), 1.13 (t, 3H, *J*= 7.4 Hz); MS *m*/*z* 194 (M+1).

### Preparation of (5-Ethyl-2-methyl-4-thiocyanato-phenoxy)-acetic acid methyl ester (compound 2C)

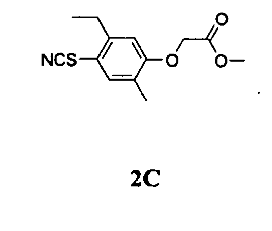

**2C** was prepared from **2B** in a similar manner as described for compound **3B** to give 1.1 g (95%) of the title compound pure enough for subsequent use. 400 MHz ¹H NMR (DMSO-*d*₆) δ 7.46 (s, 1H), 6.90 (s, 1H), 4.87 (s, 2H), 3.65 (s, 3H), 2.72 (q, 2H, *J* = 7.5 Hz), 2.13 (s, 3H), 1.14 (t, 3H, *J =* 7.5 Hz).

### Preparation of (5-Ethyl-4-mercapto-2-methyl-phenoxy)-acetic acid methyl ester (compound 2D)

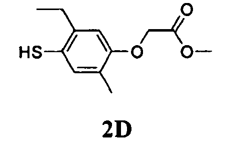

**2D** was prepared from **2C** in a similar manner as described for compound **3C** to give, after purification by flash column chromatography (gradient elution: 100% hexanes to 30% EtOAc/hexanes), 0.3 g (32%) of the title compound. 400 MHz ¹H NMR (DMSO-*d*₆) δ 7.09 (s, 1H), 6.72 (s, 1H), 4.81 (s, 2H), 3.64 (s, 3H), 2.55 (q, 2H, *J*= 7.5 Hz), 2.05 (s, 3H), 0.99 (t, 3H, *J=* 7.5 Hz); MS *m*/*z* 241 (M+1).

### Preparation of {5-Ethyl-2-methyl-4-[4-methyl-2-(4-trifluoromethyl-phenyl)-thiazol-5-ylmethylsulfanyl]-phenoxy}-acetic acid methyl ester (compound 2E)

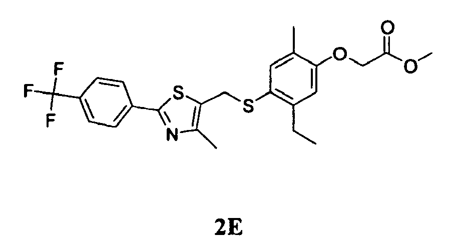

Compound **2D** and 5-chloromethyl-4-methyl-2-(4-trifluoromethyl-phenyl)-thiazole were coupled in a similar manner as described for compound **1F** to provide, after purification by flash column chromatography (gradient elution: 100% hexanes to 30% EtOAc/hexanes), the title compound (0.24 g , 59%) as a yellow solid. IR (thin film) cm⁻¹: 1749, 1326; 400 MHz ¹H NMR (DMSO-*d*₆) δ 7.98 (d, 2H, *J*= 8.0 Hz), 7.76 (d, 2H, *J*= 8.0 Hz), 7.19 (s, 1H), 6.68 (s, 1H), 4.77 (s, 2H), 4.24 (s, 2H), 3.63 (s, 3H), 2.54 (q, 2H, *J=* 7.6 Hz), 2.09 (s, 3H), 2.06 (s, 3H), 0.97 (t, 3H, *J=* 7.6 Hz); MS *m*/*z* 496 (M+1); Anal. Calc'd for C₂₄H₂₄F₃N₁O₃S₂ C, 58.17; H, 4.88; N, 2.83; found: C, 58.19; H, 4.80; N, 2.76.

### Preparation of {5-Ethyl-2-methyl-4-[4-methyl-2-(4-trifluoromethyl-phenyl)-thiazol-5-ylmethylsulfanyl]-phenoxy}-acetic acid (Reference Compound 2)

Compound **2E** was saponified in the same manner as described for compound **1** to give, after recrystalisation from CHCl₃/hexanes, the title compound (0.12 g, 96%) as a pale yellow solid. mp 169-171 °C; IR (thin film) cm⁻¹: 1736, 1325; 400 MHz ¹H NMR (DMSO-*d*₆) δ 7.98 (d, 2H, *J*= 8.0 Hz), 7.76 (d, 2H, *J*= 8.0 Hz), 7.17 (s, 1H), 6.66 (s, 1H), 4.65 (s, 2H), 4.23 (s, 2H), 2.54 (q, 2H, *J*= 7.6 Hz), 2.09 (s, 3H), 2.05 (s, 3H), 0.97 (t, 3H, *J =* 7.6 Hz); MS *m*/*z* 482 (M+1); Anal. Calc'd for C₂₄H₂₄F₃N₁O₃S₂ 0.5 H₂O C, 56.31; H, 4.73; N, 2.86; found: C, 56.53; H, 4.69; N, 2.68.

### Reference Example 3

### Synthesis of {5-Isopropyl-2-methyl-4-[4-methyl-2-(4-trifluoromethyl-phenyl)-thiazol-5-ylmethylsulfanyl]-phenoxy}-acetic acid (Reference Compound 3)

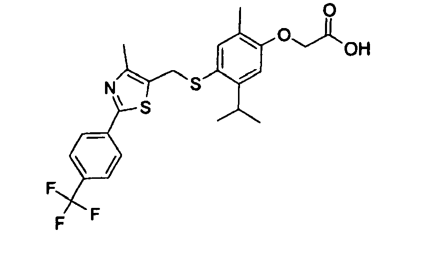

### Preparation of 5-Isopropyl-2-methyl-4-thiocyanato-phenol (compound 3A)

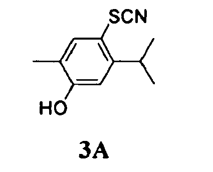

A rapidly stirred solution of 5-isopropyl-2-methyl-phenol (10 g, 67 mmol), sodium thiocyanate (15.6 g, 214 mmol), and sodium bromide (6.8 g, 66 mmol) in 40 mL MeOH at 0 °C was treated with bromine in 40 mL MeOH by dropwise addition over 30 minutes. After completion of the bromine addition, the reaction mixture was heated at 50 °C for 45 minutes. The reaction mixture was then cooled and concentrated in vacuo to about 20 mL. The resulting residue was taken up in EtOAc and filtered. The filtrate was collected and washed with saturated Na₂CO₃ (1 x 50 mL), brine (1 x 50 mL) dried (Na₂SO₄), and the solvent removed in vacuo. Purification by flash column chromatography (gradient elution: 5% EtOAc/hexanes to 35% EtOAc/hexanes to 55% EtOAc/hexanes) gave the title compound (7.9 g, 57 %) as a yellow solid. mp 72-75 °C; 400 MHz ¹H NMR (DMSO-*d*₆) δ 10.0 (s, 1H), 7.37 (s, 1H), 6.81 (s, 1H), 3.31 (m, 1H), 2.04 (s, 3H), 1.14 (d, 6H, *J* = 6.8 Hz); MS *m*/*z* 208 (M+1).

### Preparation of (5-Isopropyl-2-methyl-4-thiocyanato-phenoxy)-acetic acid methyl ester (compound 3B)

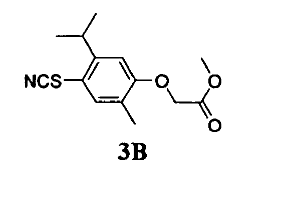

A solution of 5-isopropyl-2-methyl-4-thiocyanato-phenol (3.0 g, 14 mmol), methyl bromoacetate (0.90 g, 15.4 mmol), and cesium carbonate (6.8 g, 21 mmol) in 50 mL acetonitrile was heated at 60 °C for 3 hours. PS-Trisamine scavenger resin was then added to the warmed solution followed by an additional 30 minutes heating. The reaction mixture was then cooled and filtered. The filtrate was collected, diluted with 100 mL ether, washed with brine (1 x 50 mL), dried (Na₂SO₄), and the solvent removed in vacuo to give 3.8 g (94%) of the title compound as an orange solid, pure enough for subsequent use. mp 67-69 °C; 400 MHz ¹H NMR (DMSO-*d*₆) δ 7.46 (s, 1 H), 6.88 (s, 1 H), 4.91 (s, 2H), 3.65 (s, 3H), 3.31 (m, 1 H), 2.13 (s, 3H), 1.16 (d, 6H, *J* = 6.8 Hz); MS *m*/*z* 279 (M+).

### Preparation of (5-Isopropyl-4-mercapto-2-methyl-phenoxy)-acetic acid methyl ester (compound 3C)

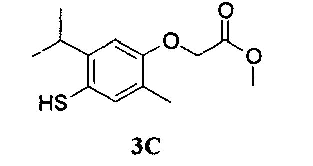

A solution of (5-isopropyl-2-methyl-4-thiocyanato-phenoxy)-acetic acid methyl ester (3g, 10.7 mmol), dithiothreitol (2.1 g, 13.9 mmol), and KH₂PO₄ (5 mL of a.02M solution) in 40 mL MeOH was heated at reflux for 1 hour after which time thin-layer chromatography (33% EtOAc hexanes) indicated the reaction to be complete. The reaction was cooled and concentrated in vacuo to about 10 mL. The resulting residue was diluted with 100 mL ether and washed with brine (2 x 50 mL), dried (Na₂SO₄), and the solvent removed in vacuo. Purification by flash column chromatography (gradient elution: 5% EtOAc/hexanes to 25% EtOAc/hexanes to 55% EtOAc/hexanes) gave the title compound (2.0 g, 72 %) as a yellow oil. 400 MHz ¹H NMR (DMSO-*d*₆) δ 7.10 (s, 1H), 6.64 (s, 1H), 4.75 (s, 2H), 3.63 (s, 3H), 3.02 (septet, 1H, *J* = 6.8 Hz), 2.05 (s, 3H), 1.08 (d, 6H, *J*= 6.8 Hz); MS *m*/*z* 255 (M+1).

### Preparation of {5-Isopropyl-2-methyl-4-[4-methyl-2-(4-trifluoromethyl-phenyl)-thiazol-5-ylmethylsulfanyl]-phenoxy}-acetic acid methyl ester (compound 3D)

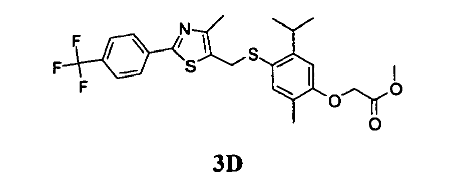

**3C** and 5-chloromethyl-4-methyl-2-(4-trifluoromethyl-phenyl)-thiazole (compound **1B)** were coupled in a similar manner as described for compound **1F** to provide, after purification by flash column chromatography (gradient elution: 10% EtOAc/hexanes to 35% EtOAc/hexanes), the title compound (0.66 g , 82%) as a yellow solid. IR (thin film) cm⁻¹: 2960, 1763, 1324; 400 MHz ¹H NMR (DMSO-d₆) δ 7.97 (d, 2H, *J*= 8.1 Hz), 7.76 (d, 2H, *J*= 8.1 Hz), 7.23 (s, 1 H), 6.64 (s, 1 H), 4.80 (s, 2H), 4.21 (s, 2H), 3.61 (s, 3H), 3.31 (m, 1H), 2.06 (s, 3H), 2.03 (s, 3H), 1.08 (d, 6H, *J*= 6.8 Hz); MS *m*/*z* 510 (M+1); Anal. Calc'd for C₂₅H₂₆F₃N₁O₃S₂ C, 58.92; H, 5.14; N, 2.75; found: C, 58.50; H, 5.39; N, 2.66.

### Preparation of {5-Isopropyl-2-methyl-4-[4-methyl-2-(4-trifluoromethyl-phenyl)-thiazol-5-ylmethylsulfanyl]-phenoxy}-acetic acid (Reference Compound 3)

3D was saponified in the same manner as described for compound 1 to give, after recrystalisation from CHCl₃/hexanes, the title compound (0.24 g, 71%) as a white solid. mp 132-134 °C; IR (thin film) cm⁻¹: 1744, 1325; 400 MHz ¹H NMR (DMSO-*d*₆) δ 7.97 (d, 2H, *J=* 8.3 Hz), 7.76 (d, 2H, *J=* 8.3 Hz), 7.22 (s, 1H), 6.64 (s, 1H), 4.67 (s, 2H), 4.20 (s, 2H), 3.31 (m, 1H), 2.06 (s, 3H), 2.03 (s, 3H), 0.93 (d, 6H, *J*= 6.8 Hz); MS *m*/*z* 496 (M+1); Anal. Calc'd for C₂₄H₂₄F₃N₁O₃S₂ 0.1 H₂O C, 57.96; H, 4.90; N, 2.82; found: C, 57.62; H, 4.82; N, 2.68.

### Reference Example 4

### Synthesis of {2,6-Dimethyl-4-[4-methyl-2-(4-trifluoromethyl-phenyl)-thiazol-5-ylmethylsulfanyl]-phenoxy}-acetic acid (Reference Compound 4)

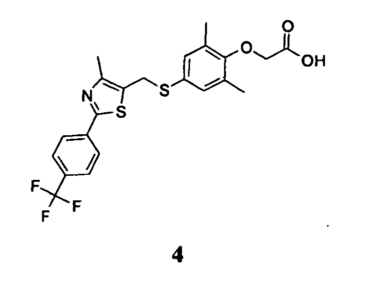

### Preparation of 2,6-Dimethyl-4-thiocyanato-phenol (compound 4A)

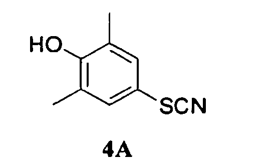

Compound **4A** was prepared from 2,6-dimethylphenol in a similar manner as described for compound **3A**. 400 MHz ¹H NMR (DMSO-*d*₆) δ 8.96 (s, 1H), 7.22 (s, 2H), 2.13 (s, 6H).

### Preparation of (2,6-Dimethyl-4-thiocyanato-phenoxy)-acetic acid methyl ester (compound 4B)

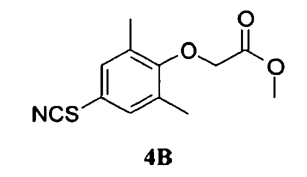

Compound **4B** was prepared from compound **4A** in a similar manner as described for compound **3B** to give 2.5 g (46%) of the title compound pure enough for subsequent use. 400 MHz ¹H NMR (DMSO-*d*₆) δ 7.11 (s, 2H), 4.41 (s, 2H), 3.63 (s, 3H), 2.14 (s, 6H).

### Preparation of (4-Mercapto-2,6-dimethyl-phenoxy)-acetic acid methyl ester (compound 4C)

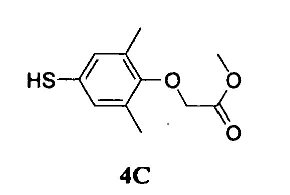

Compound **4C** was prepared from compound **4B** in a similar manner as described for compound **3C** to give, after purification by flash column chromatography (gradient elution: 100% hexanes to 30% EtOAc/hexanes), 1.8 g (82%) of the title compound. 400 MHz ¹H NMR (DMSO-*d*₆) δ 6.90 (s, 2H), 5.51 (s, 1H), 4.39 (s, 2H), 3.66 (s, 3H), 2.10 (s, 6H); MS *m*/*z* 225 (M-1).

### Preparation of {2,6-Dimethyl-4-[4-methyl-2-(4-trifluoromethyl-phenyl)-thiazol-5-ylmethylsulfanyl]-phenoxy}-acetic acid methyl ester (compound 4D)

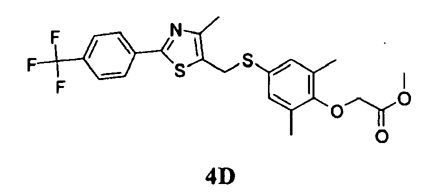

Compound **4C** and 5-chloromethyl-4-methyl-2-(4-trifluoromethyl-phenyl)-thiazole were coupled in a similar manner as described for compound **1F** to provide, after purification by flash column chromatography (gradient elution: 10% EtOAc/hexanes to 35% EtOAc/hexanes), the title compound (0.61 g , 57%) as a yellow solid. mp 104-105 °C;IR (thin film) cm⁻¹: 1754, 1325; 400 MHz ¹H NMR (DMSO-*d*₆) δ 7.99 (d, 2H, *J* = 8.3 Hz), 7.76 (d, 2H, *J =* 8.3 Hz), 7.02 (s, 2H), 4.41 (s, 2H), 4.37 (s, 2H), 3.65 (s, 3H), 2.23 (s, 3H), 2.10 (s, 6H); MS *m*/*z* 482 (M+1); Anal. Calc'd for C₂₃H₂₂F₃N₁O₃S₂ C, 57.37; H, 4.60; N, 2.91; found: C, 57.43; H, 4.55; N, 2.94.

### Preparation of {2,6-Dimethyl-4-[4-methyl-2-(4-trifluoromethyl-phenyl)-thiazol-5-ylmethylsulfanyl]-phenoxy}-acetic acid (Reference Compound 4)

Compound **4D** was saponified in the same manner as described for compound **1** to give, after recrystalisation from CHCl₃/hexanes, the title compound (0.29 g, 86%) as a pale yellow solid. mp 157-158 °C; IR (thin film) cm⁻¹: 1738, 1326; 400 MHz ¹H NMR (DMSO-*d*₆) δ 7.99 (d, 2H, *J=* 8.3 Hz), 7.76 (d, 2H, *J=* 8.3 Hz), 7.02 (s, 2H), 4.36 (s, 2H), 4.29 (s, 2H), 2.22 (s, 3H), 2.12 (s, 6H); MS *m*/*z* 468 (M+1); Anal. Calc'd for C₂₂H₂₀F₃N₁O₃S₂ 0.2 H₂O C, 56.09; H, 4.36; N, 2.97; found: C, 55.79; H, 4.22; N, 2.94.

### Reference Example 5

### Synthesis of {5-Methoxy-2-methyl-4-[4-methyl-2-(4-trifluoromethyl-phenyl)-thiazol-5-ylmethylsulfanyl]-phenoxy}-acetic acid (Reference Compound 5)

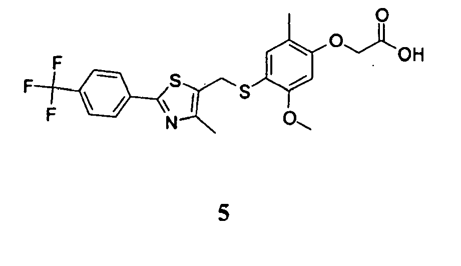

### Preparation of 5-Methoxy-2-methyl-phenol (compound 5A)

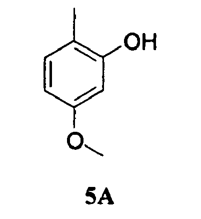

2-Hydroxy-4-methoxy-benzaldehyde (3 g, 19.7 mmol), ammonium formate (6.2 g, 99 mmol) and palladium/carbon (900 mg @ 10%) were added to 26 ml glacial acetic acid and heated at 110°C for 1 h. The reaction was cooled, filtered, and diluted with water (100 ml). The crude product was extracted with chloroform (3x50 ml), washed with water, brine, and dried over anhydrous sodium sulfate. The resulting solution was concentrated and used for the next step without further purification. MS *m*/*z* 139 (M+1).

### Preparation of 5-Methoxy-2-methyl-4-thiocyanato-phenol (compound 5B)

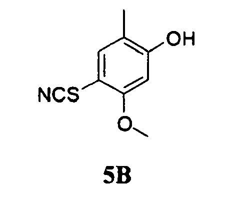

Compound **5B** was prepared from compound **5A** in a manner analogous for example **3A.** 400 MHz ¹H NMR (DMSO-*d*₆) δ 10.13 (s, 1H), 7.25 (s, 1H), 6.54 (s, 1H), 3.77 (s, 3H), 2.0 (s, 3H). MS *mlz* 196 (M+1).

### Preparation of (5-Methoxy-2-methyl-4-thiocyanato-phenoxy)-acetic acid methyl ester (compound 5C)

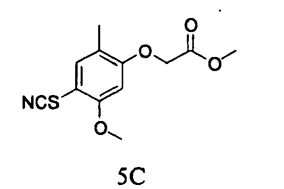

Compound **5C** was prepared from compound **5B** in a manner analogous to compound **3B.** 400 MHz ¹H NMR (DMSO-*d₆*) δ 7.33 (s, 1H), 6.72 (s, 1H), 4.93 (s, 2H), 3.84 (s, 3H), 3.66 (s, 3H), 2.09 (s, 3H); MS *m*/*z* 268 (M+1).

### Preparation of (5-Methoxy-2-methyl-4-thiocyanato-phenoxy)-acetic acid methyl ester (compound 5D)

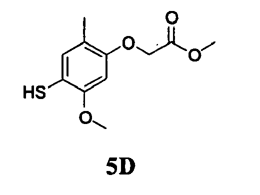

Compound **5D** was prepared from compound **5C** in a manner analogous to compound **3C**. 400 MHz ¹H NMR (DMSO-*d*₆) δ 7.02 (s, 1H), 6.54 (s, 1H), 4.79 (s, 2H), 4.41 (s, 1H), 3.72 (s, 3H), 3.64 (s, 3H), 2.02 (s, 3H); MS *m*/*z* 243 (M+1).

### Preparation of {5-Methoxy-2-methyl-4-[4-methyl-2-(4-trifluoromethyl-phenyl)-thiazol-5-ylmethylsulfanyl]-phenoxy}-acetic acid methyl ester (compound 5E)

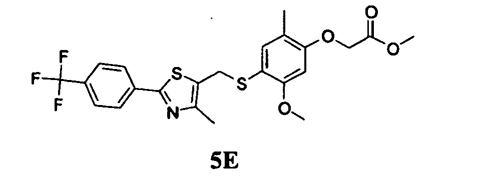

Compound **5E** was prepared from compound **5D** in a manner analogous to compound **3D.** 400 MHz ¹H NMR (DMSO-*d*₆) δ 7.98 (d, 2H, J = 8.4 Hz), 7.76 (d, 2H, *J*=8.4 Hz), 7.02 (s, 1H), 6.54 (s, 1H), 4.84 (s, 2H), 4.20 (s, 2H), 3.72 (s, 3H), 3.64 (s, 3H), 2.16 (s, 3H), 1.98 (s, 3H). ¹H NMR (DMSO-*d*₆) δ MS *m*/*z* 498 (M+1).

### Preparation of {5-Methoxy-2-methyl-4-[4-methyl-2-(4-trifluoromethyl-phenyl)-thiazol-5-ylmethylsulfanyl]-phenoxy}-acetic acid (Reference Compound 5)

Compound 5 was prepared from compound **5E** in a manner analogous to compound **1**. mp 211-213 °C; IR (thin film)·cm⁻¹: 2936, 1719, 1328, 1111; 400 MHz ¹H NMR (DMSO-*d*₆) δ 12.96 (s(br)), 1H), 7.98 (d, 2H, J = 8.4 Hz), 7.76 (d, 2H, *J*=8.4 Hz), 7.02 (d, 1H, J = 0.4 Hz), 6.53 (s, 1H), 4.71 (s, 2H), 4.20 (s, 2H), 3.73 (s, 3H), 2.16 (s, 3H), 1.98 (s, 3H); MS *m*/*z* 484 (M+1). Anal. Calc'd for C₂₂H₂₀F₃NO₄S₂ C, 54.65; H, 4.17; N, 2.90; found: C, 54.68; H, 4.15; N, 2.84.

### Reference Example 6

### Synthesis of {2-Methoxy-5-methyl-4-[4-methyl-2-(4-trifluoromethyl-phenyl)-thiazol-5-ylmethylsulfanyl]-phenoxy}-acetic acid (Reference Compound 6)

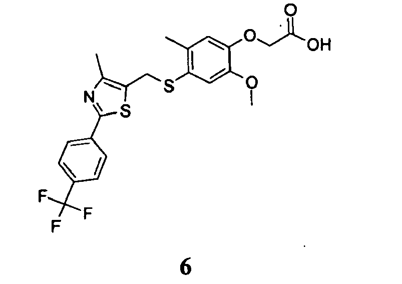

### Preparation of 2-Methoxy-5-methyl-phenol (compound 6A)

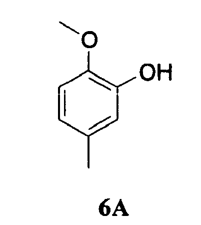

The title compound was prepared from 3-hydroxy-4-methoxy-benzaldehyde in a manner analogous to compound **5A**. 400 MHz ¹H NMR (DMSO-*d*₆) δ 8.73 (s, 1H), 6.71 (d, 1H, *J* = 8.1 Hz), 6.53 (s, 1H), 6.48 (d, 1H, *J* = 8.1 Hz), 3.65 (s, 3H), 2.10 (s, 3H); MS *m*/*z* 139 (M+1).

### Preparation of 2-Methoxy-5-methyl-4-thiocyanato-phenol (compound 6B)

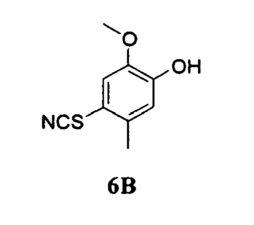

The title compound was prepared from compound **6A** in a manner analogous to compound **3A**. 400 MHz ¹H NMR (DMSO-*d*₆) δ 9.7 (s(br), 1H), 7.15 (s, 1H), 6.78 (1H), 3.73 (s, 3H), 2.3 (s, 3H); MS *m*/*z* 196 (M+1).

### Preparation of (2-Methoxy-5-methyl-4-thiocyanato-phenoxy)-acetic acid methyl ester (compound 6C)

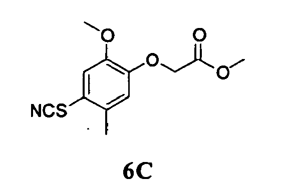

The title compound was prepared from compound **6B** in a manner analogous to compound **3B.** 400 MHz ¹H NMR (DMSO-*d*₆) δ 7.22 (1H), 6.97 (s, 1H), 4.81 (s, 2H), 3.75 (s, 3H), 3.65 (s, 3H), 2.34 (s, 3H); MS *m*/*z* 268 (M+1).

### Preparation of (4-Mercapto-2-methoxy-5-methyl-phenoxy)-acetic acid methyl ester (compound 6D)

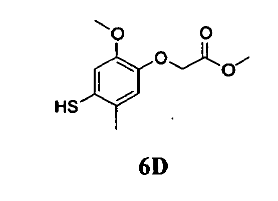

The title compound was prepared from compound **6C** in a manner analogous to compound **3C**. 400 MHz ¹H NMR (DMSO-*d*₆) δ 6.96 (s, 1H), 6.71 (s, 1H), 4.88 (s, 1H), 4.65 (s, 2H), 3.67 (s, 3H), 3.63 (s, 3H), 2.08 (s, 3H); MS *m*/*z* 243 (M+1).

### Preparation of {2-Methoxy-5-methyl-4-[4-methyl-2-(4-trifluoromethyl-phenyl)-thiazol-5-ylmethylsulfanyl]-phenoxy}-acetic acid methyl ester (compound 6E)

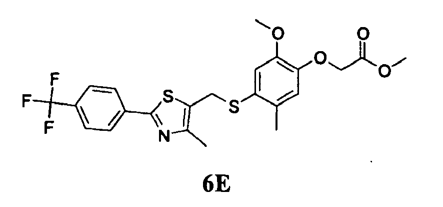

The title compound was prepared from compound **6D** in a manner analogous to compound **1F.** 400 MHz ¹H NMR (DMSO-*d*₆) δ 7.99 (d, 2H, J = 8.4 Hz), 7.76 (d, 2H, J = 8.4 Hz), 6.86 (s, 1H), 6.75 (s, 1H), 4.70 (s, 2H), 4.27 (s, 2H), 3.63 (s, 3H), 3.60 (s, 3H), 2.15 (s, 3H), 2.06 (s, 3H); MS *mlz* 498 (M+1).

### Preparation of {2-Methoxy-5-methyl-4-[4-methyl-2-(4-trifluoromethyl-phenyl)-thiazol-5-ylmethylsulfanyl]-phenoxy}-acetic acid (Reference Compound 6)

The title compound was prepared from compound **6E** in a manner analogous to compound **1.** mp 165-167 °C; IR (thin film) cm⁻¹: 2904, 1747, 1504, 1326; 400 MHz ¹H NMR (DMSO-*d₆*) δ 12.91 (s(br), 1H), 7.99 (d, 2H, J = 8.4 Hz), 7.76 (d, 2H, J = 8.4 Hz), 6.85 (s, 1H), 6.72 (s, 1H), 4.59 (s, 2H), 4.27 (s, 2H), 3.60 (s, 3H), 2.15 (s, 3H), 2.06 (s, 3H); MS *m*/*z* 484 (M+1). Anal. Calc'd for C₂₂H₂₀F₃NO₄S₂ C, 54.65; H, 4.17; N, 2.90; found: C, 54.21; H, 4.03; N, 2.79.

### Reference Example 7

### Preparation of {3,5-Dimethyl-4-[4-methyl-2-(4-trifluoromethyl-phenyl)-thiazol-5-ylmethylsulfanyl]-phenoxy}-acetic acid (Reference Compound 7)

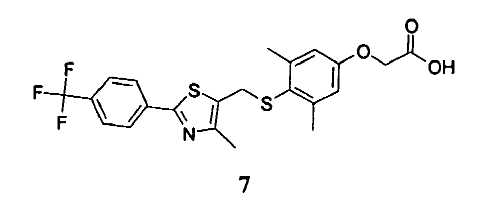

### Preparation of 3,5-Dimethyl-4-thiocyanato-phenol (compound 7A)

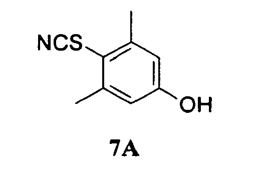

**7A** was prepared from 3,5-dimethylphenol in a similar manner as described for compound **3A**. 400 MHz ¹H NMR (DMSO-*d*₆) δ 9.61 (s, 1H), 6.64 (s, 2H), 2.04 (s, 6H).

### Preparation of (3,5-Dimethyl-4-thiocyanato-phenoxy)-acetic acid methyl ester (compound 7B)

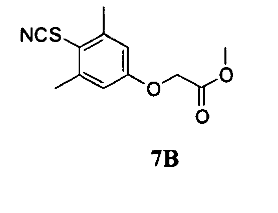

Compound **7B** was prepared from compound **7A** in a similar manner as described for compound **3B** to give 2.0 g (95%) of the title compound pure enough for subsequent use. 400 MHz ¹H NMR (DMSO-*d*₆) δ 6.63 (s, 2H), 4.72 (s, 2H), 3.62 (s, 3H), 2.08 (s, 6H).

### Preparation of (4-Mercapto-3,5-dimethyl-phenoxy)-acetic acid methyl ester (compound 7C)

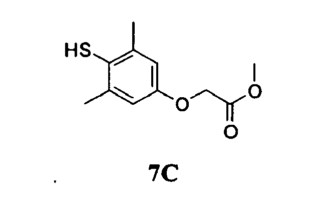

Compound **7C** was prepared from compound **7B** in a similar manner as described for compound **3C** to give, after purification by flash column chromatography (gradient elution: 100% hexanes to 30% EtOAc/hexanes), 0.38 g (21%) of the title compound. 400 MHz ¹H NMR (DMSO-*d*₆) δ 6.66 (s, 2H), 4.68 (s, 2H), 4.20 (s, 1H), 3.63 (s, 3H), 2.23 (s, 6H).

### Preparation of {3,5-Dimethyl-4-[4-methyl-2-(4-trifluoromethyl-phenyl)-thiazol-5-ylmethylsulfanyl]-phenoxy}-acetic acid methyl ester (compound 7D)

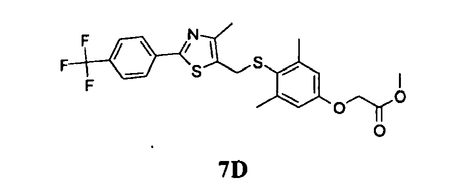

Compound **7C** and 5-chloromethyl-4-methyl-2-(4-trifluoromethyl-phenyl)-thiazole were coupled in a similar manner as described for compound **1F** to provide, after purification by flash column chromatography (gradient elution: 10% EtOAc/hexanes to 35% EtOAc/hexanes), the title compound (0.62 g , 79%) as a yellow solid. mp 113 °C; IR (thin film) cm⁻¹: 1739,1325; 400 MHz ¹H NMR (DMSO-*d*₆) δ 7.99 (d, 2H, *J=* 8.3 Hz), 7.76 (d, 2H, *J*= 8.3 Hz), 6.69 (s, 2H), 4.73 (s, 2H), 4.04 (s, 2H), 3.63 (s, 3H), 2.25 (s, 3H), 1.94 (s, 3H); MS *m*/*z* 482 (M+1); Anal. Calc'd for C₂₃H₂₂F₃N₁O₃S₂ C, 57.37; H, 4.60; N, 2.91; found: C, 57.22; H, 4.55; N, 2.78.

### Preparation of {3,5-Dimethyl-4-[4-methyl-2-(4-trifluoromethyl-phenyl)-thiazol-5-ylmethylsulfanyl]-phenoxy}-acetic acid (Reference Compound 7)

Compound **7D** was saponified in the same manner as described for compound **1F** to give, after recrystalisation from CHCl₃/hexanes, the title compound (0.22 g, 76%) as a pale yellow solid. mp 184-185 °C; IR (thin film) cm⁻¹: 1746, 1320; 400 MHz ¹H NMR (DMSO-*d*₆) δ 7.99 (d, 2H, *J=* 8.3 Hz), 7.76 (d, 2H, *J=* 8.3 Hz), 6.67 (s, 2H), 4.61 (s, 2H), 4.04 (s, 2H), 2.25 (s, 6H), 1.94 (s, 3H); MS *m*/*z* 468 (M+1); Anal. Calc'd for C₂₂H₂₀F₃N₁O₃S₂ C, 56.52; H, 4.31; N, 3.00; found: C, 56.19; H, 4.23; N, 2.91.

### Reference Example 8

### Synthesis of {3-Methoxy-5-methyl-4-[4-methyl-2-(4-trifluoromethyl-phenyl)-thiazol-5-ylmethylsulfanyl]-phenoxy}-acetic acid (Reference Compound 8)

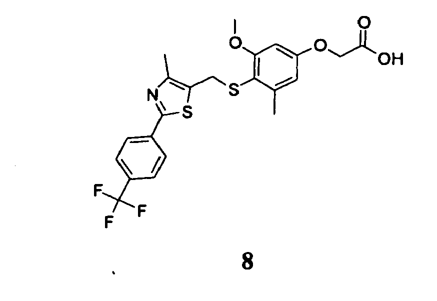

### Preparation of 3-Methoxy-5-methyl-4-thiocyanato-phenol (compound 8A)

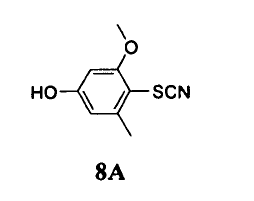

Compound **8A** was prepared from 3-methoxy-5-methyl-phenol in a similar manner as described for compound **3A** to give, after recrystalisation from CHCl₃/hexanes, 1.9 g (13%) of the title compound. 400 MHz ¹H NMR (DMSO-*d*₆) δ 10.14 (s, 1H), 6.39 (s, 2H), 3.79 (s, 3H), 2.35 (s, 3H); MS *m*/*z* 196 (M+1).

### Preparation of (3-Methoxy-5-methyl-4-thiocyanato-phenoxy)-acetic acid methyl ester (compound 8B)

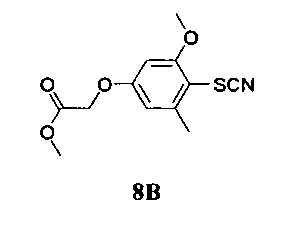

Compound **8B** was prepared from compound **8A** in a similar manner as described for compound **3B** to give 2.2 g (87%) of the title compound pure, enough for subsequent use. 400 MHz ¹H NMR (DMSO-*d*₆) δ 6.60 (s, 2H), 4.84 (s, 2H), 3.85 (s, 3H), 3.66 (s, 3H), 2.40 (s, 3H); MS *m*/*z* 268 (M+1).

### Preparation of (4-Mercapto-3-methoxy-5-methyl-phenoxy)-acetic acid methyl ester (compound 8C)

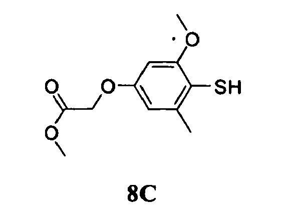

Compound **8C** was prepared from compound **8B** in a similar manner as described for compound **3C** to give, after purification by flash column chromatography (gradient elution: 5% EtOAc/hexanes to 50% EtOAc/hexanes), the title compound (0.82 g, 45%) as a white solid. 400 MHz ¹H NMR (DMSO-*d*₆) δ 6.46 (s, 1H), 6.42 (s, 1H), 4.71 (s, 2H), 4.24 (s, 1H), 3.76 (s, 3H), 3.64 (s, 3H), 2.16 (s, 3H); MS *mlz* 243 (M+1).

### Preparation of {3-Methoxy-5-methyl-4-[4-methyl-2-(4-trifluoromethyl-phenyl)-thiazol-5-ylmethylsulfanyl]-phenoxy}-acetic acid methyl ester (compound 8D)

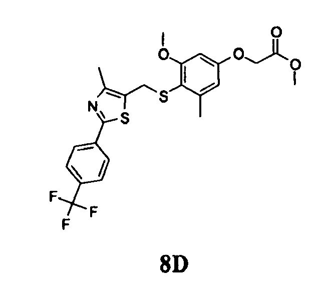

Compound **8C** and 5-chloromethyl-4-methyl-2-(4-trifluoromethyl-phenyl)-thiazole were coupled in a similar manner as described for compound **1F** to provide, after purification by flash column chromatography (gradient elution: 5% EtOAc/hexanes to 50% EtOAc/hexanes), the title compound (0.71 g, 69%) as a yellow solid. IR (thin film) cm⁻¹: 1766, 1325; 400 MHz ¹H NMR (DMSO-*d*₆) δ 7.97 (d, 2H, *J=* 8.1 Hz), 7.75 (d, 2H, J = 8.1 Hz), 6.42 (s, 1H), 6.37 (s, 1H), 4.75 (s, 2H), 4.09 (s, 2H), 3.74 (s, 3H), 3.63 (s, 3H), 2.12 (s, 3H), 2.01 (s, 3H); MS *mlz* 498 (M+1); Anal. Calc'd for C₂₃H₂₂F₃N₁O₄S₂ C, 55.52; H, 4.46; N, 2.82; found: C, 55.30; H, 4.44; N, 2.73.

### Preparation of {3-Methoxy-5-methyl-4-[4-methyl-2-(4-trifluoromethyl-phenyl)-thiazol-5-ylmethylsulfanyl]-phenoxy}-acetic acid (Reference Compound 8)

Compound **8D** was saponified in the same manner as described for compound **1F** to give, after recrystalisation from CHCl₃/hexanes, the title compound (0.14 g, 49%) as a pale yellow solid. mp 164-165 °C; IR (thin film) cm⁻¹: 1726, 1323; 400 MHz ¹H NMR (DMSO-*d*₆) δ 7.97 (d, 2H, *J=* 8.1 Hz), 7.75 (d, 2H, *J =* 8.1 Hz), 6.41 (s, 1H), 6.35 (s, 1H), 4.62 (s, 2H), 4.08 (s, 2H), 3.73 (s, 3H), 2.12 (s, 3H), 2.01 (s, 3H); MS *m*/*z* 484 (M+1); Anal. Calc'd for C₂₂H₂₀F₃N₁O₄S₂ C, 54.65; H, 4.17; N, 2.90; found: C, 54.12; H, 4.06; N, 2.69.

### Reference Example 9

### Synthesis of {2-Isopropyl-5-methyl-4-[4-methyl-2-(4-trifluoromethyl-phenyl)-thiazol-5-ylmethylsulfanyl]-phenoxy}-acetic acid (Reference Compound 9)

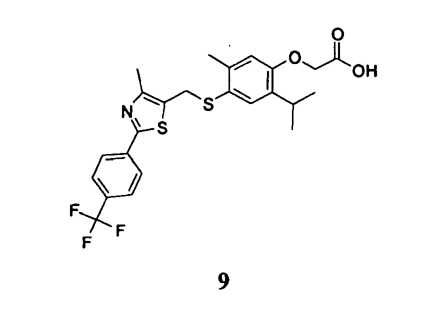

### Preparation of 2-Isopropyl-5-methyl-4-thiocyanato-phenol (compound 9A)

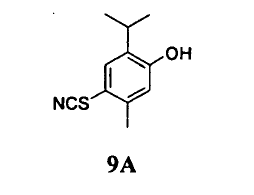

The title compound was prepared in a manner analogous to compound **3A.** 400 MHz ¹H NMR (DMSO-*d*₆) δ 8.9 (s(br), 1H), 7.37 (s, 1H), 6.86 (s, 1H), 3.23 (m, 1H), 2.38 (s, 3H), 1.12 (d, 6H, J = 7.1 Hz); MS *m*/*z* 208 (M+1).

### Preparation of (2-Isopropyl-5-methyl-4-thiocyanato-phenoxy)-acetic acid methyl ester (compound 9B)

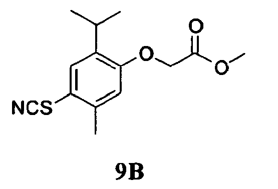

The title compound was prepared from compound **9A** in a manner analogous to compound **3B.** 400 MHz ¹H NMR (DMSO-*d*₆) δ 7.41 (s, 1H), 6.86 (s, 1H), 4.86 (s, 2H), 3.65 (s, 3H), 3.20 (m, 1H), 2.38 (s, 3H), 1.1 (d, 6H, J = 7.2 Hz); MS *m*/*z* 280 (M+1).

### Preparation of (2-Isopropyl-4-mercapto-5-methyl-phenoxy)-acetic acid methyl ester (compound 9C)

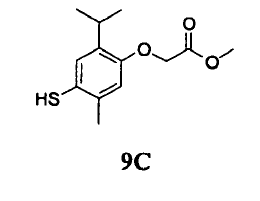

The title compound was prepared from compound **9B** in a manner analogous to compound **3C.** 400 MHz ¹H NMR (DMSO-*d*₆) δ 7.37 (s, 1H), 6.86 (s, 1H), 5.0 (s, 1H), 4.85 (s, 2H), 3.68 (s, 3H), 3.23 (m, 1H), 2.28 (s, 3H), 1.12 (d, 6H, J = 6.8 Hz); MS *m*/*z* 255 (M+1).

### Preparation of {2-Isopropyl-5-methyl-4-[4-methyl-2-(4-trifluoromethyl-phenyl)-thiazol-5-ylmethylsulfanyl]-phenoxy}-acetic acid methyl ester (compound 9D)

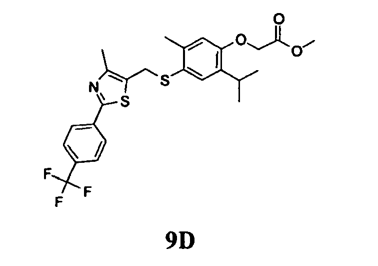

The title compound was prepared from compound **9C** in a manner analogous to example **1F.** 400 MHz ¹H NMR (DMSO-*d*₆) δ 7.98 (d, 2H, J = 8 Hz), 7.76 (d, 2H, J = 8 Hz), 6.94 (s, 1H), 6.74 (s, 1H), 4.76 (s, 2H), 4.17 (s, 2H), 3.63 (s, 3H), 3.1 (m, 1H), 2.24 (s, 3H), 1.93 (s, 3H), .96 (d, 6H, J = 7.2 Hz); MS *m*/*z* 510 (M+1).

### Preparation of {2-Isopropyl-5-methyl-4-[4-methyl-2-(4-trifluoromethyl-phenyl)-thiazol-5-ylmethylsulfanyl]-phenoxy}-acetic acid (Reference Compound 9)

The title compound was prepared from compound **9D** in a manner analogous to compound 1. mp 168-171 °C; IR (thin film) cm⁻¹: 2956, 2583, 1749, 1326; 400 MHz ¹H NMR (DMSO-*d*₆) δ 12.94 (s(br), 1H), 8.00 (d, 2H, J = 8.4 Hz), 7.78 (d, 2H, J = 8.4 Hz), 6.95 (s, 1H), 6.74 (s, 1H), 4.66 (s, 2H), 4.18 (s, 2H), 3.11 (m, 1H), 2.27 (s, 3H), 1.95 (s, 3H), 0.98 (d, 6H, J = 7.2 Hz); MS *m*/*z* 496 (M+1). Anal. Calc'd for C₂₄H₂₄F₃NO₃S₂ C, 58.17; H, 4.88; N, 2.83; found: C, 57.73; H, 4.62; N, 2.75.

### Reference Example 10

### Synthesis of {2,6-Diisopronyl-4-[4-methyl-2-(4-trifluoromethyl-phenyl)-thiazol-5-ylmethylsulfanyl]-phenoxy}-acetic acid (Reference Compound 10)

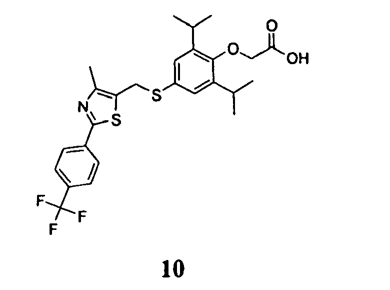

### Preparation of 2,6-Diisopropyl-4-thiocyanato-phenol (compound 10A)

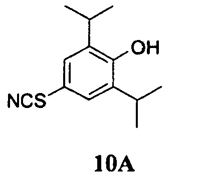

The title compound was prepared in a manner analogous to compound **3A.** 400 MHz ¹H NMR (DMSO-*d*₆) δ 8.8 (s, 1H), 7.22 (s, 2H), 3.25 (m, 2H), 1.1 (d, 12H, J = 7.2 Hz); MS *m*/*z* 236 (M+1).

### Preparation of (2,6-Diisopropyl-4-thiocyanato-phenoxy)-acetic acid methyl ester (compound 10B)

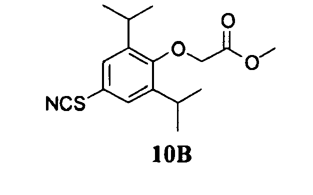

The title compound was prepared from compound **10A** in a manner analogous to compound **3B.** 400 MHz ¹H NMR (DMSO-*d*₆) δ 7.37 (s, 2H), 4.44 (s, 2H), 3.68 (s, 3H), 3.23 (m, 2H), 1.12 (d, 12H, J = 6.8 Hz); MS *m*/*z* 308 (M+1).

### Preparation of (2,6-Diisopropyl-4-mercapto-phenoxy)-acetic acid methyl ester (compound 10C)

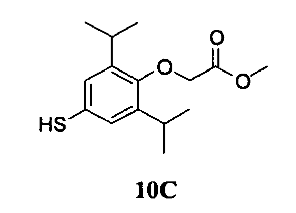

The title compound was prepared from compound **10B** in a manner analogous to compound **3C.** 400 MHz ¹H NMR (DMSO-*d*₆) δ 7.37 (s, 2H), 4.88 (s, 1H) 4.49 (s, 2H), 3.7 (s, 3H), 3.23 (m, 2H), 1.12 (d, 12H, J = 6.8 Hz); MS *m*/*z* 283(M+1).

### Preparation of{2,6-Diisopropyl-4-[4-methyl-2-(4-trifluoromethyl-phenyl)-thiazol-5-ylmethylsulfanyl]-phenoxy}-acetic acid methyl ester (compound 10D)

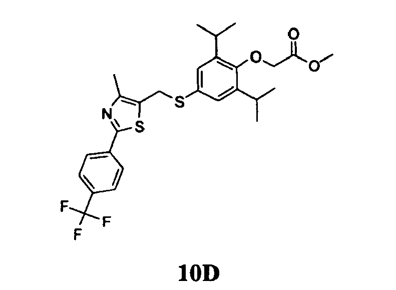

The title compound was prepared from compound **10C** in a manner analogous to compound **1F.** 400 MHz ¹H NMR (DMSO-*d*₆) δ 7.97 (d, 2H, J = 8.4 Hz), 7.76 (d, 2H, 8.4 Hz), 7.00 (s, 2H), 4.3 (m, 4H), 3.66 (s, 3H), 3.13 (m, 2H), 2.04 (3H), 1.01 (d, 12H, J = 7.2 Hz); MS *m*/*z* 538 (M+1).

### Preparation of {2,6-Diisopropyl-4-[4-methyl-2-(4-trifluoromethyl-phenyl)-thiazol-5-ylmethylsulfanyl]-phenoxy}-acetic acid (Reference Compound 10)

The title compound was prepared from compound 10D in a manner analogous to compound **1.** mp 161-163 °C; IR (thin film) cm⁻¹: 2966, 1739, 1438, 1323; 400 MHz ¹H NMR (DMSO-*d*₆) δ 12.93 (s(br), 1H), 7.8 (d, 2H, J = 8 Hz), 7.76 (d, 2H, J = 8 Hz), 7.00 (s, 2H), 4.32 (s, 2H), 4.2 (s, 2H), 3.12 (m, 2H), 2.03 (s, 3H), 1.01 (d, 12H, J = 7.2 Hz); MS *m*/*z* 524 (M+1). Anal. Calc'd for C₂₆H₂₈F₃NO₃S₂ C, 59.64; H, 5.39; N, 2.67; found: C, 58.82; H, 5.54; N, 2.48.

### Reference Example 11

### Synthesis of 2-Methyl-2-{4-[4-methyl-2-(4-trifluoromethyl-phenyl)-thiazol-5-ylmethylsulfanyl]-phenyl}-propionic acid (Reference Compound 11)

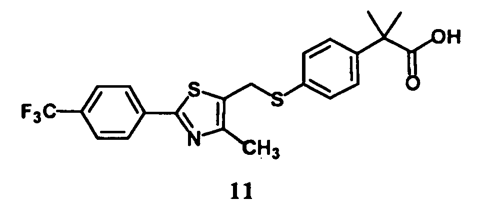

### Preparation of 2-(4-Benzyloxy-phenyl)-2-methyl-propionic acid methyl ester (11A)

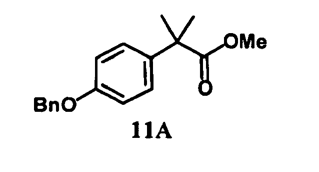

1). (4-Benzyloxy-phenyl)-acetic acid (10g, 41 mmol) was dissolved in MeOH (100 mL) and was then treated with H₂SO₄ (5 mL). The reaction mixture was refluxed overnight. MeOH was evaporated and the residue was diluted with water and ether. Layers were separated and the aqueous layer was extracted with ether (2x30mL). The combined organics were dried with MgSO₄ and condensed to afford the product (10.24g, 97%) as white crystals.
   MS: 257 (M+1)⁺
2). To a suspension ofNaH (2.34g, 58.5 mmol) in THF (150 mL) was added a solution of the above ester (5.0g, 19.8 mmol) in THF (50 mL) followed by dropwise addition of methyl iodide (6.65g, 47 mmol). The mixture was stirred at RT overnight. Water (100 mL) was added and the layers were separated. The aqueous layer was extracted with ether (2x50mL). The combined organics was dried with MgSO₄ and evaporated to give a yellow solid as the desired product **11A** (4.37g, 79%).
   MS: 285 (M+1)⁺

### Preparation of 2-(4-Dimethylthiocarbamoyloxy-phenyl)-2-methyl-propionic acid methyl ester (compound 11B)

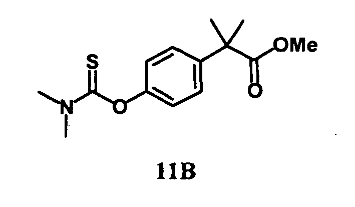

1). Compound **11A** (2.08g, 7.31 mmol) was dissolved in MeOH (50 mL) and was subjected to hydrogenation conditions catalyzed by 20% Pd/C. After 17 h, the reaction mixture was filtered and the filtrate was evaporated to give the desired product as a white solid (1.85g, 100%)
2). The phenol compound obtained from above (1.0g, 5.15 mmol) was dissolved in dioxane (10 mL) followed by addition of Et₃N (1.04g, 10.3 mmol), DMAP (63mg, 0.52 mmol), and dimethyl thiocarbamoyl chloride (0.76g, 6.18 mmol). The reaction mixture was refluxed overnight. After cooling down to RT, the reaction mixture was diluted with EtOAc (100 mL) and H₂O (100 mL). Layers were separated and the aqueous layer was extracted with EtOAc (2x50mL). Combined organics was dried over MgSO₄ and condensed to give the desired product **11B** as a brown oil (1.61g, 100%)
   MS: 282 (M+1)⁺

### Preparation 2-(4-Mercapto-phenyl)-2-methyl-propionic acid methyl ester (compound 11C)

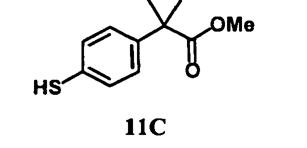

1). Compound **11B** (1.61g, 5.15 mmol) in tetradecane (20 mL) was heated to reflux overnight. After cooling down to RT, solvent was decanted out and the residue was washed with hexane. It was then taken up to EtOAc (100 mL) and washed with H₂O, dried over MgSO₄ and condensed to give the desired product as a tan solid (0.15g). More crystals (0.39g) were collected from the tetradecane solution that was first decanted. Total yield of the reaction was 34% (0.54g).
   MS: 282 (M+1)⁺
2). The above product (0.54g, 1.92 mmol) was immediately dissolved in MeOH (5 mL) and was treated with NaOMe (0.114g, 2.11mmol). The reaction mixture was heated to reflux overnight and MS indicated the presence of the desired product **11C.** The reaction was cooled to RT and used for next step without purification.
   MS: 209 (M-1)⁺

### Preparation 2-Methyl-2-{4-[4-methyl-2-(4-trifluoromethyl-phenyl)-thiazol-5-ylmethylsulfanyl]-phenyl}-propionic acid (Reference Compound 11)

1). To the above mixture was added a solution of the chloride **1B** (0.25g, 0.86 mmol) in MeOH (10 mL) and the resulting solution was heated to reflux overnight. MeOH was then removed via rotavap and the residue was dissolved in EtOAc (50mL) and washed with H₂O. Solvent was dried over MgSO₄ and condensed to give the crude product as an oil. It was purified by silica gel chromatography eluted with 10% EtOAc/Hexanes to afford the pure product (0.22g, 24%) as a yellow oil.
   MS: 466 (M+1)⁺
2). The above methyl ester (0.22g, 0.4 mmol) dissolved in THF (10 mL) and treated with LiOH.H₂O (0.1 g, 2 mmol). After reflux overnight, the reaction mixture was cooled to RT and neutralized with aq. HCl (1N). Solvent was removed and the solid was filtered off and washed with EtOAc. The filtrate was dried over MgSO₄ and condensed to give the desired product compound 11 as a yellow solid (0.17g, 96%).
   MS: 452 (M+1)⁺, CHN: Calc'd: C 58.52, H 4.46, N 3.10; Found: C 58.65, H 4.33, N 3.04.

### Example 12

### Synthesis of 2-{4-[4-Methyl-2-(4-trifluoromethyl-phenyl)-thiazol-5-ylmethylsulfanyl]-phenyl}-cyclopropanecarboxylic acid (Compound 12)

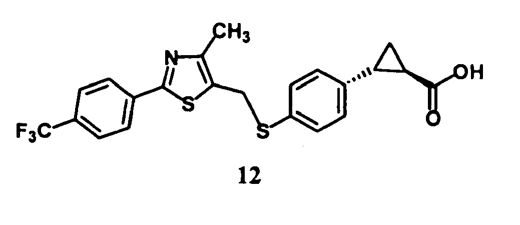

### Preparation of 2-Phenyl-cyclopropanecarboxylic acid methyl ester (12A)

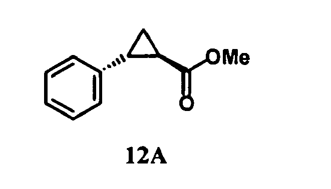

2-Phenyl-cyclopropanecarboxylic acid (5g, 30.86 mmol) was dissolved in MeOH (100 mL) and was then treated with H₂SO₄ (2 mL). The reaction mixture was refluxed overnight. MeOH was evaporated and the residue was diluted with water and ether. Layers were separated and the aqueous layer was extracted with ether (2x30mL). The combined organics were dried with MgSO₄ and condensed to afford the product compound **12A** (5.3g, 97%) as white crystals.
MS: 177 (M+1)⁺

### Preparation of 2-(4-Chlorosulfonyl-phenyl)-cyclopropanecarboxylic acid methyl ester (compound 12B)

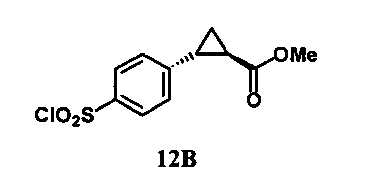

Chlorosulfonic acid (10 mL) was cooled to 0°C. Then compound **12A** (2.0g, 11.36 mmol) was added over 30 min. The mixture was stirred at RT for 3h and was poured into ice (100g). The cloudy solution was extracted with ether (2x100mL). The extracts were dried with magnesium sulfate and concentrated to give a brown oil which was passed through a short pad of silica gel to afford the desired product **12B** (2.96g, 95%) as white plates.
NMR (¹H, CDCl₃): δ 7.95 (2H, m), 7.29 (2H, m), 3.74 (3H, s), 2.68 (1H, m), 2.03 (1H, m), 1.78 (1H, m), 1.41 (1H, m).

### Preparation of 2-(4-Mercapto-phenyl)-cyclopropanecarboxylic acid methyl ester (compound 12C)

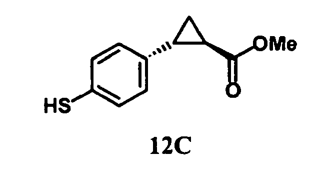

The above product **12B** was refluxed with tin powder (4.4g, 37.7 mmol) in MeOH (10 mL) and 4M HCl/dioxane (10 mL). After 3h, the reaction mixture was poured into ice with CH₂Cl₂ (100 mL). The phases were separated and the aqueous layer was extracted with CH₂Cl₂ (2x50mL). The combined organic layers were dried with magnesium sulfate, filtered and evaporated to give the thiol compound **12C** as a yellow oil (1.06g, 70%).
MS: 207 (M-1)⁺

### Preparation of 2-[4-(4-Methyl-2-p-tolyl-thiazol-5-ylsulfanyl)-phenyl]-cyclopropanecarboxylic acid methyl ester (compound 12D)

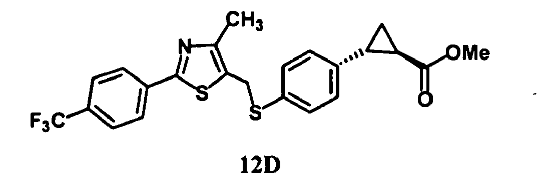

Compound **12C** (1.06g, 5.09 mmol) was dissolved in acetonitrile (80 mL) with the chloride **1B** (1.3g, 4.4 mmol) and Cs₂CO₃ (3.3g, 1.07 mmol). The reaction mixture was stirred at RT overnight. Ether (50 mL) and H₂O were added and stirring was continued for another 5 min. The layers were separated and the aqueous layer was extracted with ether (2x100 mL). The combined organics was dried over MgSO₄ and concentrated to an oil. The crude product was purified by column chromatography eluted with EtOAc and hexanes to give the desired product, compound **12D** as a thick yellow oil (1.1g , 47%).
MS: 464 (M+1)⁺

### Preparation of 1-{4-[4-Methyl-2-(4-trifluoromethyl-phenyl)-thiazol-5-ylmethylsulfanyl]-phenyl}-cyclopropanecarboxylic acid (Compound 12)

To the solution of the above methyl ester, compound **12D** (0.38g, 0.82 mmol) in MeOH (7mL) and THF (7mL) was added LiOH.H₂O (69mg, 1.64 mmol). After refluxing overnight, the solution was cooled to RT and solvents were removed by rotavap. The residue was dissolved in water and neutralized with 1N HCl. The cloudy solution was extracted with EtOAc (3x50 mL) and the extracts were dried with MgSO₄, and concentrated. The crude product was purified by chromatography to afford a yellow solid, which was further washed with ether to yield the desired product as yellowish crystals (116mg, 31%).
MS: 450 (M+1)⁺.

### Example 13

### Synthesis of 1-{4-[4-Methyl-2-(4-tritluoromethyl-phenyl)-thiazol-5-ylmethylsulfanyl]-phenyl}-cyclopropanecarboxylic acid (Compound 13)

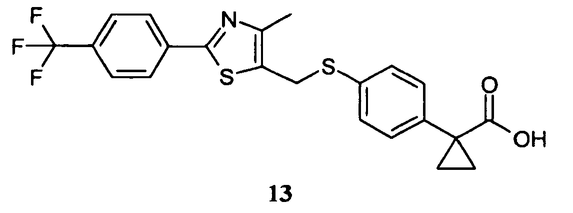

### Preparation of 1-Phenyl-cyclopropanecarboxylic acid methyl ester (compound 13A)

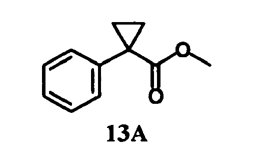

Compound **13A** was made following the procedure in Example **12A,** by replacing 2-Phenyl-cyclopropanecarboxylic acid with compound 1-Phenyl-cyclopropanecarboxylic acid. Compound **13A** was prepared in quantitative yield. MS: 177(M+1)⁺.

### Preparation of 1-(4-Chlorosulfonyl-phenyl)-cyclopropanecarboxylic acid methyl ester (compound 13B)

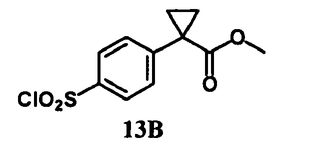

Compound **13B** was prepared analogously to compound **12B.** 57% yield. MS: 239 (M-Cl)⁺.

### Preparation of 1-(4-Mercapto-phenyl)-cyclopropanecarboxylic acid methyl ester (compound 13C)

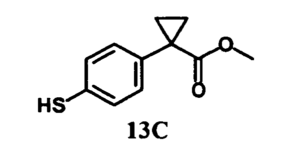

Compound **13C** was prepared analogously to compound **12C.**

### Preparation of 1-{4-[4-Methyl-2-(4-trifluoromethyl-phenyl)-thiazol-5-ylmethylsulfanyl]-phenyl}-cyclopropanecarboxylic acid methyl ester (compound 13D):

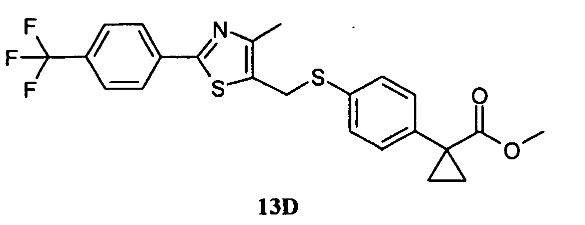

Compound **13D** was prepared analogously to compound **12D** using the crude product compound **13C.** Yield was 12% in 2 steps. MS for 15D: 464 (M+1)⁺

### Preparation of 1-{4-[4-Methyl-2-(4-trifluoromethyl-phenyl)-thiazol-5-ylmethylsulfanyl]-phenyl}-cyclopropanecarboxylic acid (Compound 13):

Compound **13** was prepared analogously to compound **12.** Compound **13** was prepared in 54% yield. MS: 450 (M+1)⁺.

### Example 14

### Synthesis of 1-{4-[4-Methyl-2-(4-trifluoromethyl-phenyl)-thiazol-5-ylmethylsulfanyl]-phenyl}-cyclopentanecarboxylic acid (Compound 14)

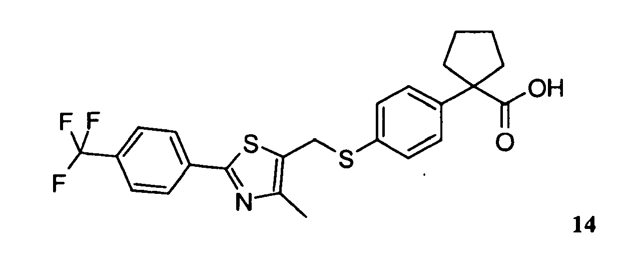

### Preparation of 1-Phenyl-cyclopentanecarboxylic acid methyl ester (compound 14A)

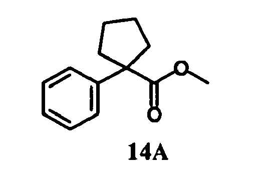

Compound **14A** was made following the procedure in Example **12A,** by replacing 2-Phenyl-cyclopropanecarboxylic acid with 1-Phenyl-cyclopentanecarboxylic acid. Compound **14A** was prepared in quantitative yield. MS: 205 (M+1)⁺.

### Preparation of 1-(4-Chlorosulfonyl-phenyl)-cyclopentanecarboxylic acid methyl ester (compound 14B)

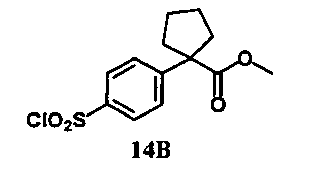

Compound **14B** was prepared analogously to compound **12B.** 50% yield. MS: 267 (M-Cl)⁺:

### Preparation of 1-(4-Mercapto-phenyl)-cyclopentanecarboxylic acid methyl ester(compound 14C)

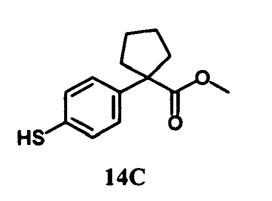

Compound **14C** was prepared analogously to compound **12C.**

### Preparation of 1-{4-[4-Methyl-2-(4-trifluoromethyl-phenyl)-thiazol-5-ylmethylsulfanyl]-phenyl}-cyclopentanecarboxylic acid methyl ester(compound 14D)

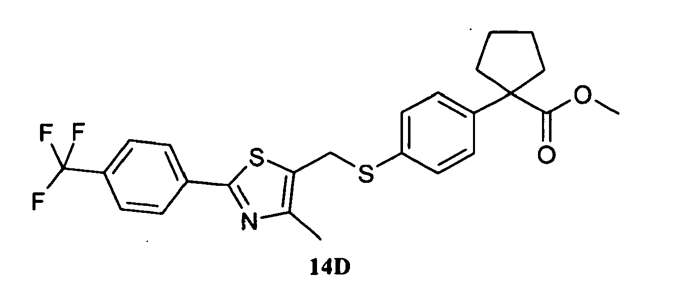

Compound **14D** was prepared analogously to compound **12D** using the crude product compound **14C.** Yield was 31% in 2 steps. MS for **14D:** 492 (M+1)⁺

### Preparation of 1-{4-[4-Methyl-2-(4-trifluoromethyl-phenyl)-thiazol-5-ylmethylsulfanyl]-phenyl}-cyclopentanecarboxylic acid (Compound 14)

Compound **14** was prepared analogously to compound **12** in 80% yield. MS: 478 (M+1)⁺.

### Reference Example 15

### Synthesis of 4-Methyl-4-{4-[4-methyl-2-(4-trifluoromethyl-phenyl)-thiazol-5-ylmethylsulfanyl]-phenyl}-pentanoic acid (Reference Compound 15)

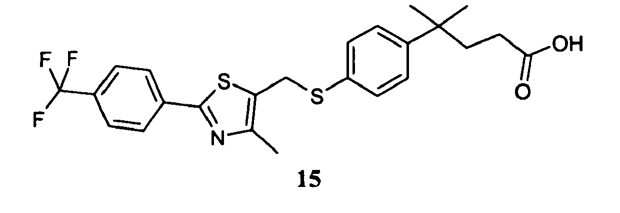

### Preparation of 4-Methyl-4-phenyl-pentanoic acid methyl ester (compound 15A)

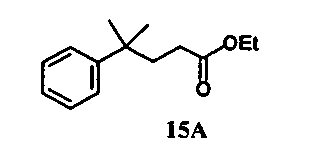

Compound **15A** was prepared by hydrogenation of the corresponding crotonate catalyzed by Pd/C (10%) in 93% yield. MS: 221 (M+1)⁺.

### Preparation of 4-(4-Chlorosulfonyl-phenyl)-4-methyl-pentanoic acid ethyl ester(compound 15B)

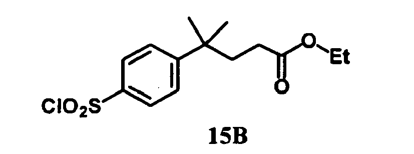

Compound **15B** was prepared analogously to compound **12B** in 85% yield. MS: 283 (M-Cl)⁺.

### Preparation of 4-(4-Mercapto-phenyl)-4-methyl-pentanoic acid ethyl ester (compound 15C)

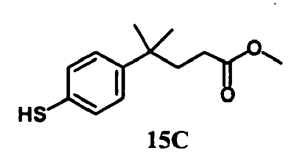

Compound **15C** was prepared analogously to compound **12C.**

### Preparation of 4-Methyl-4-{4-[4-methyl-2-(4-trifluoromethyl-phenyl)-thiazol-5-ylmethylsulfanyl]-phenyl}-pentanoic acid ethyl ester (compound 15D)

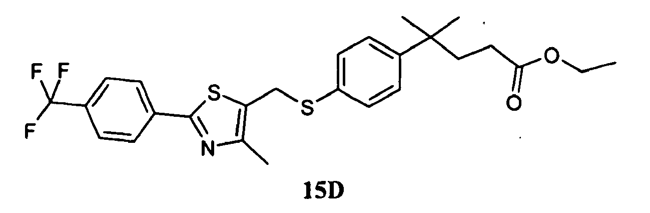

Compound **15D** was prepared analogously to compound **12D** using the crude product compound **15C.** Yield was 2 1 % in 2 steps. MS for 17D: 508 (M+1)⁺.

### Preparation of 4-Methyl-4-{4-[4-methyl-2-(4-trifluoromethyl-phenyl)-thiazol-5-ylmethylsulfanyl]-phenyl}-pentanoic acid (Reference Compound 15)

Compound **15** was prepared analogously to compound **12.** Compound **15** was prepared in 38% yield. MS: 480 (M+1)⁺.

### Reference Example 16

### Synthesis of 3-{4-[4-Methyl-2-(4-trifluoromethyl-phenyl)-thiazol-5-ylmethylsulfanyl]-phenoxy}-provionic acid (Reference Compound 16)

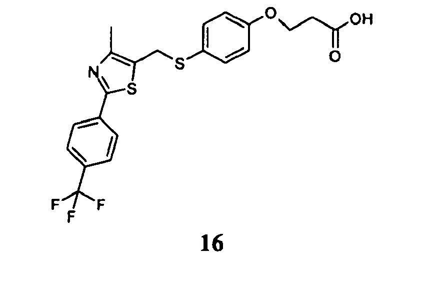

### Preparation of 3-Phenoxy-propionic acid methyl ester (compound 16A)

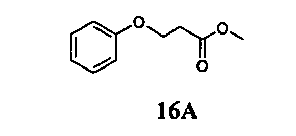

Compound **16A** is commercially available from Aldrich of Milwaukee, Wisconsin.

### Preparation of 3-(4-Chlorosulfonyl-phenoxy)-propionic acid methyl ester (compound 16B)

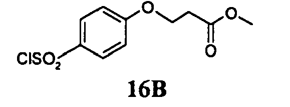

Compound **16B** was prepared analogously to compound **12B.** 69% yield.

### Preparation of 3-(4-Mercapto-phenoxy)-propionic acid methyl ester (compound 16C)

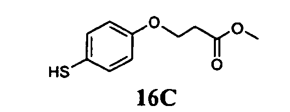

Compound **16C** was prepared analogously to compound **12C.**

### Preparation of 3-{4-[4-Methyl-2-(4-trifluoromethyl-phenyl)-thiazol-5-ylmethylsulfanyl]-phenoxy}-propionic acid methyl ester (compound 16D)

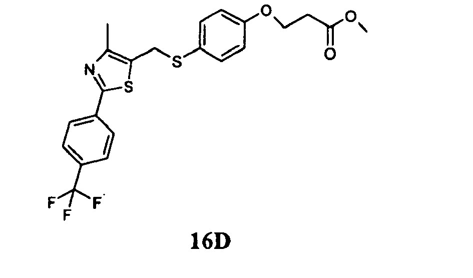

Compound **16D** was prepared analogously to compound **12D** using the crude product compound **16C.** Compound **16D** was prepared in 49% yield. MS: 211 (M-1)⁺.

### Synthesis of 3-{4-[4-Methyl-2-(4-trifluoromethyl-phenyl)-thiazol-5-ylmethylsulfanyl]-phenoxy}-propionic acid (Reference Compound 16)

Compound **16** was prepared by treating the ester with concentrated HBr in 17% yield.
MS: 454 (M+1)⁺.

### Reference Example 17

### Synthesis of 2-{3-Methoxy-4-[4-methyl-2-(4-trifluoromethyl-phenyl)-thiazol-5-ylmethylsulfanyl]-phenyl}-2-methyl-propionic acid (Reference Compound 17)

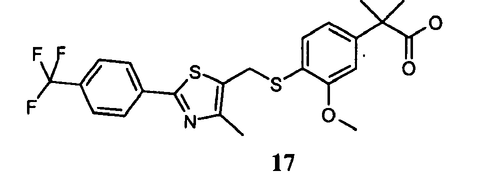

### Preparation of 2-(3-Methoxy-phenyl)-2-methyl-propionic acid methyl ester (Compound 17A)

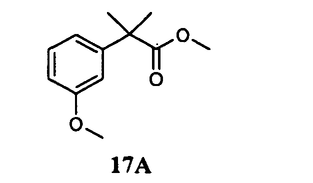

**17A** was prepared analogously to compound **11A.** MS: 209 (M+1)⁺.

### Preparation of 2-(4-Chlorosulfonyl-3-methoxy-phenyl)-2-methyl-propionic acid methyl ester (Compound 17B)

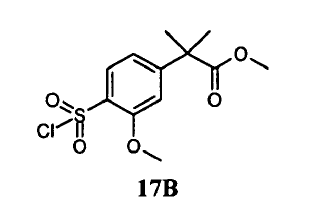

**17B** was prepared analogously to compound **12B.** 39% yield. MS: 271 (M-Cl)⁺.

### Preparation of 2-(4-Mercapto-3-methoxy-phenyl)-2-methyl-propionic acid methyl ester (Compound 17C)

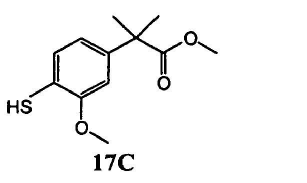

**17C** was prepared analogously to compound **12C.** Used as unpurified oil. MS: 239 (M-1)⁺.

### Preparation of 2-{3-Methoxy-4-[methyl-2-(4-trifluoromethyl-phenyl)-thiazol-5-ylmethylsulfanyl]-2-methyl-propionic acid methyl ester (Compound 17D)

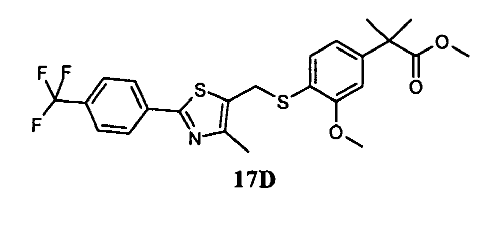

Compound **17D** was prepared analogously to compound **12D.** Yield was 12% after flash column purification. MS: 496 (M+1)⁺.

### Preparation of 2-{3-Methoxy-4-[4-methyl-2-(4-trifluoromethyl-phenyl)-thiazol-5-ylmethylsulfanyl]-phenyl}-2-methyl-propionic acid (Reference Compound 17)

Compound **17** was prepared analogously to compound **12.** Compound **17** was prepared in 50% yield. MS: 482 (M+1)⁺.

### Reference Example 18

### Synthesis of 3-Methyl-3-{4-[4-methyl-2-(4-trifluoromethyl-phenyl)-thiazol-5-ylmethylsulfanyl]-phenyl}-butyric acid (Reference Compound 18)

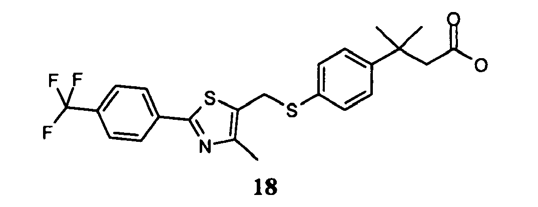

### Preparation of 3-Methyl-3-phenyl-butyric acid methyl ester (Compound 18A

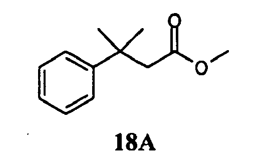

**18A** was prepared analogously to compound **11A.** 96% yield. MS: 179 (M+1)⁺.

### Preparation of 2-Methyl-2-phenyl-propan-1-ol (Compound 18B)

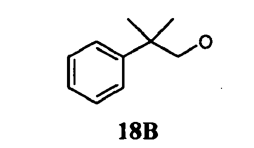

**18B** was prepared analogously to compound **1A.** Quantitative yield. MS: 133 (M-H₂O)⁺.

### Preparation of 3-Methyl-3-phenyl-butyronitrile (Compound 18C)

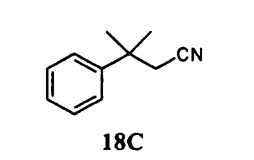

Methane sulfonyl chloride was added to a stirred solution of compound **18B** dissolved in CH₂Cl₂ and triethyl amine at 0°C. The mixture was stirred at RT for 4 hr. It was then diluted with CH₂Cl₂ and washed with 1 x sat. NH₄Cl, 1 x brine, dried (MgSO₄) and the solvent removed in vacuo to give 2.68 g (88%) of the methanesulfonated compound.
The methanesulfonated intermediate obtained from above was dissolved in DMSO and added with sodium cyanide. The mixture was heated at 100°C overnight. After cooling down to RT, the reaction mixture was diluted with water and layered with ether. The layers were separated and the aqueous layer extracted with ether. The combined organics were dried over MgSO₄ and condensed to give the crude product as an oil. It was purified by silica gel chromatography eluted with 10% EtOAc/Hexanes to afford the desired compound **18C** as a clear oil (0.26 g, 26%).
MS: 160 (M+1)⁺.

### Preparation of 4-(2-Cyan-1,1-dimethyl-ethyl)-benzenesulfonyl chloride (Compound 18D)

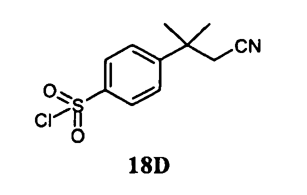

**18D** was prepared analogously to compound **12B.** 71% yield. MS: 222 (M-Cl)⁺.

### Preparation of 3-(4-Mercapto-phenyl)-3-methyl-butyronitrile (Compound 18E)

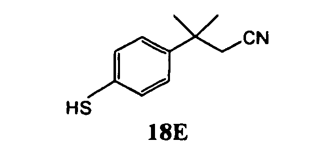

**18E** was prepared analogously to compound **12C.** 92% yield. MS: 252 (M+1)⁺.

### Preparation of 3-Methyl-3-{4-[4-methyl-2-(4-trifluoromethyl-phenyl)-thiazol-5-ylmethylsulfanyl]-phenyl}-butyronitrile (Compound 18F)

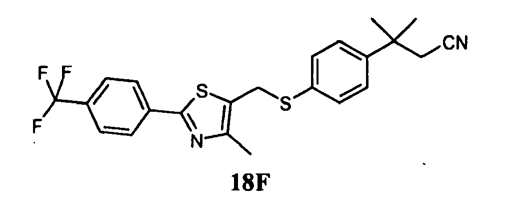

Compound **18F** was prepared analogously to compound **12D.** Yield was 20% after flash column purification. MS: 447 (M+1)⁺,

### Preparation of 3-Methyl-3-{ 4-[4-methyl-2-(4-trifluoromethyl-phenyl)-thiazol-5-ylmethylsulfanyl]-phenyl}-butyric acid (Reference Compound 18)

Compound **18F** stirred in 2-methoxy ethanol was added with 30 % NaOH solution. The mixture was heated at reflux overnight. The solvent was removed in vacuo to give the crude product as a dark oil. It was purified by silica gel chromatography eluted with 15% EtOAc/Hexanes to afford the desired compound **18** as a yellow solid (0.014 g, 8%). MS: 466 (M+1)⁺.

### Reference Example 19

### Synthesis of 3-{2,4-Dimethoxy-5-[4-methyl-2-(4-trifluoromethyl-phenyl)-thiazol-5-ylmethylsulfanyl]-phenyl}-propionic acid (Reference Compound 19)

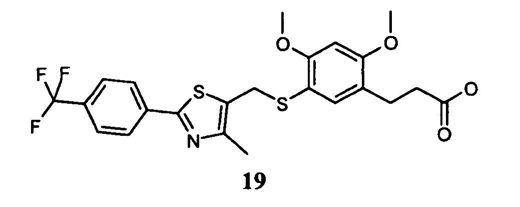

### Preparation of 3-(2,4-Dimethoxy-phenyl)-propionic acid ethyl ester(Compound 19A)

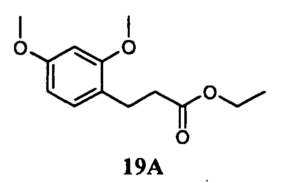

2,4-Dimethoxybenzaldehyde in THF was added to a stirred solution of sodium hydride and triethyl phosphonoacetate in THF at 0°C. After 15 min., water was added and the mixture was stirred at RT for 2 hr. The layers were separated and the aqueous layer was extracted with ether. The combined organics was dried with MgSO₄ and evaporated to a yellow oil in quantitative yield. MS: 237 (M+1)⁺.

Compound **19A** was then prepared by hydrogenation of the unsaturated oil catalyzed by Pd/C (10%) in 88% yield. MS: 239 (M+1)⁺.

### Preparation of 3-(5-Chlorosulfonyl-2,4-dimethoxy-phenyl)-propionic acid ethyl ester (Compound 19B)

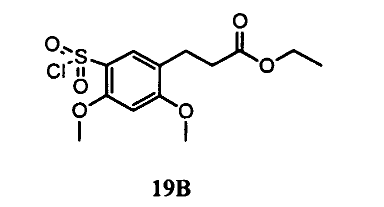

**19B** was prepared analogously to compound **12B.** 52% yield. MS: 301 (M-Cl)⁺.

### Preparation of 3-(5-Mercapto-2,4-dimethoxy-phenyl)-propionic acid ethyl ester (Compound 19C)

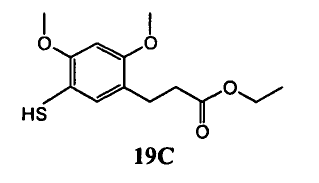

**19C** was prepared analogously to compound **12C.** Used as unpurified oil. MS: 271 (M+1)⁺.

### Preparation of 3-{2,4-Dimethoxy-5-[4-methyl-2-(4-trifluoromethyl-phenyl)-thiazol-5-ylmethylsulfanyl]-phenyl}-propionic acid ethyl ester (Compound 19D)

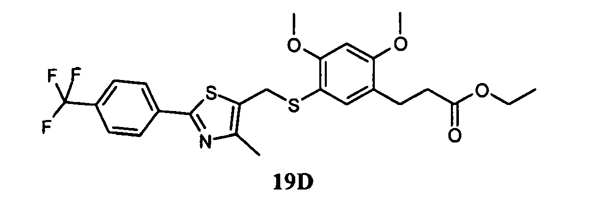

Compound **19D** was prepared analogously to compound **12D.** Yield was 45% after flash column purification. MS: 526 (M+1)⁺.

### Preparation of 3-{2,4-Dimethoxy-5-[4-methyl-2-(4-trifluoromethyl-phenyl)-thiazol-5-ylmethylsulfanyl]-phenyl}-propionic acid (Reference Compound 19)

Compound **19** was prepared analogously to compound **12.** Compound **19** was prepared in 54% yield. MS: 498 (M+1)⁺.

### Reference Example 20

### Synthesis of 3-{2,5-Dimethoxy-4-[4-methyl-2-(4-trifluoromethyl-phenyl)-thiazol-5-ylmethylsulfanyl]-phenyl}-propionic acid (Reference Compound 20)

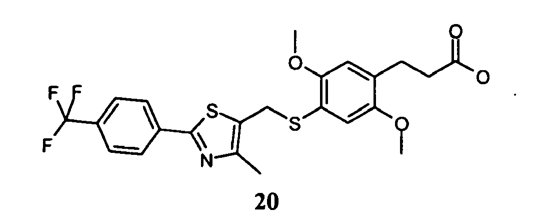

### Preparation of 3-(2,5-Dimethoxy-phenyl)-propionic acid ethyl ester (Compound 20A)

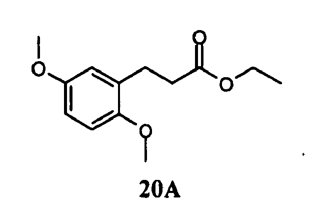

**20A** was prepared analogously to compound **19A.** 94% yield. MS: 239 (M+1)⁺.

### Preparation of 3-(4-Chlorosulfonyl-2,5-dimethoxy-phenyl)-propionic acid ethyl ester (Compound 20B)

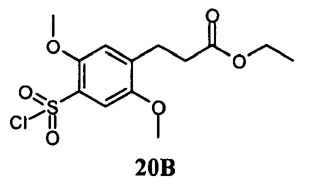

**20B** was prepared analogously to compound **12B.** 88% yield. MS: 301 (M-Cl)⁺.

### Preparation of 3-(4-Mereapto-2,5-dimethoxy-phenyl)-propionic acid ethyl ester (Compound 20C)

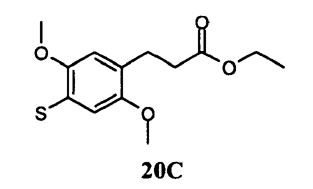

**20C** was prepared analogously to compound **12C.** Used as unpurified oil. MS: 271 (M+1)⁺.

### Preparation of 3-{2,5-Dimethoxy-4-[4-methyl-2-(4-tritluoromethyl-phenyl)-thiazol-5-ylmethylsulfanyl]-phenyl}-propionic acid ethyl ester (Compound 20D)

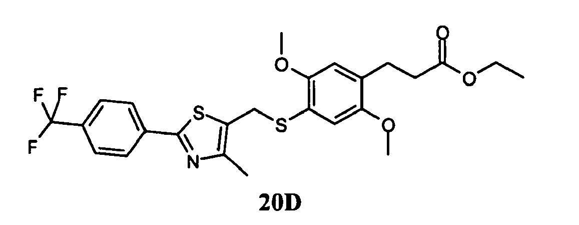

Compound **20D** was prepared analogously to compound **12D.** Yield was 26% after flash column purification. MS: 526 (M+1)⁺.

### Preparation of 3-{2,5-Dimethoxy-4-[4-methyl-2-(4-trifluoromethyl-phenyl)-thiazol-5-ylmethylsulfanyl]-phenyl}-propionic acid (Reference Compound 20)

Compound **20** was prepared analogously to compound **12.** Compound **20** was prepared in 98% yield. MS: 498 (M+1)⁺.

### Reference Example 21

### Synthesis of {2,4-Dimethoxy-5-[4-methyl-2-(4-trifluoromethyl-phenyl)-thiazol-5-ylmethylsulfanyl]}-acetic acid (Reference Compound 21)

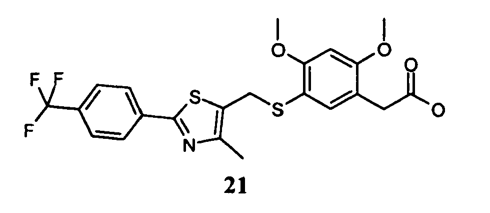

### Preparation of (2,4-Dimethoxy-phenyl)-acetic acid methyl ester (Compound 21A)

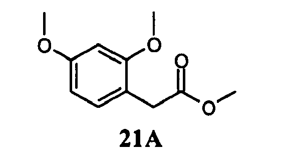

**21A** was prepared analogously to compound **12A.** Quantitative yield. MS: 211 (M+1)⁺.

### Preparation of (5-Chlorosulfonly-2, 4-dimethoxy-phenyl)-acetic acid methyl ester (Compound 21B)

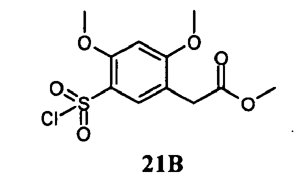

**21B** was prepared analogously to compound **12B.** 83% yield. MS: 307 (M+1)⁺.

### Preparation of (5-Mercapto-2, 4-dimethoxy-phenyl)-acetic acid methyl ester (Compound 21C)

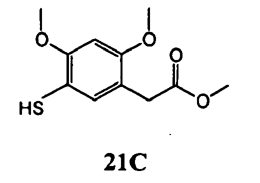

**21C** was prepared analogously to compound **12C.** Used as unpurified oil. MS: 243 (M+1)⁺.

### Preparation of {2,4-Dimethoxy-5-[4-methyl-2-(4-trifluoromethyl-phenyl)-thiazol-5-ylmethylsulfanyl]-acetic acid methyl ester (Compound 21D)

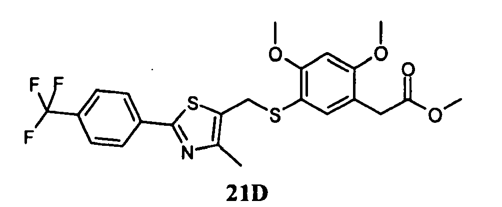

Compound **21D** was prepared analogously to compound **12D.** Yield was 25% after flash column purification. MS: 498 (M+1)⁺.

### Preparation of {2,4-Dimethoxy-5-[4-methyl-2-(4-trifluoromethyl-phenyl)-thiazol-5-ylmethylsulfanyl]-acetic acid (Reference Compound 21)

Compound **21** was prepared analogously to compound **12.** Compound **21** was prepared in 82% yield. MS: 484 (M+1)⁺.

### Reference Example 22

### Synthesis of 3-{4-[4-Methtyl-2-(4-trifluoromethyl-phenyl)-thiazol-5-ylmethoxy]-phenyl}-2-pyrrol-1-yl-propionic acid (Reference Compound 22)

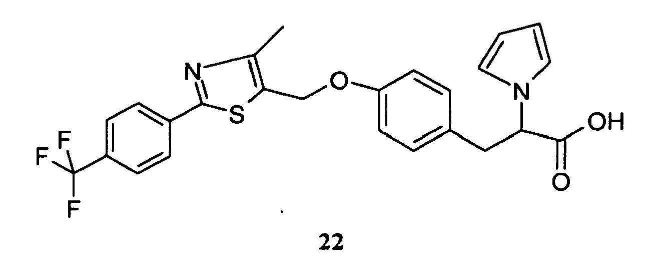

### Preparation of 3-{4-[4-Methtyl-2-(4-trifluoromethyl-phenyl)-thiazol-5-ylmethoxy]-phenyl}-2-pyrrol-1-yl-propionic acid methyl ester (Compound 22A)

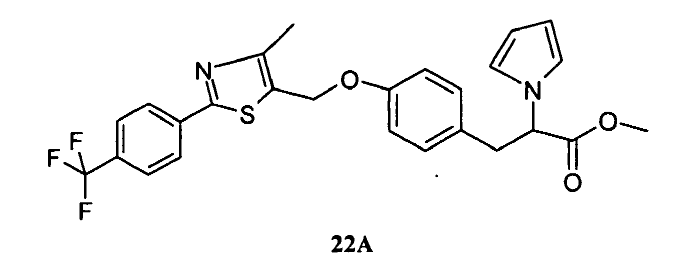

Compound **22A** was prepared according to the method of example **1F** utilizing 3-(4-hydroxy-phenyl)-2-pyrrol-1-yl-propionic acid methyl ester. MS: 501 (M+1)⁺.

### Preparation of 3-{4-[4-Methtyl-2-(4-trifluoromethyl-phenyl)-thiazol-5-ylmethoxyl-phenyl}-2-pyrrol-1-yl-propionic acid (Reference Compound 22)

Compound **22** was prepared according to the method of example **1** utilizing compound **22A.** MS: 487 (M+1)⁺.

### Reference Example 23

### Synthesis of 3-{4-[4-Methtyl-2-(4-trifluoromethylphenyl)-thiazol-5-ylmethoxy]-phenyl}-2-phenyl-pronionic acid (Reference Compound 23)

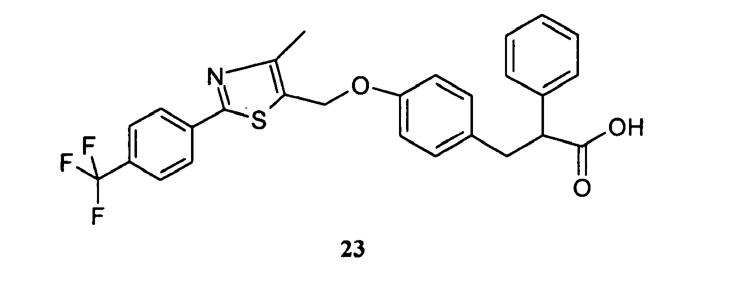

### Preparation of 3-{4-[4-Methtyl-2-(4-trifluoromethyl-phenyl)-thiazol-5-ylmethoxy]-phenyl}-2-phenyl-propionic acid methyl ester (Compound 23A)

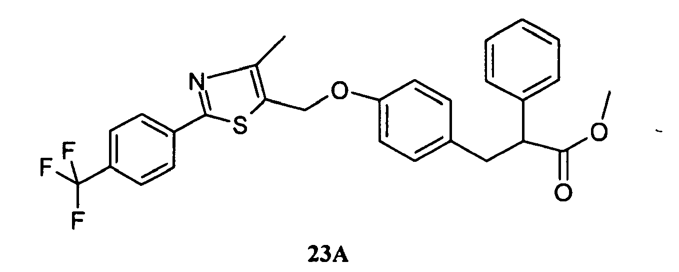

Compound **23A** was prepared according to the method of example **1F** utilizing 3-(4-hydroxy-phenyl)-2-phenylpropionic acid methyl ester. MS: 512 (M+1)⁺.

### Preparation of 3-{4-[4-Methtyl-2-(4-trifluoromethyl-phenyl)-thiazol-5-ylmethoxy]-phenyl}-2-phenyl-propionic acid (Reference Compound 23)

Compound **23** was prepared according to the method of example 1 utilizing compound **23A.** MS: 498 (M+1)⁺.

### Reference Example 24

### Synthesis of 3-{4-[4-Methtyl-2-(4-trifluoromethyl-phenyl)-thiazol-5-ylmethylsulfanyl]-phenyl}-2-pyrrol-1-yl-propionic acid (Reference Compound 24)

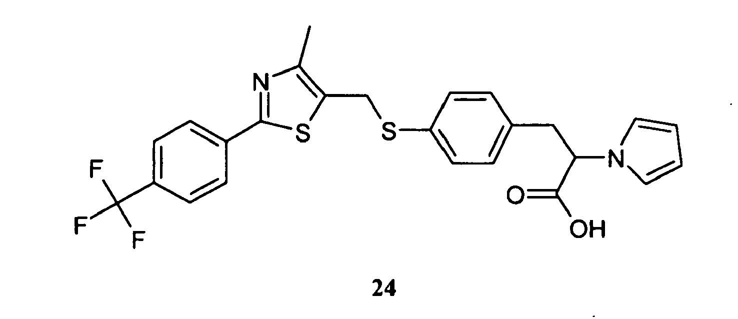

### Preparation of 3-(4-Dimethylcarbamoylsulfanyl-phenyl)-2-pyrrol-1-yl-propionic acid methyl ester. (Compound 24A)

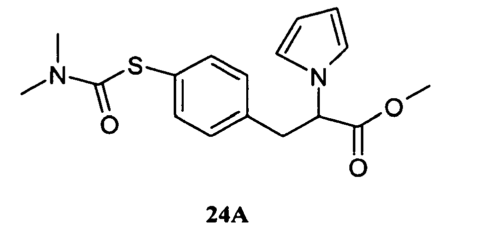

3-(4-Hydroxy-phenyl)-2-phenylpropionic acid methyl ester, Et₃N, 4-dimethylaminopyridine and dimethyl thiocarbamoyl chloride in dioxane was refluxed for 16h. After removing most of solvent, the mixture was partition between ethyl acetate and water. The organic layer was separated, washed with water, dried, filtered and evaporated to afford 4-dimethylthiocarbamoyloxy-phenyl-propionic acid methyl ester, was used in the next step without further purification.
4-Dimethylthiocarbamoyloxy-phenyl-propionic acid methyl ester in THF was added drop wise into a refluxed solution of tetradecane, refluxed for another 3 hours. The solvent was decanted after cooling to room temperature, remaining oil washed several times with hexane. It was purified by flash column chromatography to afford compound **24A.** MS: 333(M+1)⁺.

### Preparation of 3-{4-[4-Methtyl-2-(4-trifluoromethyl-phenyl)-thiazol-5-ylmethylsulfanyl]-phenyl}-2-pyrrol-1-yl-propionic acid (Reference Compound 24)

A solution of compound **24A** in MeOH and NaOH was refluxed for 5 h. Then the 5-chloromethyl-4-methyl-2-(4-trifluoromethyl-phenyl)-thiazol was added and the mixture was refluxed for another hour. The solvent was removed and the crude product was purified by flash column chromatography to afford the compound **24.**
MS: 503 (M+1)⁺.

### Reference Example 25

### Synthesis of 2-[5-(acetylamino)-2-methyl-4-({4-methyl-2-[4-(trifluoromethyl)phenyl](1,3-thiazol-5-yl)}methylthio)phenoxy]acetic acid (Reference Compound 25)

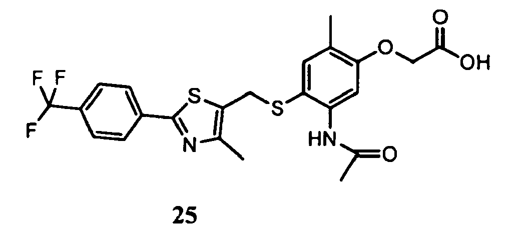

### Preparation of (2-amino-4-hydroxy-5-methylphenyl)thiocarbonitrile (Compound 25 A)

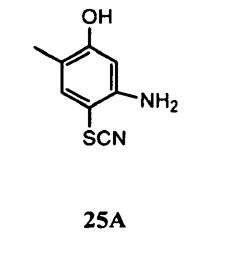

To a mixture of 5-amino-2-methylphenol, sodium thiocyanate and sodium bromide in anhydrous methanol, and bromine in methanol was added dropwise over 30 minutes and allowed to stir at 0 °C for 1 h. Saturated sodium bicarbonate was added to bring pH 7, and the crude product was extracted with ethyl acetate. The combined organics were washed with brine, dried over anhydrous sodium sulfate, concentrated, and purified using normal phase chromatography to afford the title product. 400 MHz ¹H NMR (DMSO-d₆) δ 9.72 (s, 1H), 7.10 (s, 1H), 6.30 (s, 1H), 5.59 (s, 2H), 1.98 (s, 3H).

### Preparation of methyl 2-(3-amino-4-cyanothio-6-methylphenoxy)acetate (Compound 25B)

The title compound was prepared in the manner analogous to Example **3B** with the product from example **25A** methyl bromoacetate and cesium carbonate in acetonitrile. 400 MHz ¹H NMR (DMSO-d₆) δ 7.20 (s, 1H), 6.28 (s, 1H), 5.71 (s, 2H), 4.77 (s, 2H), 3.71 (s, 3H), 2.04 (s, 3H).

### Preparation of methyl 2-[5-(acetylamino)-4-cyanothio-2-methylphenoxy]acetate (Compound 25C)

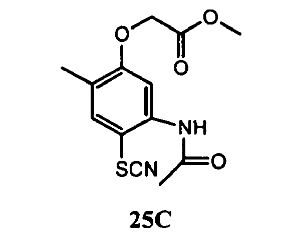

A mixture of the product from example **25B** and acetic anhydride in pyridine was stirred at ambient temperature overnight, then ethyl acetate was added, washed with water, brine, dried over anhydrous sodium sulfate, concentrated to give **25C.** 400 MHz ¹H NMR (DMSO-d₆) δ 7.55 (s, 1H), 6.81 (s, 1H), 4.90 (s, 2H), 3.70 (s, 3H), 2.23 (s, 3H), 2.07 (s, 3H).

### Preparation of methyl 2-[5-(acetylamino)-2-methyl-4-sulfanylphenoxy]acetate (Compound 25D)

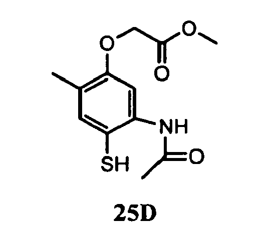

A mixture of the product from example **25C,** dithiothreitol and 0.2M potassium in methanol was refluxed for 1 h. After cooling, ethyl acetate was added, washed with water, brine, dried over anhydrous sodium sulfate, concentrated, and purified using normal phase chromatography to afford the title product. 400 MHz ¹H NMR (DMSO-d₆) δ 9.26 (s, 1H), 7.25 (s, 1H), 7.20 (s, 1H), 4.81 (s, 2H), 3.71 (s, 3H), 2.12 (s, 3H), 1.95 (s, 3H).

### Preparation of methyl 2-[5-(acetylamino)-2-methyl-4-({4-methyl-2-[4-(trifluoromethyl)phenyl](1,3-thiazol-5-yl)}methylthio)phenoxy]acetate (Compound 25E)

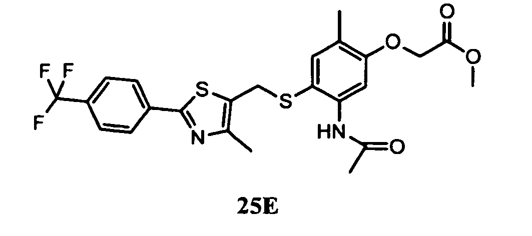

The title compound was prepared in the manner analogous to Example **1F** with the product from example **25D,** 5-(chloromethyl)-4-methyl-2-[4-(trifluoromethyl)phenyl]-1,3-thiazole and cesium carbonate in anhydrous acetonitrile. 400 MHz ¹H NMR (CDCl₃) δ 8.25 (s, 1H), 7.92 (m, 3H), 7.68 (d, 2H), 4.70 (s, 2H), 4.00 (s, 2H), 3.81 (s, 3H), 2.20 (s, 3H), 2.12 (s, 3H), 2.02 (s,3H). MS *m*/*z* 524.83 (M+1).

### Preparation of 2-[5-(acetylamino)-2-methyl-4-({4-methyl-2-[4-(trifluoromethyl)phenyl](1,3-thiazol-5-yl)}methylthio)phenoxy]acetic acid (Reference Compound 25)

The title compound was prepared in the manner analogous to Example **1** with the product from example **25E** and lithium hydroxide monohydrate in tetrahydrofuran/water mixture (10:1). MS *mlz* 510.75 (M+1).

### Reference Example 26

### Synthesis of 2-[5-fluoro-2-methyl-4-({4-methyl-2-[4-(trifluoromethyl)phenyl](1,3-thiazol-5-yl)}methylthio)phenoxy]acetic acid (Reference Compound 26)

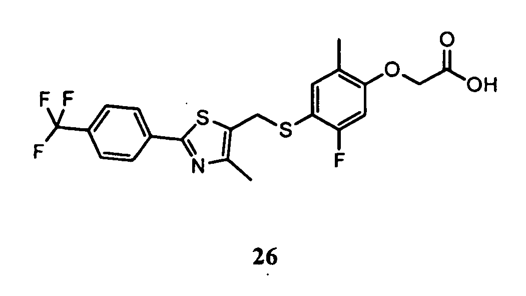

### Preparation of 5-fluoro-2-methylphenol (compound 26A)

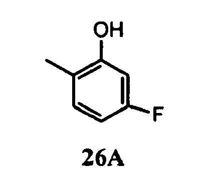

A mixture of 5-fluoro-2-methylphenylamine in concentrated sulfuric acid and water was heated until a clear solution was obtained, then cooled to 0 °C, a solution of sodium nitrite in water was added dropwise over 15 minutes, then poured into boiled saturated copper sulfate solution and heated for 15 minutes, cooled and extracted with ethyl acetate washed with water, brine, dried over anhydrous sodium sulfate, and concentrated to give **26A** in good purity. 400 MHz ¹H NMR (CDCl₃) δ 7.05 (t, 1H), 6.56 (m, 2H), 4.92 (s, 1H), 2.19 (s, 3H).

### Preparation of (2-fluoro-4-hydroxy-5-methylphenyl)thiocarbonitrile (Compound 26B)

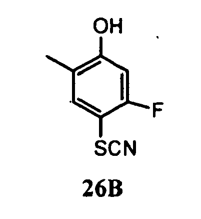

The title compound was prepared in the manner analogous to Example **3A** with the product from example **26A** sodium thiocyanate, sodium bromide and bromine in anhydrous methanol. 400 MHz ¹H NMR (CDCl₃) δ 7.35 (d, 1H), 6.67 (d, 1H), 5.41 (s, 1H), 2.22 (s, 3H).

### Preparation of methyl 2-(4-cyanothio-5-fluoro-2-methylphenoxy)acetate (Compound 26C)

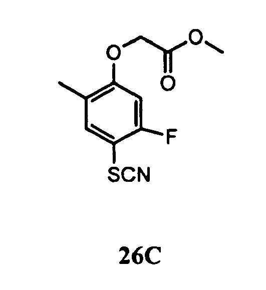

The title compound was prepared in the manner analogous to Example **3B** with the product from example **26B,** methyl bromoacetate, and cesium carbonate in anhydrous acetonitrile. 400 MHz ¹H NMR (CDCl₃) δ 7.40 (d, 1H), 6.55 (d, 1H), 4.68 (s, 2H), 3.85 (s, 3H), 2.26 (s, 3H).

### Preparation of methyl 2-(5-fluoro-2-methyl-4-sulfanylphenoxy)acetate (Compound 26D)

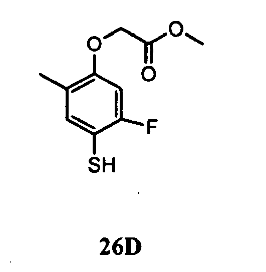

The title compound was prepared in the manner analogous to Example **3C** with the product from example **26C,** dithiothreitol and 0.2M potassium dihydrogenphosphate in anhydrous methanol. 400 MHz ¹H NMR (DMSO-d₆) δ 7.21 (d, 1H), 6.88 (d, 1H) 5.10 (s, 1H), 4.84 (s, 2H), 3.69 (s, 3H), 2.10 (s, 3H).

### Preparation of methyl 2-[5-fluoro-2-methyl-4-({4-methyl-2-[4-(trifluoromethyl)phenyl](1,3-thiazol-5-yl)}methylthio)phenoxy]acetate (Compound 26E)

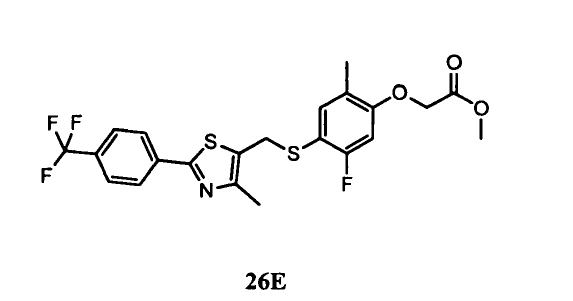

The title compound was prepared in the manner analogous to Example **1F** with the product from example **26D,** 5-(chloromethyl)-4-methyl-2-[4-(trifluoromethyl)phenyl]-1,3-thiazole, and cesium carbonate in 15 ml of anhydrous acetonitrile. 400 MHz ¹H NMR (CDCl₃) δ 7.98 (d, 2H), 7.65 (d, 2H), 7.12 (d, 1H), 6.47 (d, 1H), 4.63 (s, 2H), 4.12 (s, 2H), 3.81 (s, 3H), 2.25 (s, 3H), 2.13 (s, 3H).

### Preparation of 2-[5-fluoro-2-methyl-4-({4-methyl-2-[4-(trifluoromethyl)phenyl](1,3-thiazol-5-yl)}methylthio)phenoxy]acetic acid (Reference Compound 26)

The title compound was prepared in the manner analogous to Example **1** with the product from example **26E**, and lithium hydroxide monohydrate in tetrahydrofuran/water mixture (10:1). MS *m*/*z* 471.72(M+1).

### Reference Example 27

### Synthesis of 2-[5-methoxy-2-methyl-4-({4-methyl-2-[4-(trifluoromethyl)phenyl](1,3-thiazol-5-yl)}methoxy)phenylthio]acetic acid (Reference Compound 27)

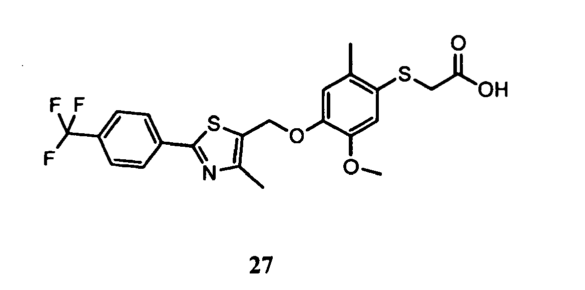

### Preparation of (4-hydroxy-5-methoxy-2-methylphenyl)thiocarbonitrile (Compound 27A)

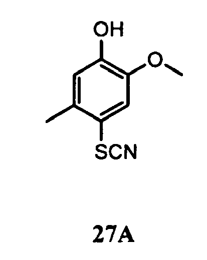

The title compound was prepared in the manner analogous to Example **6B** with 2-methoxy-5-methylphenol, sodium thiocyanate, sodium bromide, and bromine in, methanol. 400 MHz ¹H NMR (DMSO-d₆) δ 9.78 (s, 1H), 7.20 (s, 1H), 6.82 (s, 1H), 3.79 (s, 3H), 2.32 (s, 3H).

### Preparation of [5-methoxy-2-methyl-4-({4-methyl-2-[4-(trifluoromethyl)phenyl](1,3-thiazol-5-yl)}methoxy)phenyl]thiocarbonitrile (Compound 27B)

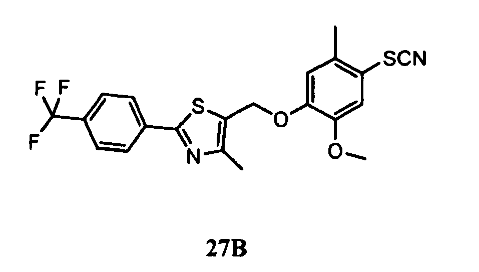

The title compound was prepared in the manner analogous to Example **1F** with the product from example **27A,** 5-(chloromethyl)-4-methyl-2-[4-(trifluoromethyl)phenyl]-1,3-thiazole, and cesium carbonate in anhydrous acetonitri le. 400 MHz ¹H NMR (DMSO-d₆) δ 8.14 (d, 2H), 7.85 (d, 2H), 7.29 (s, 1H), 7.25 (s, 1H), 5.40 (s, 2H), 3.80 (s, 3H), 2.50 (s, 3H), 2.47 (s, 3H).

### Preparation of methyl 2-[5-methoxy-2-methyl-4-({4-methyl-2-[4-(trifluoromethyl)phenyl](1,3-thiazol-5-yl)}methoxy)phenylthio]acetate (Compound 27C)

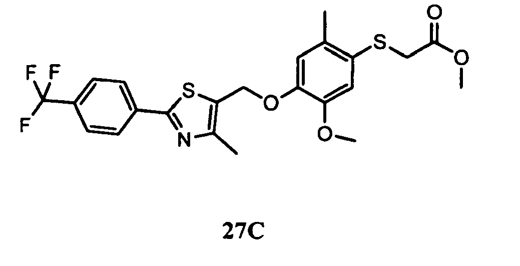

The product from example **27B,** and sodium sulfide in anhydrous methanol was refluxed for 2 h, then cooled and concentrated under reduced pressure. The residue was dissolved in anhydrous acetonitrile, methyl bromoacetate and cesium carbonate were added. The reaction mixture was heated at 60 °C for 2 h, then cooled and filtered through Celite®, concentrated, purified using normal phase chromatography. 400 MHz ¹H NMR (CDCl₃) δ 8.04 (d, 2H), 7.70 (d, 2H), 7.08 (s, 1H), 6.82 (s, 1H), 5.23 (s, 2H), 3.88 (s, 3H), 3.70 (s, 3H), 3.52 (s, 2H), 2.50 (s, 3H), 2.39 (s, 3H). MS *m*/*z* 498 (M+1).

### Preparation of 2-[5-methoxy-2-methyl-4-({4-methyl-2-[4-(trifluoromethyl)phenyl](1,3-thiazol-5-yl)}methoxy)phenylthio]acetic acid (Reference Compound 27)

The title compound was prepared in the manner analogous to Example **1** with the product from example **27C,** and lithium hydroxide monohydrate in tetrahydrofuran/water mixture (10:1). MS *m*/*z* 483.87 (M+1).

### Reference Example 28

### Synthesis of 2,2-difluoro-2-[5-methoxy-2-methyl-4-({4-methyl-2-[4-(trifluoromethyl)phenyl](1,3-thiazol-5-yl)}methoxy)phenylthio]acetic acid (Reference Compound 28)

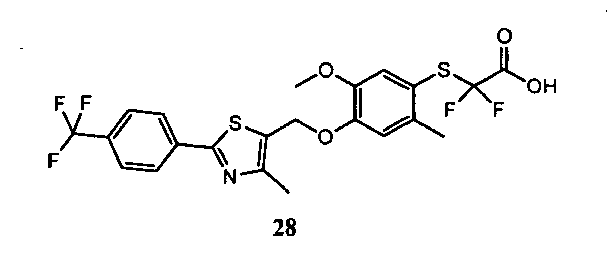

### Preparation of 5-Methoxy-2-methyl-4-[4-methyl-2-(4-trifluoromethyl-phenyl)-thiazol-5-ylmethoxy]-benzene disulfide (compound 28A)

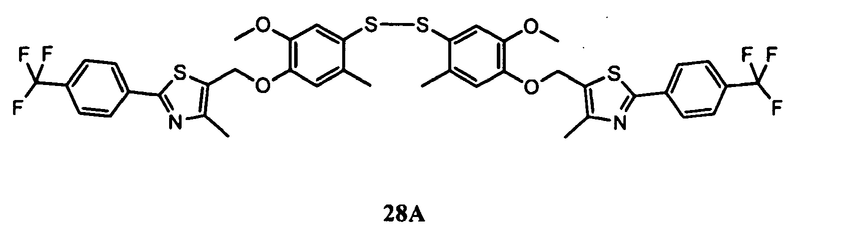

The title compound was prepared from compound **27B** and 0.2 M potassium dihydrogenphosphate in methanol in the manner analogous to Example 3C. 400 MHz ¹H NMR (DMSO-*d*₆) δ 8.13 (d, 4H), 7.88 (d, 4H), 7.10 (s, 2H), 6.98 (s, 2H), 5.38 (s, 4H), 3.70 (s, 6H), 2.50 (s, 6H), 2.25 (s, 6H).

### Preparation of ethyl 2,2-difluoro-2-[5-methoxy-2-methyl-4-({4-methyl-2-[4-(trifluoromethyl)phenyl](1,3-thiazol-5-yl)}methoxy)phenylthio]acetate (Compound 28B)

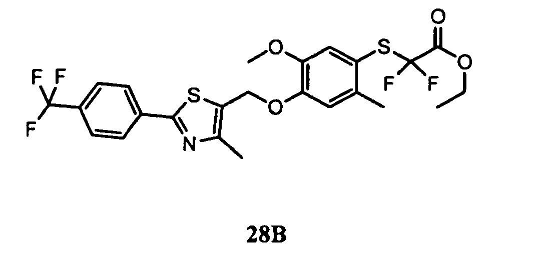

The mixture of the product from example **28A,** triphenylphospine, HCI in water and dioxane was heated at 60 °C for 12 h, then cooled to room temperature and concentrated in vacuo. The residue was dissolved in anhydrous acetonitrile, ethyl 2-bromo-2,2-difluoroacetate and cesium carbonate were added. The reaction mixture was stirred at room temperature for 2 h, then filtered through Celite^{®}. The filtrate was concentrated, and purified using normal phase chromatography to afford the title product. 400 MHz ¹H NMR (CDCl₃) δ 8.03 (d, 2H), 7.68 (d, 2H), 7.15 (s, 1H), 6.90 (s, 1H), 5.29 (s, 2H), 4.25 (q, 2H), 3.88 (s, 3H), 2.55 (s, 3H), 2.44 (s, 3H), 1.29 (t, 3H).

### Preparation of 2,2-difluoro-2-[5-methoxy-2-methyl-4-({4-methyl-2-[4-(trifluoromethyl)phenyl](1,3-thiazol-5-yl)}methoxy)phenylthio]acetic acid (Reference Compound 28)

The product from example **28B** was dissolved in a mixture of methanol and water, then treated with sodium hydroxide. After stirring at 70 °C for 1 h, the reaction mixture was cooled to room temperature, and acidified to pH 3 with 1 N HCl. The white precipitate was collected by filtration, washed subsequently with water and hexanes, and dried in vacuo to afford the title product. MS *m*/*z* 520 (M+1).

### Example 29

### Synthesis of 1-[{3-Methoxy-4-[4-methyl-2(4-trifluoromethylphenyl)thiazol-5-ylmethylsulfanyl]benzyl}pyrrolidine-2-carboxylic acid (Compound 29)

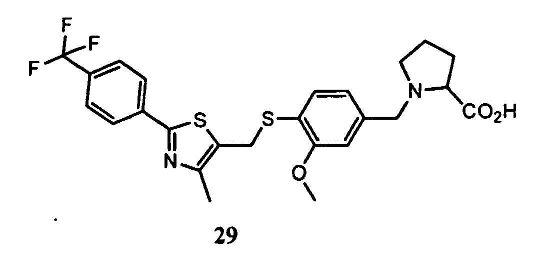

### Preparation of Dimethylthiocarbamic acid O-(4-formyl-2methoxyphenyl) ester (compound 29A)

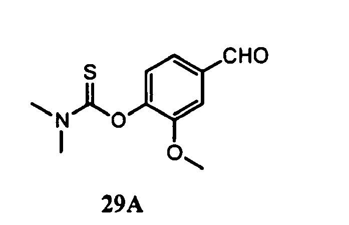

To a solution of vanillin in Dioxane, under nitrogen atmosphere, was added dimethylthiocarbonyl chloride, triethylamine, and DMAP. The resulting mixture was warmed to reflux and refluxed 16h, then diluted with water and extracted with EtOAc. The combined extracts were washed with water and brine, and the organic phase dried over sodium sulfate then concentrated. The residue was purified by recrystallization in MeOH and water to give **29A** as a white solid. (mp 126-127°C) Analyzed for C₁₃H₁₅NO₂: Calc: C, 55.21%; H, 5.48%; N, 5.85%; Found: C, 55.17%; H, 5.42%; N, 5.92%.

### Preparation of 4-Mecapto-3-methoxybenzaldehyde (compound 29B)

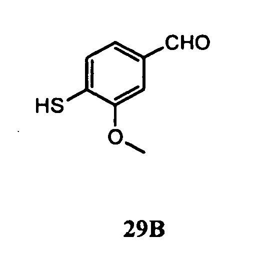

Compound **29A** was heated to 230-240°C under nitrogen atmosphere for I h, then cooled to room temperature. The residue was dissolved in methano, placed under nitrogen atmosphere, and solution of 50% aqueous sodium hydroxide in water was added. The resulting mixture was refluxed for 16h, cooled to room temperature, acidified with 2N HCL and extracted with EtOAc. The combined extracts were washed with saturated aqueous sodium bicarbonate and brine. The organic phase was dried over sodium sulfate and concentrated under vacuum. The residue was purified by flash chromatography (silica gel, 10% EtOAc/Hexane) to give **29B** as a yellow oil. NMR (400Mhz, CDCl₃) δ 3.96 (s, 3H), 4.16 (s, 1H), 7.32-7.40 (m, 3H), 9.87 (s, 1H).
MS: (m+1) 169.

### Preparation of 3-Methoxy-4-[4-methyl-2-(4-trifluoromethylphenyl)thiazol-5-ylmethylsufanyl]benzaldehyde (compound 29C)

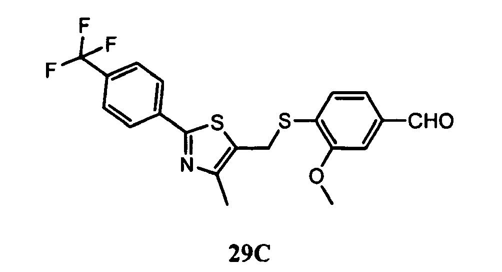

To a solution of **29B** in acetonitrile, under nitrogen atmosphere, was added 5-chloromethyl-4-methyl-2-(4-trifluoromethylphenyl)thiazole followed by cesium carbonate. The resulting mixture was stirred 16h, diluted with water. The precipitate was collected by filtration, dried, dissolved in ethyl ether, and filtered. The filtrate was concentrated under vacuum and the residue purified by flash chromatography (silica gel, 20% EtOAc/Hexane to give **29C** as a yellow solid. NMR (400Mhz, CDCl₃) δ 2.55 (s, 3H), 3.92 (s, 3H), 4.74 (s, 2H), 7.46-7.50 (m, 1H), 7.68-7.72 (m, 3H), 8.08-8.11 (m, 2H), 8.20-8.23 (m, 1H), 9.89 (s, 1H). MS: (m+1)424.

### Preparation of 1-{3-Methoxy-4-[4-methyl-2-(4-trifluoromethylphenyl)thiazol-5-ylmethylsulfanyl]benzyl}pyrrolidine-2-carboxylic acid methyl ester (compound 29 D)

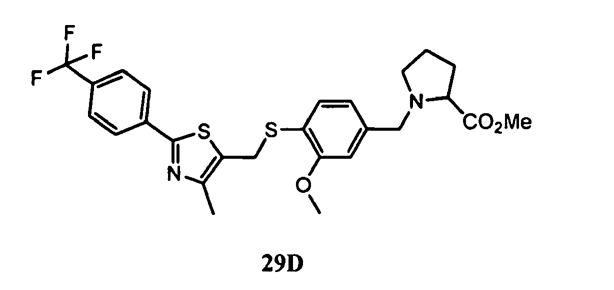

To a solution of **29C** in THF and N,N'-dimethylethyleneurea under nitrogen atmosphere was added DL-methylproline hydrochloride. The resulting mixture was stirred until clear, then glacial acetic acid was added dropwise followed by sodium triacetoxyborohydride in small portions over 30 min. The reaction mixture was stirred 16h, quenched with water, and extracted with EtOAc. The combined extracts were washed with brine. The organic phase was dried over magnesium sulfate and concentrated under vacuum. The residue was purified by flash chromatography (silica gel, 20% EtOAc/Hexane) to give **29D** as a yellow solid. NMR (400Mhz, CDCl₃) δ 2.03-2.20 (m, 2H), 2.22- 2.40 (m, 2H) 2.51 (s, 3H), 2.62-2.80 (m, 1H) 3.38-3.45 (m, 1H) 3.79 (s, 3H), 4.02-4.18 (m, 2H) 4.80 (s, 2H), 6.68-6.71 (m, 1/2H), 6.88-6.91 (m, 1/2H), 7.18 (d, *J*= 7Hz, 1/2H), 7.43 (d, *J*= 7Hz, 1/2H) 7.63-7.69 (m, 3H), 8.00 (d, *J* = 4,2H). MS: (m+1) 537.

### Preparation of 1-[{3-Methoxy-4-[4-methyl-2(4-trifluoromethylphenyl)thiazol-5-ylmethylsulfanyl]benzyl}pyrrolidine-2-carboxylic acid (Compound 29)

To a solution of **29D** in methanol was added lithium hydroxide monohydrate. The resulting mixture was warmed to reflux and refluxed for 16h, then neutralized with 1N aqueous HCl and diluted with water. The precipitate was collected, air dried, then triturated in EtOAc to give **29** as a white solid. MS: (m+1) 523.

### Reference Example 30

### Synthesis of ({2-Methyl-4-(4-methyl-2-(4-trifluoromethyl-phenyl)-thiazol-5-ylmethylsulfanyl]-phenoxy}-phenyl-acetic acid (Reference Compound 30)

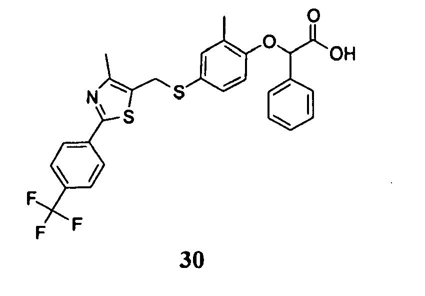

### Preparation of 4-thiocyanato-2-methyl-phenol (compound 30A)

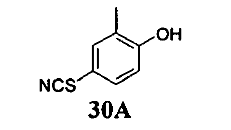

The title compound was prepared in a manner analogous to compound **3A.** 400 MHz ¹H NMR (DMSO-*d*₆) δ 10.1 (s, 1H), 7.36 (s, 1H), 7.30 (d, 1H, *J* = 8.1 Hz), 6.77 (d, 1H, *J =* 8.1 Hz), 2.08 (s, 3H).

### Preparation of (4-thiocyanato-2-methyl-phenoxy)-phenyl-acetic acid ethyl ester (Compound 30B)

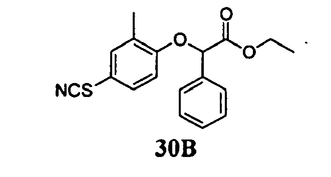

The title compound was prepared in a manner analogous to compound **3B** by reacting copound 30A with bromo-phenyl-acetic acid ethyl ester. MS: (m+1) 328.

### Preparation of (4-Mercapto-2-methyl-phenoxy)-phenyl-acetic acid ethyl ester (Compound 30C)

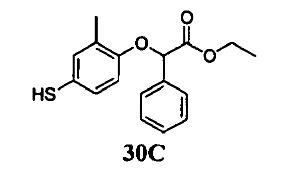

The title compound was prepared in a manner analogous to compound 3C. 400 MHz ¹H NMR (DMSO-*d*₆) δ 7.51 (d, 2H, *J*= 8.5 Hz), 7.35 (m, 3H), 7.07 (s, 1H), 6.99 (d, 1H, *J*= 10.5 Hz), 6.76 (d, 1H, *J*= 10.5 Hz), 5.90 (s, 1H), 5.03 (s, 1H), 4.04 (m, 2H), 2.15 (s, 3H), 1.04 (t, 3H, *J*= 7.3 Hz).

### Preparation of {2-Methyl-4-[4-methyl-2-(4-trifluoromethyl-phenyl)-thiazol-5-ylmethylsulfanyl]-phenoxy}-phenyl-acetic acid ethyl ester (Compound 30D)

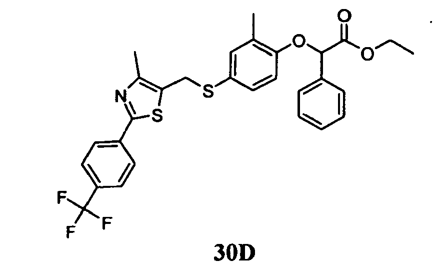

The title compound was prepared in a manner analogous to compound **1F,** using compound **30C** and **1B.** MS: (m+1) 558.

### Preparation of ({2-Methyl-4-[4-methyl-2-(4-trifluoromethyl-phenyl)-thiazol-5-ylmethylsulfanyl]-phenoxy}-phenyl-acetic acid (Reference Compound 30)

**30D** was saponified in the same manner as described for compound **1F** to give, after recrystalisation from CHCl₃/hexanes, the title compound as a solid. *m*/*z* = 530 (M+1).

### Reference Example 31

### Synthesis of {5-Chloro-2-methyl-4-[4-methyl-2-(4-trifluoromethyl-phenyl)-thiazol-5-ylmethylsulfanyl]-phenoxy}-acetic acid (Reference Compound 31)

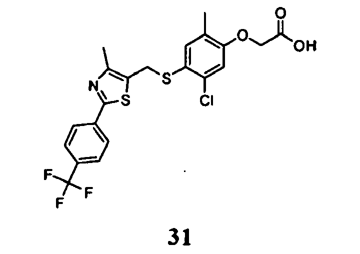

### Preparation of 5-Chloro-2-methyl-4-thiocyanato-phenol (compound 31A)

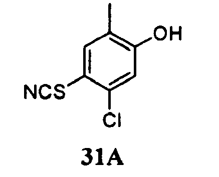

The title compound was prepared from 5-chloro-2-methyl-phenol in a manner analogous to compound **3A.** MS *mlz* 199 (M+).

### Preparation of (5-chloro-2-methyl-4-thiocyanato-phenoxy)-acetic acid methyl ester (Compound 31B)

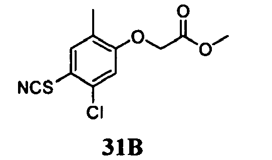

The title compound was prepared from compound **31A** in a manner analogous to compound **3B.** MS *mlz* 245 (M-CN).

### Preparation of (5-chloro-4-mercapto-2-methyl-phenoxy)-acetic acid methyl ester (Compound 31C)

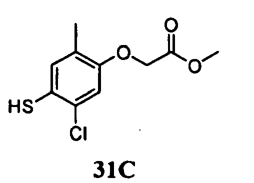

The title compound was prepared from compound **31B** in a manner analogous to compound **3C.** MS *m*/*z* 245 (M-1).

### Preparation of {5-Chloro-2-methyl-4-[4-methyl-2-(4-trifluoromethylphenyl)thiazol-5-ylmethylsulfanyl]-phenoxy}-acetic acid methyl ester (Compound 31D)

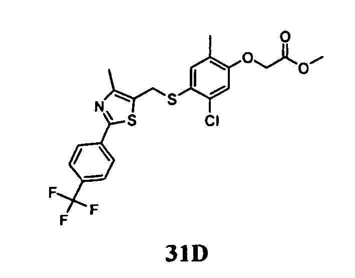

**31C** and 5-chloromethyl-4-methyl-2-(4-trifluoromethyl-phenyl)-thiazole (compound **1B)** were coupled in a similar manner as described for compound **1F** to provide, after purification by flash column chromatography (gradient elution: 5% EtOAc/hexanes to 35% EtOAc/hexanes), the title compound as a yellow solid. MS *m*/*z* 502 (M+1).

### Preparation of {5-Chloro-2-methyl-4-[4-methyl-2-(4-trifluoromethyl-phenyl)-thiazol-5-ylmethylsulfanyl]-phenoxy}-acetic acid (Reference Compound 31)

**31D** was saponified in the same manner as described for compound **1F** to give, after recrystalisation from CHCl₃/hexanes, the title compound as a solid. MS *m*/*z* 488 (M+1).

### Reference Example 32

### Synthesis of 2-[5-(methoxymethyl)-2-methyl-4-({4-methyl-2-[4-(trifluoromethyl)phenyl](1,3-thiazol-5-yl)}methylthio)phenoxy]acetic acid (Reference Compound 32)

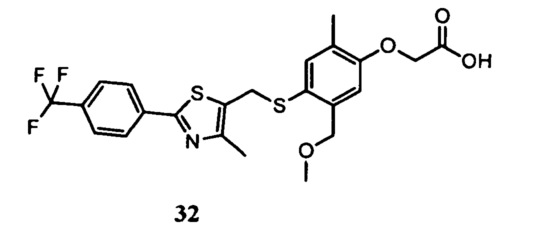

### Preparation of 5-(hydroxymethyl)-2-methylphenol (Compound 32A)

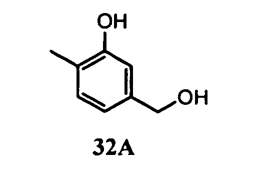

To 3-hydroxy-4-methylbenzoic acid in THF was added borane/THF complex (1.0 M solution) slowly at 0°C under nitrogen. After completion of addition, the mixture was allowed to warm to room temperature, then heated at 70 °C for 2 h, cooled to room temperature again, 1 N HCl (150 ml) was added slowly and stirred at room temperature. The reaction mixture was concentrated in vacuo, then ethyl acetate was added, washed with brine, dried over sodium sulfate, and concentrated to give **32A** in good purity. 400 MHz ¹H NMR (DMSO-*d*₆) δ 9.19 (s, 1H), 6.98 (d, 1H), 6.77 (s, 1H), 6.62 (d, 1H), 5.03 (t, 1H), 4.39 (d, 2H), 2.08 (s, 3H).

### Preparation of 5-(methoxymethyl)-2-methylphenol (Compound 32B)

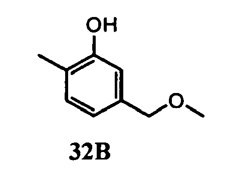

To a stirred solution of **32A** in methanol was added concentrated sulfuric acid slowly at room temperature. The mixture was refluxed overnight. After cooling, pH was adjusted to 3 ∼ 4 with 2 N sodium hydroxide and concentrated, then ethyl acetate was added, washed with brine, dried over sodium sulfate, and concentrated to give **32B** in good purity. 400 MHz ¹H NMR (DMSO-*d*₆) δ 9.23 (brs, 1H), 7.00 (d, 1H), 6.76 (s, 1H), 6.62 (d, 1H), 4.29 (s, 2H), 3.23 (s, 3H), 2.10 (s, 3H).

### Preparation of 5-Methoxymethyl-2-methyl-4-thiocyanato-phenol (Compound 32C)

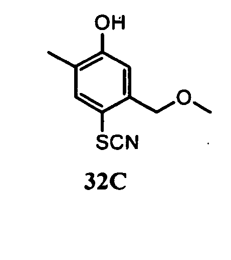

To a stirred solution of the product from example **32B,** sodium thiocyanate, and sodium bromide in methanol at 0 °C was added a solution of bromine in methanol dropwise. After the completion of the bromine addition, the reaction mixture was stirred at room temperature for 1 h, then concentrated in vacuo. The resulting residue was taken up in ethyl acetate, washed with saturated sodium bicarbonate solution, brine, dried over sodium sulfate, concentrated, and purified using normal phase chromatography to afford the title product. 400 MHz ¹H NMR (DMSO-*d*₆) δ 10.18 (brs, 1H), 7.46 (s, 1H), 6.99 (s, 1H), 4.48 (s, 2H), 3.32 (s, 3H), 2.17 (s, 3H).

### Preparation of (5-Methoxymethyl-2-methyl-4-thiocyanato-phenoxy)-acetic acid methyl ester (Compound 32D)

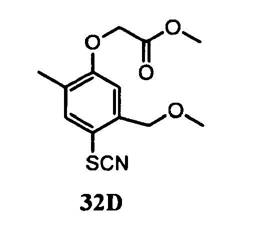

The mixture of the product from example **32C,** methyl bromoacetate, and cesium carbonate in anhydrous acetonitrile was heated at 60 °C for 2 h. After cooling, the reaction mixture was filtered through Celite^{®}. The filtrate was diluted with diethyl ether, washed with brine, dried over sodium sulfate, and concentrated to give **32D.** 400 MHz ¹H NMR (DMSO-*d*₆) δ 7.58 (s, 1H), 7.06 (s, 1H), 4.96 (s, 2H), 4.50 (s, 2H), 3.74 (s, 3H), 3.32 (s, 3H), 2.22 (s, 3H).

### Preparation of methyl 2-[5-(methoxymethyl)-2-methyl-4-sulfanylphenoxy]acetate (Compound 32E)

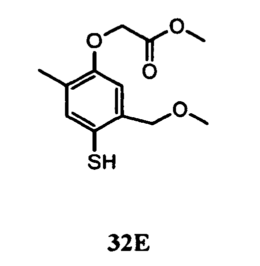

A solution of the product from example **32D,** dithiothreitol, and 0.2 M potassium dihydrogenphosphate in methanol was refluxed for 1 h under nitrogen, then cooled and concentrated in vacuo. The resulting residue was taken up in diethyl ether, and washed with brine, dried over sodium sulfate, concentrated, and purified using normal phase chromatography to afford the title product. 400 MHz ¹H NMR (CDCl₃) δ 7.17 (s, 1H), 6.75 (s, 1H), 4.63 (s, 2H), 4.44 (s, 2H), 3.80 (s, 3H), 3.45 (s, 1H), 3.40 (s, 3H), 2.22 (s, 3H).

### Preparation of methyl 2-[5-(methoxymethyl)-2-methyl-4-({4-methyl-2-[4-(trifluoromethyl)phenyl](1,3-thiazol-5-yl)}methylthio)phenoxy]acetate (Compound 32F)

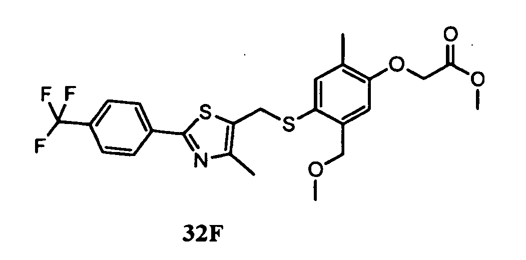

Compound 32E was dissolved in anhydrous acetonitrile, then 5-(chloromethyl)-4-methyl-2-[4-(trifluoromethyl)phenyl]-1,3-thiazole was added followed by cesium carbonate. The mixture was stirred at room temperature for 2 h, then filtered through Celite^{®}, concentrated, and purified using normal phase chromatography to afford the title product. 400 MHz ¹H NMR (CDCl₃) δ 7.98 (d, 2H), 7.65 (d, 2H), 7.21 (s, 1H), 6.80 (s, 1H), 4.69 (s, 2H), 4.48 (s, 2H), 4.11 (s, 2H), 3.80 (s, 3H), 3.37 (s, 3H), 2.22 (s, 3H), 2.19 (s, 3H).

### Preparation of 2-[5-(methoxymethyl)-2-methyl-4-({4-methyl-2-[4-(trifluoromethyl)phenyl](1,3-thiazol-5-yl)}methylthio)phenoxy]acetic acid (Reference Compound 32)

The title compound was prepared in the manner analogous to Example **1** with the product from example **32F** and lithium hydroxide monohydrate) in THF and water. MS *m*/*z* 498 (M+1).

### Example 33

### Synthesis of (4-{4-[4-Methyl-2-(4-trifluoromethyl-phenyl)-thiazol-5-ylmethylsulfanyl]-phenyl}-cyclohexyl)-acetic acid (Compound 33):

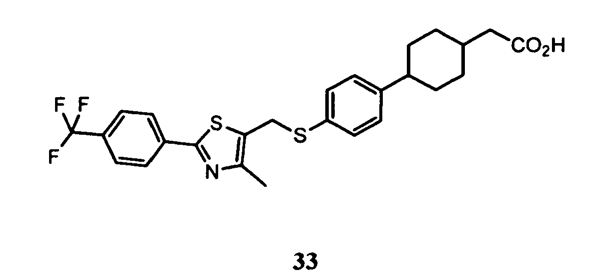

### Preparation of [4-(4-Chlorosulfonyl-phenyl)-cyclohexyl]-acetic acid ethyl ester (compound 33A):

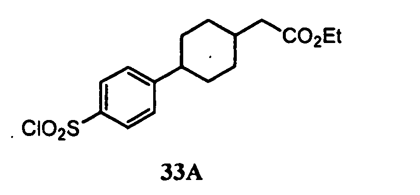

Compound **33A** was prepared as follows. Chlorosulfonic acid was cooled to 0 °C. Then (4-Phenyl-cyclohexyl)-acetic acid ethyl ester was added over 30 min. The mixture was stirred at RT for 3h and was poured into ice (100g). The cloudy solution was extracted with ether (2x50mL). The extracts were dried with magnesium sulfate and concentrated to give a brown oil which was passed through a short pad of silica gel to afford the desired product. MS 345 (M+1)⁺

### Preparation of [4-(4-Mercapto-phenyl)-cyclohexyl]-acetic acid methyl ester (Compound 33B):

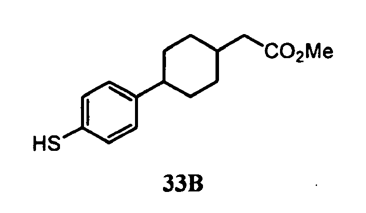

Compound **33A** was refluxed with tin powder in MeOH and 4M HCl/dioxane. After 3h, the reaction mixture was poured into ice with CH₂Cl₂. The phases were separated and the aqueous layer was extracted with CH₂Cl₂. The combined organic layers were dried with magnesium sulfate, filtered and evaporated to give the crude thiol. The ethyl ester was completely transesterified to the methyl ester. MS 263 (M-1)⁺

### Preparation of (4-{4-[4-Methyl-2-(4-trifluoromethyl-phenyl)-thiazol-5-ylmethylsulfanyl]-phenyl}-cyclohexyl)-acetic acid methyl ester (Compound 33C):

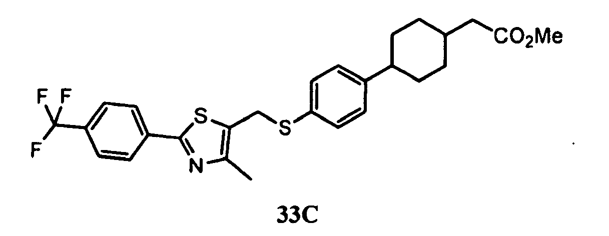

Compound **33B** (crude mixture from above) was dissolved in acetonitrile with chloride 5-chloromethyl-4-methyl-2-(4-trifluoromethyl-phenyl)-thiazole and Cs₂CO₃. The reaction mixture was stirred at RT overnight. Ether and water were added and stirring was continued for another 5 min. The layers were separated and the aqueous layer was extracted with ether. The combined organics were dried over MgSO₄ and concentrated to an oil. The crude product was purified by column chromatography eluted with EtOAc and hexanes to give the desired product. MS 520 (M+1)⁺

### Preparation of (4-{4-[4-Methyl-2-(4-trifluoromethyl-phenyl)-thiazol-5-ylmethylsulfanyl]-phenyl}-cyclohexyl)-acetic acid (Compound 33):

To a solution of **33C** in MeOH and THF was added LiOH.H₂O. After refluxing overnight, the solution was cooled to RT and solvents were removed. The residue was dissolved in water and neutralized with 1N HCl. The cloudy solution was extracted with EtOAc and the extracts were dried with MgSO₄and concentrated. The crude product was purified by chromatography to afford the desired product. MS 506 (M+1)⁺

### BIOLOGICAL ASSAYS

The compounds of the present invention have demonstrated PPAR modulating activity in the standard assays commonly employed by those skilled in the art. Accordingly, such compounds and formulations comprising such compounds are useful for treating, preventing or controlling hypercholesterolemia and hyperlipidemia.

### A. Selectivity Measurements

### 1. Test A. Transient transfections assay using the HepG2 hepatoma cell line.

HepG2 cells were transiently transfected with an expression plasmids encoding hPPARα, hPPARβ or mPPARγ chimeric receptors and a reporter containing the yeast upstream activating sequence (UAS) upstream of the viral E1B promoter controlling a luciferase reporter gene. In addition, the plasmid pRSVβ-gal was used to control for transfection efficiency. HepG2 cells were grown in DMEM supplemented with 10%FBS and 1µM non-essential amino acid. On the first day, cells were split into 100mm dishes at 2.5x10⁶/dish and incubated overnight at 37C°/5% CO₂ On the second day the cells were transiently transfected with plasmid DNA encoding a chimeric receptor, the luciferase reporter gene; and β-gal. For each 100 mm dish, 15µg of lucifease reporter (PG5E1b) DNA, 15µg of Ga14-PPAR chimeric receptor DNA, and 1.5µg of β-gal plasmid DNA were mixed with 1.4ml of opti-MEM in the tube. 28µl of LipoFectamine-2000 reagent was added to 1.4ml of opti-MEM in the tube, and incubate for 5 min at RT. The diluted Lipofectamine-2000 reagent was combined with the DNA mixture, and incubate for 20 min at RT. After fresh medium was added to each 100mm dish of cells, 2.8ml of Lipofectamine2000-DNA mixture was added dropwise to the 100mm dish containing 14ml of medium, and incubate 37°C overnight. On day three cells were trypsinized off the 100 mm dishes and re-plated on 96 well plates. Cells were plated at 2.5x 10⁴ cells per well in 150µl of media and 50µl of compound diluted by media was added. The test compounds added were in the range from 50µM to 50pM. After addition of compounds, the plates were incubated at 37C° for 24 hours. Subsequently cells were washed with once with 100µl of PBS, lysed, and processed for measuring luciferase and β-gal activity using Dual-Light luciferase kit from Tropix ®, according to the manufacturer's recommendations, on an EG&G Bethold MicroLumat LB96P luminometer. EC₅₀ values were obtained using the GraphPad Prism^{™} program. Surprisingly, the compounds of the present invention exhibit activity for both PPARα and PPARβ. Accordingly, the compounds of the present invention should find considerable therapeutic applications for hypercholesterolemia and hyperlipidemia. The Hep G2-hBeta EC₅₀ ("EC₅₀-β") data as well as the Hep G2-hAlpha EC₅₀ ("EC₅₀₋ α") data of the compounds of the invention are presented in Table 2 below.

**Table 2**

| **Example or Reference Example** | **EC50-β nM** | **EC50-α nM** |
|---|---|---|
| 1 | 14.4 | 42 |
| 2 | 361.3 | 1227.5 |
| 3 | 1281.0 | 538 |
| 4 | 23.4 | 161 |
| 5 | 2.3 | 121 |
| 6 | 1235.0 | 1391 |
| 7 | 472.5 | 1338 |
| 8 | 45.3 | 840 |
| 9 | 891.3 | 5751.5 |
| 10 | - | - |
| 11 | 48.8 | 507.3 |
| 12 | 303.5 | 135 |
| 13 | 47.6 | 1098.2 |
| 14 | 6740.0 | 992 |
| 15 | - | - |
| 16 | 1953.2 | 1229 |
| 17 | 19.9 | 5.5 |
| 18 | 803.0 | - |
| 19 | 1124.0 | 1540 |
| 20 | 34.9 | 146 |
| 21 | 1246.5 | 442 |
| 22 | - | - |
| 23 | - | - |
| 24 | 2000000 | 2000000 |
| 25 | 2000000 | - |
| 26 | .07 | 170.1 |
| 27 | - | - |
| 28 | - | - |
| 29 | 2000000 | - |
| 30 | - | - |
| 31 | 105.2 | 2047.4 |
| 32 | 649.0 | 625.1 |
| 33 | 2000000 | - |

### B. Effect of PPAR-beta compounds on lipid and human apoprotein A1 concentrations in the hApoA1 transgenic mouse

Mice, transgenic for human apoA1, were purchased from Jackson laboratories. All animals were allowed normal chow (Ralston-Purina) and water ad libitum in temperature controlled rooms, under a 12-h light, 12-h dark cycle beginning with lights on at 6 AM. During the treatment phase of the study the mice were dosed daily between 6 and 9 AM by oral gavage using a suspension vehicle of 1.5% carboxymethylcellulose plus 0.2 percent Tween-20 (CMC/Tween) containing the specified compounds. Control animals received vehicle alone. Vehicle volume represented 0.25 percent of body weight. Under anesthesia, tail blood was obtained weekly in the morning at the indicated days of study. At termination, tissue samples (liver, intestine, fat, and muscle) were taken to study effects on genes effecting lipid metabolism. Each of the compounds of the present invention that were tested effected a significant increase in HDL over the values observe for the control animals. Furthermore, these compounds resulted in triglyceride levels which were lower than observed in controls. The compounds of the present invention exhibited a 61 to 123 mg/dL increase in HDL compared to a 44 mg/dL increase for the controls at the end of the study (day 16). Similarly, compounds of the present invention exhibited an increase on average for the HDL to (LDL + VLDL) ratio. This ratio was 14 to 18.3 at the beginning of the study. At the end of the study this ratio was found to be from 11.9 to 83.9. (Controls dropped from 15.5 to 11.9)

### FORMULATIONS

The compounds of the present invention can be administered alone or in combination with one or more therapeutic agents. These include, for example, other agents for treating, preventing or controlling hypercholesteremia, hyperlipidemia, obesity, hyperglycemia, hypercholesteremia, hyperlipidemia, atherosclerosis, hypertriglyceridemia, and hyperinsulinemia.

The compounds are thus well suited to formulation for convenient administration to mammals for the prevention and treatment of such disorders.

The following examples further illustrate typical formulations provided by the invention.

### Formulation 1

| **Ingredient** | **Amount** |
|---|---|
| compound of Formulas I-V | 0.5 to 800 mg |
| sodium benzoate | 5 mg |
| isotonic saline | 1000 mL |

The above ingredients are mixed and dissolved in the saline for IV administration to a patient.

### Formulation 2

| **Ingredient** | **Amount** |
|---|---|
| compound of Formulas I-V | 0.5 to 800 mg |
| cellulose, microcrystalline | 400 mg |
| stearic acid | 5 mg |
| silicon dioxide | 10 mg |
| sugar, confectionery | 50 mg |

The ingredients are blended to uniformity and pressed into a tablet that is well suited for oral administration to a patient.

### Formulation 3

| **Ingredient** | **Amount** |
|---|---|
| compound of Formulas I-V | 0.5 to 800 mg |
| starch, dried | 250 mg |
| magnesium stearate | 10 mg |

The ingredients are combined and milled to afford material suitable for filling hard gelatin capsules administered to a patient.

### Formulation 4

| **Ingredient** | **Amount % wt./(total wt.)** |
|---|---|
| compound of Formulas I-V | 1 to 50 |
| Polyethylene glycol 1000 | 32 to 75 |
| Polyethylene glycol 4000 | 16 to 25 |

The ingredients are combined via melting and then poured into molds containing 2.5 g total weight.

While embodiments of the invention have been illustrated and described, it is not intended that these embodiments illustrate and describe all possible forms of the invention.

## Claims

1. A compound having a formula I: 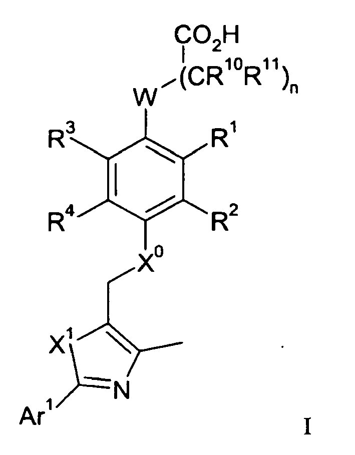 or a pharmaceutically acceptable salt thereof,
wherein:
W is CR⁵R⁶, -(CH₂)ₚ-(C₃-C₈cycloalkylene), or -(CH₂)ₚ-(C₃-C₈ heterocycloalkylene);
X⁰ and X¹ are independently O or S;
Ar¹ is aryl or heteroaryl;
R¹, R², R³, and R⁴ are hydrogen, C₁-C₆alkyl, C₁-C₆alkoxy, halo-C₁-C₁₁-alkyl, -O-(CH₂)pCF₃, halogen, nitro, cyano, -OH, -SH, -CF₃, -S(O)ₚC₁-C₁₁alkyl, S(O)ₚaryl, -(CH₂)ₘOR⁷, -(CH₂)ₘNR⁸R⁹, -COR⁷, -CO₂H, -CO₂R⁷, or -NR⁸R⁹;
R⁵ and R⁶ are joined together to form a 3 to 7 member cycloalkyl or cycloalkenyl;
R⁷ is independently hydrogen, C₁-C₁₁alkyl, C₂-C₁₂alkenyl, C₂-C₁₂alkynyl or aryl;
R⁸ and R⁹ are independently hydrogen, C₁-C₁₁alkyl, C₂-C₁₂alkenyl, C₂-C₁₂ alkynyl, -COC₁-C₁₁alkyl, -COaryl, C₃-C₈cycloalkyl, -CO₂C₁-C₁₁alkyl, -CO₂aryl, -SO₂ C₁-C₁₁alkyl, -SO₂aryl, or joined together to form a 4 to 7 member ring having 1 to 3 heteroatoms;
R¹⁰ and R¹¹ are independently hydrogen, halo, aryl or heteroaryl;
m is 0 to 5;
n is 0 to 5;
p is 0 to 2;
'aryl' means an aromatic ring which is unsubstituted or optionally substituted by 1 to 4 substituents selected from C₁-C₆alkyl, C₁-C₆alkoxy, C₁-C₆ thioalkoxy, halogen, nitro, cyano, -OH, -SH, -CF₃, -CO₂H, -CO₂C₁-C₆ alkyl, -(CH₂)₀₋₂CF₃, -NH₂, -NHC₁-C₆alkyl, -SO₂C₁-C₁₁alkyl, -SO₂NH₂, -CONR'R", or -N(C₁-C₆ alkyl)₂ where R' and R"are independently C₁-C₁₁alkyl, C₂-C₁₂ alkenyl, C₂-C₁₂alkynyl or joined together to form a 4 to 7 member ring; and 'heteroaryl' means an aromatic ring containing one or more heteroatoms which is unsubstituted or optionally substituted by 1 to 4 substituents selected from C₁-C₆alkyl, C₁-C₆alkoxy, C₁-C₆thioalkoxy, halogen, nitro, cyano, -OH, -SH, -CF₃, -CO₂H, -CO₂C₁-C₆alkyl, -(CH₂)₀₋₂CF₃, -NH₂, -NHC₁-C₆alkyl, -SO₂C₁-C₁₁alkyl, -SO₂NH₂, -CONR'R", or -N(C₁-C₆ alkyl)₂ where R' and R"are independently C₁-C₁₁alkyl, C₂-C₁₂alkenyl, C₂-C₁₂alkynyl or joined together to form a 4 to 7 member ring.

2. A compound of claim 1, wherein
W is CR⁵R⁶, C₃-C₈cycloalkylene, or -(CH₂)-(C₃-C₈)heterocycloalkylene;
X⁰ and X¹ are S;
Ar¹ is 4-trifluoromethylphenyl;
R¹, R², R³, and R⁴ are hydrogen, C₁-C₆alkyl, C₁-C₆alkoxy, halogen, -CF₃, -(CH₂)ₘOR⁷, or -(CH₂)ₘNR⁸R⁹;
R⁵ and R⁶ are joined together to form a 3 to 7 member cycloalkyl ring;
R⁷ is hydrogen, or C₁-C₁₁alkyl;
R⁸ and R⁹ are independently hydrogen, C₁-C₁₁alkyl, or -COC₁-C₁₁alkyl;
m is 0 to 5; and
m is 0.

3. A compound of claim 1, wherein
W is 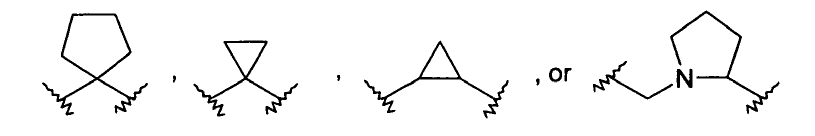 X⁰ and X¹ are S;
Ar¹ is 4-trifluoromethylphenyl;
R¹, R², R³, and R⁴ are hydrogen, C₁-C₆alkyl, C₁-C₆alkoxy, halogen, -CF₃, -(CH₂)ₘOR⁷, or -(CH₂)ₘNR⁸R⁹;
R⁷ is independently hydrogen, or C₁-C₆alkyl;
R⁸ and R⁹ are each independently hydrogen, or C₁-C₆alkyl;
m is 0 to 5; and
n is 0.

4. A compound of claim 1, wherein
R² and R³ are hydrogen; and
R¹ and R⁴ are C₁-C₆alkyl or C₁-C₆alkoxy.

5. A compound of claim 1, wherein
R² and R³ are hydrogen;
R¹ is C₁-C₁₁alkyl; and
R⁴ is C₁-C₁₁alkoxy.

6. A compound of claim 1, wherein
R² and R³ are hydrogen;
R¹ is methyl, ethyl, isopropyl, n-propyl, t-butyl, n-butyl, or isobutyl; and
R⁴ is methoxy, ethoxy, isopropoxy, n-propoxy, t-butoxy, or isobutoxy.

7. A compound according to claim 1 selected from the group:
2-{4-[4-Methyl-2-(4-trifluoromethyl-phenyl)-thiazol-5-ylmethylsulfanyl]-phenyl}-cyclopropanecarboxylic acid;
1- {4-[4-Methyl-2-(4-trifluoromethyl-phenyl)-thiazol-5-ylmethylsulfanyl]-phenyl}-cyclopropanecarboxylic acid;
1- {4-[4-Methyl-2-(4-trifluoromethyl-phenyl)-thiazol-5-ylmethylsulfanyl]-phenyl}-cyclopentanecarboxylic acid;
1-[{3-Methoxy-4-[4-methyl-2(4-trifluoromethylphenyl)thiazol-5-ylmethylsulfanyl]benzyl}pyrrolidine-2-carboxylic acid;
(4- {4-[4-Methyl-2-(4-trifluoromethyl-phenyl)-thiazol-5-ylmethylsulfanyl]-phenyl}-cyclohexyl)-acetic acid; and
pharmaceutically acceptable salts thereof.

8. A pharmaceutical composition comprising a compound of any one of claims 1 to 7 and one or more pharmaceutically acceptable carriers, diluents, or excipients.

9. Use of a compound of any of claims 1 to 7 for the manufacture of a medicament for treating, preventing or controlling hyperlipidemia in a mammal.

10. Use of a compound of any of claims 1 to 7 for the manufacture of a medicament for treating, preventing or controlling hypercholesteremia in a mammal.

11. Use of a compound of any of claims 1 to 7 for the manufacture of a medicament for treating, preventing or controlling atherosclerosis in a mammal.

12. A method of making a compound having formula I: 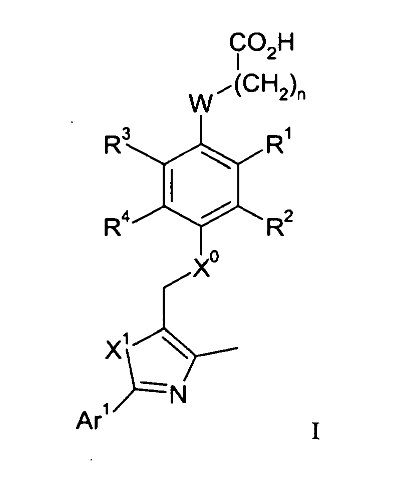 the method comprising, reacting: 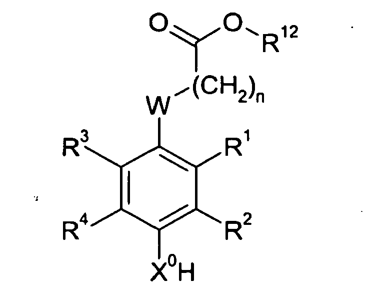 with: 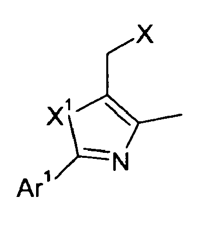 wherein:
W is CR⁵R⁶, -(CH₂)ₚ-(C₃-C₈cycloalkylene), or -(CH₂)ₚ-(C₃-C₈ heterocycloalkylene);
X⁰ and X¹ are independently O or S;
Ar¹ is aryl or heteroaryl;
R¹, R², R³, and R⁴ are hydrogen, C₁-C₆alkyl, C₁-C₆alkoxy, halo-C₁-C₁₁-alkyl, -O-(CH₂)ₚCF₃, halogen, nitro, cyano, -OH, -SH, -CF₃, -S(O)ₚC₁-C₁₁-alkyl, S(O)ₚaryl, -(CH₂)ₘOR⁷, -(CH₂)ₘNR⁸R⁹, -COR⁷, -CO₂H, -CO₂R⁷, or -NR⁸R⁹;
R⁵ and R⁶ are joined together to form a 3 to 7 member cycloalkyl or cycloalkenyl;
R⁷ is independently hydrogen, C₁-C₁₁alkyl, C₂-C₁₂alkenyl, C₂-C₁₂alkynyl or aryl;
R⁸ and R⁹ are independently hydrogen, C₁-C₁₁ alkyl, C₂-C₁₂alkenyl, C₂-C₁₂ alkynyl, -COC₁-C₁₁alkyl, -COaryl, C₃-C₈cycloalkyl, -CO₂C₁-C₁₁alkyl, -CO₂aryl, -SO₂ C₁-C₁₁alkyl, -SO₂aryl, or joined together to form a 4 to 7 member ring having 1 to 3 heteroatoms;
m is 0 to 5;
n is 0 to 5;
p is 0 to 2;
'aryl' means an aromatic ring which is unsubstituted or optionally substituted by 1 to 4 substituents selected from C₁-C₆alkyl, C₁-C₆alkoxy, C₁-C₆ thioalkoxy, halogen, nitro, cyano, -OH, -SH, -CF₃, -CO₂H, -CO₂C₁-C₆ alkyl, -(CH₂)₀₋₂CF₃, -NH₂, -NHC₁-C₆alkyl, -SO₂C₁-C₁₁alkyl, -SO₂NH₂, -CONR'R", or -N(C₁-C₆ alkyl)₂ where R' and R"are independently C₁-C₁₁alkyl, C₂-C₁₂ alkenyl, C₂-C₁₂alkynyl or joined together to form a 4 to 7 member ring; 'heteroaryl' means an aromatic ring containing one or more heteroatoms which is unsubstituted or optionally substituted by 1 to 4 substituents selected from C₁-C₆alkyl, C₁-C₆alkoxy, C₁-C₆thioalkoxy, halogen, nitro, cyano, -OH, -SH, -CF₃, -CO₂H, -CO₂C₁-C₆alkyl, -(CH₂)₀₋₂CF₃, -NH₂, -NHC₁-C₆alkyl, -SO₂C₁-C₁₁alkyl, -SO₂NH₂, -CONR'R", or -N(C₁-C₆ alkyl)₂ where R' and R"are independently C₁-C₁₁alkyl, C₂-C₁₂alkenyl, C₂-C₁₂alkynyl or joined together to form a 4 to 7 member ring;
R¹² is a lower alkyl; and
X is a halogen.

## Patentansprüche

1. Verbindung mit einer Formel I: 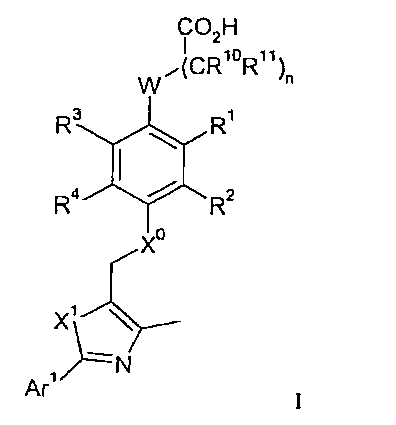 oder ein pharmazeutisch verträgliches Salz davon,
worin:
W für CR⁵R⁶, -(CH₂)ₚ-(C₃-C₈-Cycloalkylen) oder -(CH₂)ₚ-(C₃-C₈-Heterocycloalkylen) steht;
X⁰ und X¹ unabhängig O oder S sind;
Ar¹ Aryl oder Heteroaryl ist;
R¹, R², R³ und R⁴ Wasserstoff, C₁-C₆-Alkyl, C₁-C₆-Alkoxy, Halogen-C₁-C₁₁-alkyl, -O-(CH₂)ₚCF₃, Halogen, Nitro, Cyano, -OH, -SH, -CF₃, -S(O)ₚC₁-C₁₁-Alkyl, S(O)ₚAryl, -(CH₂)ₘOR⁷, -(CH₂)ₘNR⁸R⁹, -COR⁷, -CO₂H, -CO₂R⁷ oder -NR⁸R⁹ sind;
R⁵ und R⁶ unter Bildung eines 3- bis 7-gliedrigen Cycloalkyl oder Cycloalkenyl verbunden sind;
R⁷ unabhängig Wasserstoff, C₁-C₁₁-Alkyl, C₂-C₁₂-Alkenyl, C₂-C₁₂-Alkinyl oder Aryl ist;
R⁸ und R⁹ unabhängig Wasserstoff, C₁-C₁₁-Alkyl, C₂-C₁₂-Alkenyl, C₂-C₁₂-Alkinyl, -COC₁-C₁₁-Alkyl, -CO-Aryl, C₃-C₈-Cycloalkyl, -CO₂C₁-C₁₁-Alkyl, -CO₂-Aryl, -SO₂C₁-C₁₁-Alkyl, -SO₂-Aryl sind oder unter Bildung eines 5- bis 7-gliedrigen Rings mit 1 bis 3 Heteroatome(n) miteinander verbunden sind;
R¹⁰ und R¹¹ unabhängig Wasserstoff, Halogen, Aryl oder Heteroaryl sind;
m 0 bis 5 ist;
n 0 bis 5 ist;
p 0 bis 2 ist;
wobei "Aryl" einen aromatischen Ring bedeutet, der unsubstituiert ist oder gegebenenfalls mit 1 bis 4 Substituenten, ausgewählt aus C₁-C₆-Alkyl, C₁-C₆-Alkoxy, C₁-C₆-Thioalkoxy, Halogen, Nitro, Cyano, -OH, -SH, -CF₃, -CO₂H, -CO₂C₁-C₆-Alkyl, -(CH₂)₀₋₂CF₃, -NH₂, -NHC₁-C₆-Alkyl, -SO₂C₁-C₁₁-Alkyl, -SO₂NH₂, -CONR'R" oder -N(C₁-C₆-Alkyl)₂, worin R' und R" unabhängig C₁-C₁₁-Alkyl, C₂-C₁₂-Alkenyl, C₂-C₁₂-Alkinyl sind oder unter Bildung eines 4- bis 7-gliedrigen Rings miteinander verbunden sind, substituiert ist; und
"Heteroaryl" einen aromatischen Ring bedeutet, der ein oder mehrere Heteroatom(e) enthält, der unsubstituiert ist oder gegebenenfalls mit 1 bis 4 Substituenten, ausgewählt aus C₁-C₆-Alkyl, C₁-C₆-Alkoxy, C₁-C₆-Thioalkoxy, Halogen, Nitro, Cyano, -OH, -SH, -CF₃, -CO₂H, -CO₂C₁-C₆-Alkyl, -(CH₂)₀₋₂CF₃,-NH₂, -NHC₁-C₆-Alkyl, -SO₂C₁-C₁₁-Alkyl, -SO₂NH₂, -CONR'R" oder -N(C₁-C₆-Alkyl)₂, worin R' und R" unabhängig C₁-C₁₁-Alkyl, C₂-C₁₂-Alkenyl, C₂-C₁₂-Alkinyl sind oder unter Bildung eines 4-bis 7-gliedrigen Rings miteinander verbunden sind, substituiert ist.

2. Verbindung nach Anspruch 1, worin
W für CR⁵R⁶, C₃-C₈-Cycloalkylen oder -(CH₂)-(C₃-C₈)Heterocycloalkylen steht;
X⁰ and X¹ S sind;
Ar¹ 4-Trifluormethylphenyl ist;
R¹, R², R³ und R⁴ Wasserstoff, C₁-C₆-Alkyl, C₁-C₆-Alkoxy, Halogen, -CF₃, -(CH₂)ₘOR⁷ oder -(CH₂)ₘNR⁸R⁹ sind;
R⁵ und R⁶ unter Bildung eines 3- bis 7-gliedrigen Cycloalkylrings miteinander verbunden sind;
R⁷ Wasserstoff oder C₁-C₁₁-Alkyl ist;
R⁸ und R⁹ unabhängig Wasserstoff, C₁-C₁₁-Alkyl oder -COC₁-C₁₁-Alkyl sind;
m 0 bis 5 ist und
m 0 ist.

3. Verbindung nach Anspruch 1, worin
W für 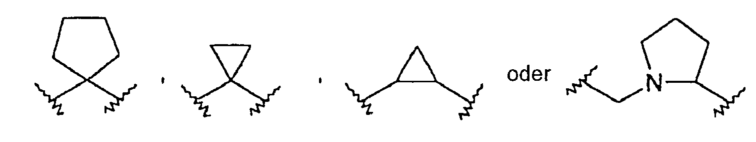 steht;
X⁰ und X¹ S sind;
Ar¹ 4-Trifluormethylphenyl ist;
R¹, R², R³ und R⁴ Wasserstoff, C₁-C₆-Alkyl, C₁-C₆-Alkoxy, Halogen, -CF₃, -(CH₂)ₘOR⁷ oder -(CH₂)ₘNR⁸R⁹ sind;
R⁷ unabhängig Wasserstoff oder C₁-C₆-Alkyl ist;
R⁸ und R⁹ jeweils unabhängig Wasserstoff oder C₁-C₆-Alkyl sind;
m 0 bis 5 ist und
n 0 ist.

4. Verbindung nach Anspruch 1, worin
R² und R³ Wasserstoff sind und
R¹ und R⁴ C₁-C₆-Alkyl oder C₁-C₆-Alkoxy sind.

5. Verbindung nach Anspruch 1, worin
R² und R³ Wasserstoff sind;
R¹ C₁-C₁₁-Alkyl ist und
R⁴ C₁-C₁₁-Alkoxy ist.

6. Verbindung nach Anspruch 1, worin
R² und R³ Wasserstoff sind;
R¹ Methyl, Ethyl, Isopropyl, n-Propyl, t-Butyl, n-Butyl oder Isobutyl ist und
R⁴ Methoxy, Ethoxy, Isopropoxy, n-Propoxy, t-Butoxy oder Isobutoxy ist.

7. Verbindung nach Anspruch 1, ausgewählt aus der Gruppe:
2-{4-[4-Methyl-2-(4-trifluormethylphenyl)-thiazol-5-ylmethylsulfanyl]-phenyl}-cyclopropancarbonsäure;
1-{4-[4-Methyl-2-(4-trifluormethylphenyl)-thiazol-5-ylmethylsulfanyl]-phenyl}-cyclopropancarbonsäure;
1-{4-[4-Methyl-2-(4-trifluormethylphenyl)-thiazol-5-ylmethylsulfanyl]-phenyl}-cyclopentancarbonsäure;
1-[{3-Methoxy-4-[4-methyl-2-(4-trifluormethylphenyl)thiazol-5-ylmethylsulfanyl]benzyl} pyrrolidin-2-carbonsäure;
(4-{4-[4-Methyl-2-(4-trifluormethylphenyl)-thiazol-5-ylmethylsulfanyl]-phenyl}-cyclohexyl)-essigsäure; und
pharmazeutisch verträglichen Salzen davon.

8. Pharmazeutische Zusammensetzung, umfassend eine Verbindung nach einem der Ansprüche 1 bis 7 und einen pharmazeutisch verträglichen Träger oder mehrere pharmazeutisch verträgliche Träger, ein pharmazeutisch verträgliches Verdünnungsmittel oder Exzipiens oder mehrere pharmazeutisch verträgliche Verdünnungsmittel oder Exzipientien.

9. Verwendung einer Verbindung nach einem der Ansprüche 1 bis 7 für die Herstellung eines Medikaments zur Behandlung, Prävention oder Kontrolle von Hyperlipidämie bei einem Säuger.

10. Verwendung einer Verbindung nach einem der Ansprüche 1 bis 7 für die Herstellung eines Medikaments zur Behandlung, Prävention oder Kontrolle von Hypercholesterinämie bei einem Säuger.

11. Verwendung einer Verbindung nach einem der Ansprüche 1 bis 7 für die Herstellung eines Medikaments zur Behandlung, Prävention oder Kontrolle von Atherosklerose bei einem Säuger.

12. Verfahren zur Herstellung einer Verbindung mit der Formel I: 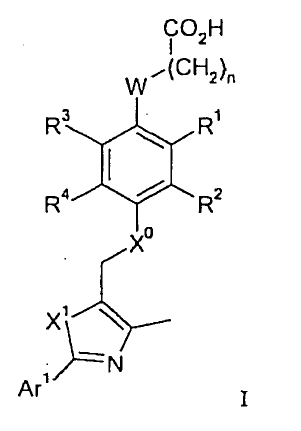 wobei das Verfahren umfasst: Umsetzen von 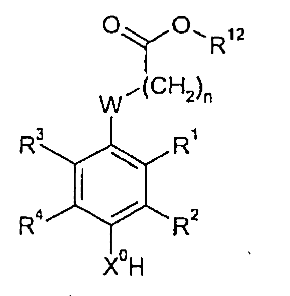 mit 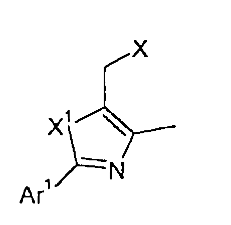 worin:
W für CR⁵R⁶, -(CH₂)ₚ-(C₃-C₈-Cycloalkylen) oder -(CH₂)ₚ-(C₃-C₈-Heterocycloalkylen) steht;
X⁰ und X¹ unabhängig 0 oder S sind;
Ar¹ Aryl oder Heteroaryl ist;
R¹, R², R³ und R⁴ Wasserstoff, C₁-C₆-Alkyl, C₁-C₆-Alkoxy, Halogen-C₁-C₁₁-alkyl, -O-(CH₂)ₚCF₃, Halogen, Nitro, Cyano, -OH, -SH, -CF₃, -S(O)ₚC₁-C₁₁-Alkyl, S(O)ₚAryl, -(CH₂)ₘOR⁷, -(CH₂)ₘNR⁸R⁹, -COR⁷, -CO₂H, -CO₂R⁷ oder -NR⁸R⁹ sind;
R⁵ und R⁶ unter Bildung eines 3- bis 7-gliedrigen Cycloalkyl oder Cycloalkenyl miteinander verbunden sind;
R⁷ unabhängig Wasserstoff, C₁-C₁₁-Alkyl, C₂-C₁₂-Alkenyl, C₂-C₁₂-Alkinyl oder Aryl ist;
R⁸ und R⁹ unabhängig Wasserstoff, C₁-C₁₁-Alkyl, C₂-C₁₂-Alkenyl, C₂-C₁₂-Alkinyl, -COC₁-C₁₁-Alkyl, -CO-Aryl, C₃-C₈-Cycloalkyl, -CO₂C₁-C₁₁-Alkyl, -CO₂-Aryl, -SO₂C₁-C₁₁-Alkyl, -SO₂-Aryl sind oder unter Bildung eines 4- bis 7-gliedrigen Rings mit 1 bis 3 Heteroatom(en) verbunden sind;
m 0 bis 5 ist;
n 0 bis 5 ist;
p 0 bis 2 ist;
wobei "Aryl" einen aromatischen Ring bezeichnet, der unsubstituiert oder gegebenenfalls mit 1 bis 4 Substituenten, ausgewählt aus C₁-C₆-Alkyl, C₁-C₆-Alkoxy, C₁-C₆-Thioalkoxy, Halogen, Nitro, Cyano, -OH, -SH, -CF₃, -CO₂H, -CO₂C₁-C₆-Alkyl, -(CH₂)₀₋₂CF₃, -NH₂, -NHC₁-C₆-Alkyl, -SO₂C₁-C₁₁-Alkyl, -SO₂NH₂, -CONR'R" und -N(C₁-C₆-Alkyl)₂, worin R' und R" unabhängig C₁-C₁₁-Alkyl, C₂-C₁₂-Alkenyl, C₂-C₁₂-Alkinyl sind oder unter Bildung eines 4- bis 7-gliedrigen Rings miteinander verbunden sind, substituiert ist;
,,Heteroaryl" einen aromatischen Ring bezeichnet, der ein oder mehrere Heteroatom(e) enthält, der unsubstituiert ist oder gegebenenfalls mit 1 bis 4 Substituenten, ausgewählt aus C₁-C₆-Alkyl, C₁-C₆-Alkoxy, C₁-C₆-Thioalkoxy, Halogen, Nitro, Cyano, -OH, -SH, -CF₃, -CO₂H, -CO₂C₁-C₆-Alkyl, -(CH₂)₀₋₂CF₃, -NH₂, -NHC₁-C₆-Alkyl, -SO₂C₁-C₁₁-Alkyl, -SO₂NH₂, -CONR'R" und -N(C₁-C₆-Alkyl)₂, worin R' und R" unabhängig C₁-C₁₁-Alkyl, C₂-C₁₂-Alkenyl, C₂-C₁₂-Alkinyl sind oder unter Bildung eines 4-bis 7-gliedrigen Rings miteinander verbunden sind, substituiert ist;
R¹² Niederalkyl ist und
X ein Halogen ist.

## Revendications

1. Composé de formule I : 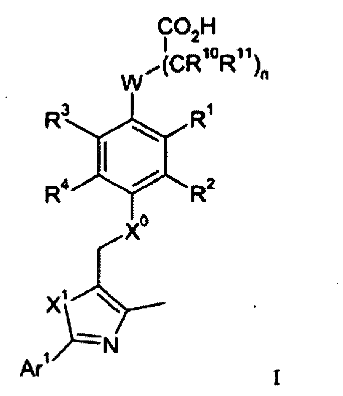 ou un de ses sels pharmaceutiquement acceptables, formule dans laquelle :
W représente un groupe CR⁵R⁶, -(CH₂)ₚ(cycloalkylène en C₃ à C₈) ou -(CH₂)ₚ(hétérocycloalkylène en C₃ à C₈) ;
X⁰ et X¹ représentent indépendamment 0 ou S ;
Ar¹ représente un groupe aryle ou hétéroaryle ;
R¹, R², R³ et R⁴ représentent un atome d'hydrogène, un groupe alkyle en C₁ à C₆, alkoxy en C₁ à C₆, halogénalkyle en C₁ à C₁₁, -O-(CH₂)ₚCF₃, halogéno, nitro, cyano, -OH, -SH, -CF₃, -S(O)ₚ(alkyle en C₁ à C₁₁), S(O)ₚaryle, -(CH₂)ₘOR⁷, -(CH₂)ₘNR⁸R⁹, -COR⁷, -CO₂H, -CO₂R⁷ ou -NR⁸R⁹ ;
R⁵ et R⁶ sont joints l'un à l'autre pour former un groupe cycloalkyle ou cycloalcényle tri- à heptagonal ;
R⁷ représente indépendamment un atome d'hydrogène, un groupe alkyle en C₁ à C₁₁, alcényle en C₂ à C₁₂, alcynyle en C₂ à C₁₂ ou aryle;
R⁸ et R⁹ représentent indépendamment un atome d'hydrogène, un groupe alkyle en C₁ à C₁₁, alcényle en C₂ à C₁₂, alcynyle en C₂ à C₁₂, -CO(alkyle en C₁ à C₁₁), -COaryle, cycloalkyle en C₃ à C₈, -CO₂ (alkyle en C₁ à C₁₁), -CO₂aryle, -SO₂ (alkyle en C₁ à C₁₁) ou -SO₂aryle, ou bien sont joints l'un à l'autre pour former un noyau tétra- à heptagonal comprenant 1 à 3 hétéroatomes ;
R¹⁰ et R¹¹ représentent indépendamment un atome d'hydrogène, un groupe halogéno, aryle ou hétéroaryle ;
m a une valeur de 0 à 5 ;
n a une valeur de 0 à 5 ;
p a une valeur de 0 à 2 ;
le terme "aryle" désigne un noyau aromatique qui est non substitué ou facultativement substitué avec 1 à 4 substituants choisis entre des substituants alkyle en C₁ à C₆, alkoxy en C₁ à C₆, thioalkoxy en C₁ à C₆, halogéno, nitro, cyano, -OH, -SH, -CF₃, -CO₂H, -CO₂(akyle en C₁ à C₆), -(CH₂)₀₋₂CF₃, -NH₂, -NH(alkyle en C₁ à C₆) , -SO₂(alkyle en C₁ à C₁₁), -SO₂NH₂, -CONR'R'' et -N(alkyle en C₁ à C₆)₂, dans lesquels R' et R" représentent indépendamment un groupe alkyle en C₁ à C₁₁, alcényle en C₂ à C₁₂ ou alcynyle en C₂ à C₁₂, ou bien sont joints l'un à l'autre pour former un noyau tétra- à heptagonal ; et
le terme "hétéroaryle" désigne un noyau aromatique contenant un ou plusieurs hétéroatomes qui est non substitué ou facultativement substitué avec 1 à 4 substituants choisis entre des substituants alkyle en C₁ à C₆, alkoxy en C₁ à C₆, thioalkoxy en C₁ à C₆, halogéno, nitro, cyano, -OH, -SH, -CF₃, -CO₂H, -CO₂(akyle en C₁ à C₆), -(CH₂)₀₋₂CF₃, -NH₂, -NH(alkyle en C₁ à C₆), -SO₂(alkyle en C₁ à C₁₁), -SO₂NH₂, -CONR'R" et -N(alkyle en C₁ à C₆)₂, dans lesquels R' et R" représentent indépendamment un groupe alkyle en C₁ à C₁₁, alcényle en C₂ à C₁₂ ou alcynyle en C₂ à C₁₂, ou bien sont joints l'un à l'autre pour former un noyau tétra- à heptagonal.

2. Composé suivant la revendication 1, dans lequel
W représente un groupe CR⁵R⁶, cycloalkylène en C₃ à C₈ ou -(CH₂)-(hétérocycloalkylène en C₃ à C₈) ;
X⁰ et X¹ représentent S ;
Ar¹ représente un groupe 4-trifluorométhylphényle ;
R¹, R², R³ et R⁴ représentent un atome d'hydrogène, un groupe alkyle en C₁ à C₆, alkoxy en C₁ à C₆, halogéno, -CF₃, -(CH₂)ₘOR⁷ ou -(CH₂)ₘNR⁸R⁹ ;
R⁵ et R⁶ sont joints l'un à l'autre pour former un noyau cycloalkyle tri- à heptagonal ;
R⁷ représente un atome d'hydrogène ou un groupe alkyle en C₁ à C₁₁ ;
R⁸ et R⁹ représentent indépendamment un atome d'hydrogène, un groupe alkyle en C₁ à C₁₁ ou -CO(alkyle en C₁ à C₁₁) ;
m a une valeur de 0 à 5 ; et
m est égal à 0.

3. Composé suivant la revendication 1, dans lequel
W représente un groupe 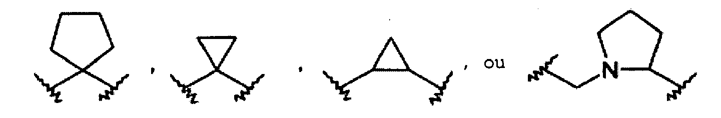 X⁰ et X¹ représentent S ;
Ar¹ représente un groupe 4-trifluorométhylphényle ;
R¹, R² R³ et R⁴ représentent un atome d'hydrogène, un groupe alkyle en C₁ à C₆, alkoxy en C₁ à C₆, halogéno, -CF₃, -(CH₂)ₘOR⁷ ou -(CH₂)ₘNR⁸R⁹;
R⁷ représente un atome d'hydrogène ou un groupe alkyle en C₁ à C₆ ;
R⁸ et R⁹ représentent chacun indépendamment un atome d'hydrogène ou un groupe alkyle en C₁ à C₆ ;
m a une valeur de 0 à 5 ; et
n est égal à 0.

4. Composé suivant la revendication 1, dans lequel
R² et R³ représentent un atome d'hydrogène; et
R¹ et R⁴ représentent un groupe alkyle en C₁ à C₆ ou alkoxy en C₁ à C₆.

5. Composé suivant la revendication 1, dans lequel
R² et R³ représentent un atome d'hydrogène ;
R¹ représente un groupe alkyle en C₁ à C₁₁ ; et
R⁴ représente un groupe alkoxy en C₁ à C₁₁.

6. Composé suivant la revendication 1, dans lequel
R² et R³ représentent un atome d'hydrogène ;
R¹ représente un groupe méthyle, éthyle, isopropyle, n-propyle, tertiobutyle, n-butyle ou isobutyle ; et
R⁴ représente un groupe méthoxy, éthoxy, isopropoxy, n-propoxy, tertiobutoxy ou isobutoxy.

7. Composé suivant la revendication 1, choisi dans le groupe consistant en :
l'acide 2-{4-[4-méthyl-2-(4-trifluorométhyl-phényl)-thiazole-5-ylméthylsulfanyl]-phényl}-cyclopropanecarboxylique ;
l'acide 1-{4-[4-méthyl-2-(4-trifluorométhyl-phényl)-thiazole-5-ylméthylsulfanyl]-phényl}-cyclopropanecarboxylique ;
l'acide 1-{4-[4-méthyl-2-(4-trifluorométhyl-phényl)-thiazole-5-ylméthylsulfanyl]-phényl}-cyclopentanecarboxylique ;
l'acide 1-[{3-méthoxy-4-[4-méthyl-2-(4-trifluorométhylphényl)thiazole-5-ylméthylsulfanyl]benzyl}pyrrolidine-2-carboxylique ;
l'acide (4-{4-[4-méthyl-2-(4-trifluorométhyl-phényl)-thiazole-5-ylméthylsulfanyl]-phényl}-cyclohexyl)-acétique ; et
ses sels pharmaceutiquement acceptables.

8. Composition pharmaceutique comprenant un composé de l'une quelconque des revendications 1 à 7 et un ou plusieurs supports, diluants ou excipients pharmaceutiquement acceptables.

9. Utilisation d'un composé de l'une quelconque des revendications 1 à 7 pour la production d'un médicament destiné à traiter, prévenir ou lutter contre, l'hyperlipidémie chez un mammifère.

10. Utilisation d'un composé de l'une quelconque des revendications 1 à 7 pour la production d'un médicament destiné à traiter, prévenir ou lutter contre, l'hypercholestérolémie chez un mammifère.

11. Utilisation d'un composé de l'une quelconque des revendications 1 à 7 pour la production d'un médicament destiné à traiter, prévenir ou lutter contre, l'athérosclérose chez un mammifère.

12. Procédé pour la préparation d'un composé de formule I : 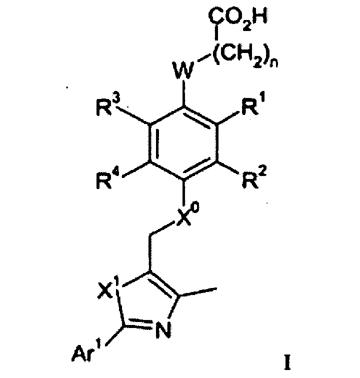 procédé comprenant la réaction d'un composé de formule : 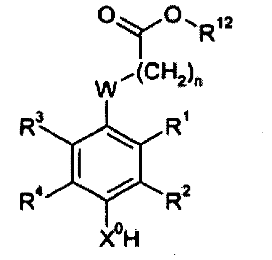 avec un composé de formule : 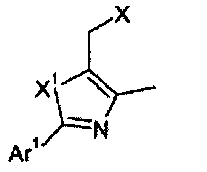 où :
W représente un groupe CR⁵R⁶, -(CH₂)ₚ(cycloalkylène en C₃ à C₈) ou -(CH₂)ₚ(hétérocycloalkylène en C₃ à C₈) ;
X⁰ et X¹ représentent indépendamment O ou S ;
Ar¹ représente un groupe aryle ou hétéroaryle ;
R¹, R², R³ et R⁴ représentent un atome d'hydrogène, un groupe alkyle en C₁ à C₆, alkoxy en C₁ à C₆, halogénalkyle en C₁ à C₁₁, -O-(CH₂)ₚCF₃, halogéno, nitro, cyano, -OH, -SH, -CF₃, -S(O)ₚ(alkyle en C₁ à C₁₁), S(O)ₚaryle, -(CH₂)ₘOR⁷, -(CH₂)ₘNR⁸R⁹, -COR⁷, -CO₂H, -CO₂R⁷ ou -NR⁸R⁹ ;
R⁵ et R⁶ sont joints l'un à l'autre pour former un groupe cycloalkyle ou cycloalcényle tri- à heptagonal ;
R⁷ représente indépendamment un atome d'hydrogène, un groupe alkyle en C₁ à C₁₁, alcényle en C₂ à C₁₂, alcynyle en C₂ à C₁₂ ou aryle ;
R⁸ et R⁹ représentent indépendamment un atome d'hydrogène, un groupe alkyle en C₁ à C₁₁, alcényle en C₂ à C₁₂, alcynyle en C₂ à C₁₂, -CO(alkyle en C₁ à C₁₁), -COaryle, cycloalkyle en C₃ à C₈, -CO₂ (alkyle en C₁ à C₁₁), -CO₂aryle, -SO₂ (alkyle en C₁ à C₁₁) ou -SO₂aryle, ou bien sont joints l'un à l'autre pour former un noyau tétra- à heptagonal comprenant 1 à 3 hétéroatomes ;
m a une valeur de 0 à 5 ;
n a une valeur de 0 à 5;
p a une valeur de 0 à 2;
le terme "aryle" désigne un noyau aromatique qui est non substitué ou facultativement substitué avec 1 à 4 substituants choisis entre des substituants alkyle en C₁ à C₆, alkoxy en C₁ à C₆, thioalkoxy en C₁ à C₆, halogéno, nitro, cyano, -OH, -SH, -CF₃, -CO₂H, -CO₂(akyle en C₁ à C₆), - (CH₂)₀₋₂CF3 , -NH₂, -NH(alkyle en C₁ à C₆), -SO₂(alkyle en C₁ à C₁₁), -SO₂NH₂, -CONR'R" et -N(alkyle en C₁ à C₆)₂, dans lesquels R' et R" représentent indépendamment un groupe alkyle en C₁ à C₁₁, alcényle en C₂ à C₁₂ ou alcynyle en C₂ à C₁₂, ou bien sont joints l'un à l'autre pour former un noyau tétra- à heptagonal ;
le terme "hétéroaryle désigne un noyau aromatique contenant un ou plusieurs hétéroatomes qui est non substitué ou facultativement substitué avec 1 à 4 substituants choisis entre des substituants alkyle en C₁ à C₆, alkoxy en C₁ à C₆, thioalkoxy en C₁ à C₆, halogéno, nitro, cyano, -OH, -SH, -CF₃, -CO₂H, -CO₂(akyle en C₁ à C₆), - (CH₂)₀₋₂CF₃, -NH₂, -NH (alkyle en C₁ à C₆) , -SO₂(alkyle en C₁ à C₁₁), -SO₂NH₂, -CONR'R", et -N(alkyle en C₁ à C₆)₂, dans lesquels R' et R" représentent indépendamment un groupe alkyle en C₁ à C₁₁, alcényle en C₂ à C₁₂ ou alcynyle en C₂ à C₁₂, ou bien sont joints l'un à l'autre pour former un noyau tétra- à heptagonal ;
R¹² représente un groupe alkyle inférieure ; et
X représente un atome d'halogène.
